# EUROPÄISCHE PATENTSCHRIFT

(11) **EP 1 178 987 B1**
(45) Veröffentlichungstag und Bekanntmachung des Hinweises auf die Patenterteilung: **13.08.2003**
(21) Anmeldenummer: 00920731.7
(22) Anmeldetag: 20.04.2000
(51) Int. Cl.: C07D 413/10, C07D 249/12, C07D 261/08, A01N 43/80

(54) **SUBSTITUIERTE BENZOYLISOXAZOLE UND IHRE VERWENDUNG ALS HERBIZIDE**
SUBSTITUTED BENZOYLISOXAZOLES AND THE USE THEREOF AS HERBICIDES
BENZOYLISOXAZOLES SUBSTITUES ET LEUR UTILISATION COMME HERBICIDES

(30) Priorität: 06.05.1999 DE 19920791
(43) Veröffentlichungstag der Anmeldung: 13.02.2002
(73) Patentinhaber: Bayer CropScience AG, 40789 Monheim (DE)
(72) Erfinder: MÜLLER, Klaus-Helmut, D-40593 Düsseldorf (DE); LEHR, Stefan, D-40764 Langenfeld (DE); SCHALLNER, Otto, D-40789 Monheim (DE); SCHWARZ, Hans-Georg, D-40764 Langenfeld (DE); WROBLOWSKY, Heinz-Jürgen, D-40764 Langenfeld (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR)
(86) Internationale Anmeldenummer: EP0003608
(87) Internationale Veröffentlichungsnummer: WO00068227

(56) Entgegenhaltungen:
- EP-A- 0 487 357
- WO-A-95/22903
- WO-A-95/31446
- WO-A-96/26192
- WO-A-97/27187
- WO-A-99/03856

## Beschreibung

Die Erfindung betrifft neue substituierte Benzoylisoxazole, Verfahren zu ihrer Herstellung und ihre Verwendung als Herbizide.

Es ist bereits bekannt, daß bestimmte substituierte Benzoylisoxazole herbizide Eigenschaften aufweisen (vgl. EP-A-418 175, EP-A-487 357, EP-A-527 036, EP-A-527 037, EP-A-560 483, EP-A-609 797, EP-A-609 798, EP-A-636 622, US-A-5 834 402, US-A-5 863 865, WO-A-96/26192, WO-A-97/27187, WO-A-97/43270, WO-A-99/03856). Die Wirkung dieser Verbindungen ist jedoch nicht in allen Belangen zufriedenstellend.

Es wurden nun die neuen Verbindungen der allgemeinen Formel (I), 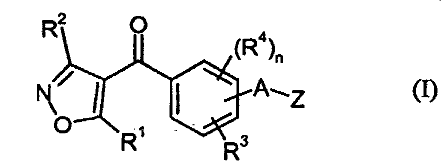 in welcher
- n: für die Zahlen 0, 1, 2 oder 3 steht,
- A: für Methylen, Ethyliden (Ethan-1,1-diyl) oder Dimethylen (Ethan-1,2-diyl) steht,
- R¹: für Wasserstoff, fiir jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl oder Ethyl substituiertes Cyclopropyl, Cyclobutyl, Cyclopentyl oder Cyclohexyl steht,
- R²: für Wasserstoff, Cyano, Carbamoyl, Fluor, Chlor, Brom, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxy, Ethoxy, n- oder i-Propoxy, Methoxycarbonyl, Ethoxycarbonyl, n-oder i-Propoxycarbonyl, oder für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl oder Ethylsulfonyl steht,
- R³: für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propyl-thio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
- R⁴: für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, fiir jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
- Z: für die heterocyclischen Gruppierungen 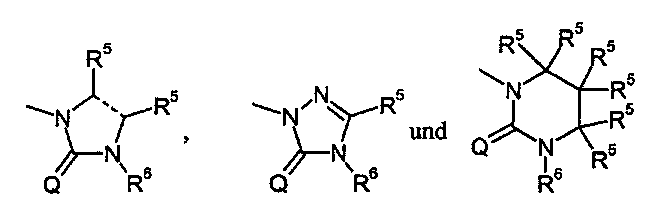 steht,
- R⁵: für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy steht,
- R⁶: für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl, oder zusammen mit R⁵ für Propan-1,3-diyl (Trimethylen), Butan-1,4-diyl (Tetramethylen) oder Pentan-1,5-diyl (Pentamethylen) steht, und
- Q: für Sauerstoff oder Schwefel steht,
gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.
- R¹: steht ganz besonders bevorzugt für Wasserstoff, fiir jeweils gegebenenfalls durch Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, oder für gegebenenfalls durch Cyano, Fluor, Chlor, Brom, Methyl oder Ethyl substituiertes Cyclopropyl.
- R²: steht ganz besonders bevorzugt für Wasserstoff, Cyano, Carbamoyl, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxycarbonyl, Ethoxycarbonyl, n- oder i-Propoxycarbonyl, oder für jeweils gegebenenfalls durch Fluor und/oder Chlor substituiertes Methylthio, Ethylthio, n- oder i-Propylthio, Methylsulfinyl, Ethylsulfinyl, Methylsulfonyl oder Ethylsulfonyl.
- R³: steht ganz besonders bevorzugt für Wasserstoff, Nitro, Cyano, Fluor, Chlor, Brom, Iod, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.
- R⁴: steht ganz besonders bevorzugt für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.
- A: steht am meisten bevorzugt für Methylen.
- Q: steht am meisten bevorzugt für Sauerstoff.
- R¹: steht am meisten bevorzugt für Cyclopropyl.
- R²: steht am meisten bevorzugt für Wasserstoff, Methoxycarbonyl oder Ethoxycarbonyl.
- R³: steht am meisten bevorzugt für Chlor, Brom, Cyano, Trifluormethyl oder Methylsulfonyl.
- R⁴: steht am meisten bevorzugt für Wasserstoff, Cyano, Chlor, Nitro, Methyl, Trifluormethyl, Methoxy oder Methylsulfonyl.

Erfindungsgemäß bevorzugt sind die Verbindungen der Formel (I), in welchen eine Kombination der vorstehend als bevorzugt aufgeführten Bedeutungen vorliegt.

Erfindungsgemäß besonders bevorzugt sind die Verbindungen der Formel (I), in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt.

Erfindungsgemäß am meisten bevorzugt sind die Verbindungen der Formel (I), in welchen eine Kombination der vorstehend als am meisten bevorzugt aufgeführten Bedeutungen vorliegt.

Aus den als bevorzugt, besonders bevorzugt, ganz besonders bevorzugt oder als am meisten bevorzugt angegebenen Bedeutungen sind die Verbindungen der allgemeinen Formel (IA) noch einmal besonders hervorzuheben, 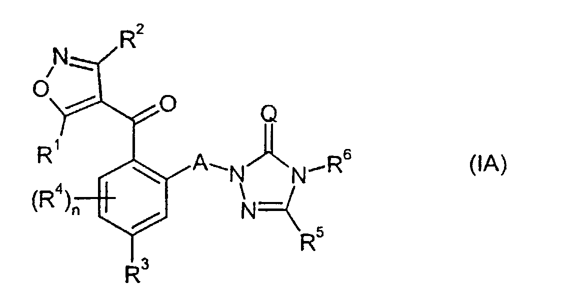 in welchen
- n, A, Q, R¹, R², R³, R⁴, R⁵ und R⁶: die vorausgehend angegebenen Bedeutungen haben, wobei die Verbindungen der Formel (IA), bei welchen A für Methylen steht, hierbei ganz besonders hervorzuheben sind.

Aus den als bevorzugt, besonders bevorzugt, ganz besonders bevorzugt oder als am meisten bevorzugt angegebenen Bedeutungen sind außerdem die Verbindungen der allgemeinen Formel (IB) noch einmal besonders hervorzuheben, 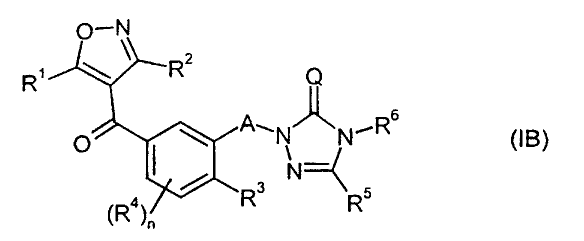 in welchen
- n, A, Q, R¹, R², R³, R⁴, R⁵ und R⁶: die vorausgehend angegebenen Bedeutungen haben.

Aus den als bevorzugt, besonders bevorzugt, ganz besonders bevorzugt oder als am meisten bevorzugt angegebenen Bedeutungen sind außerdem Verbindungen der allgemeinen Formel (IC) noch einmal besonders hervorzuheben, 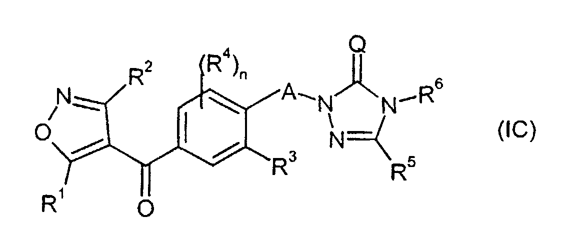 in welchen
- n, A, Q, R¹, R², R³, R⁴, R⁵ und R⁶: die vorausgehend angegebenen Bedeutungen haben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt.

### Gruppe 1

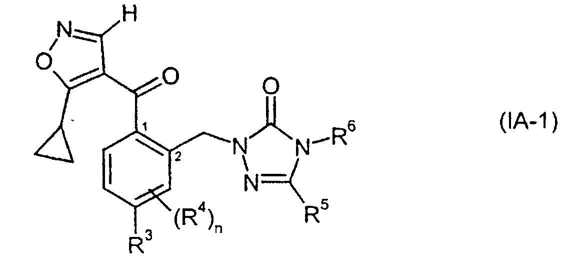

R³, (R⁴)ₙ, R⁵ und R⁶ haben hierbei beispielhaft die in der nachstehenden Tabelle angegebenen Bedeutungen:

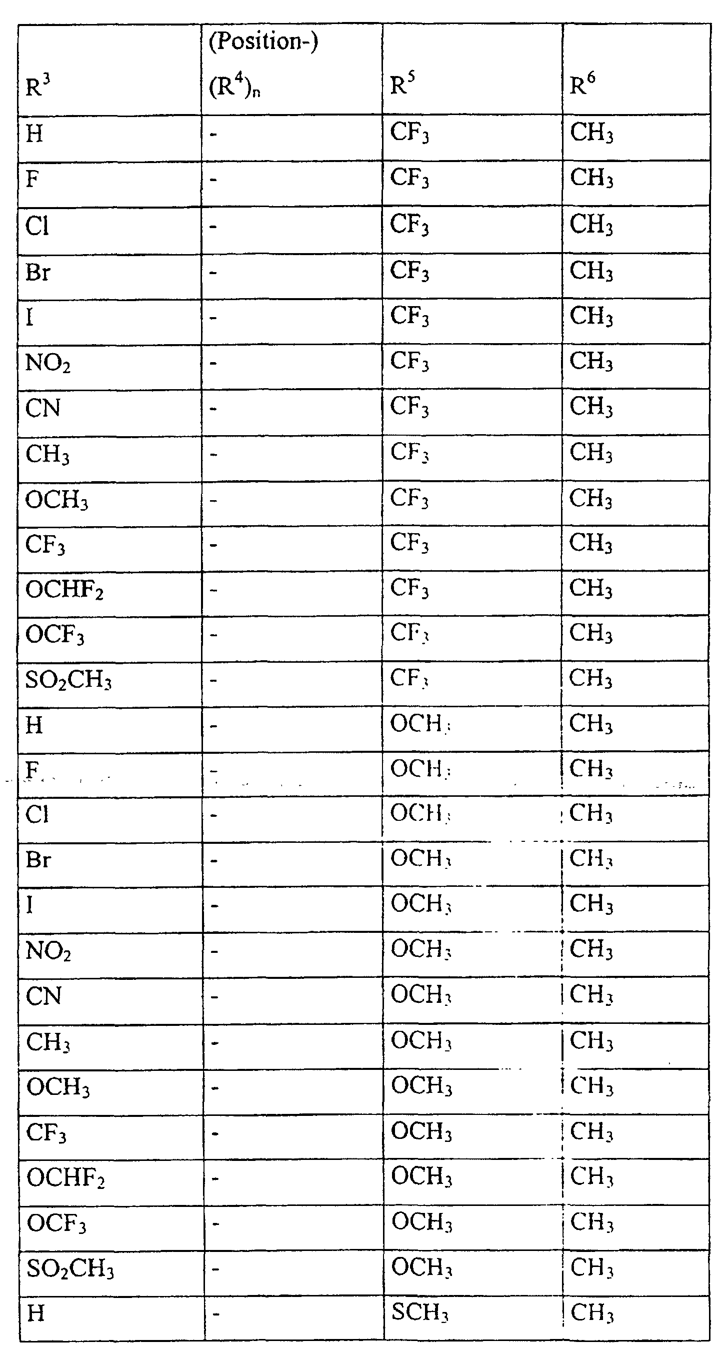

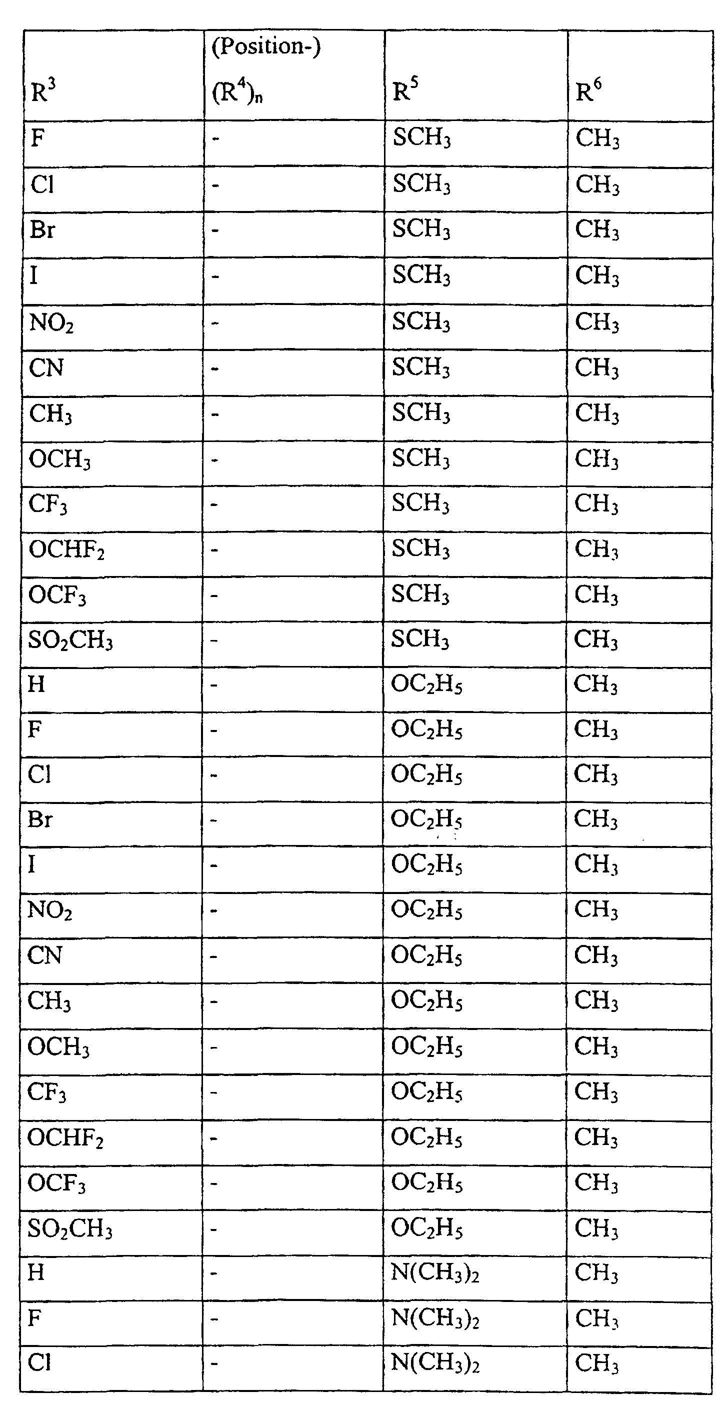

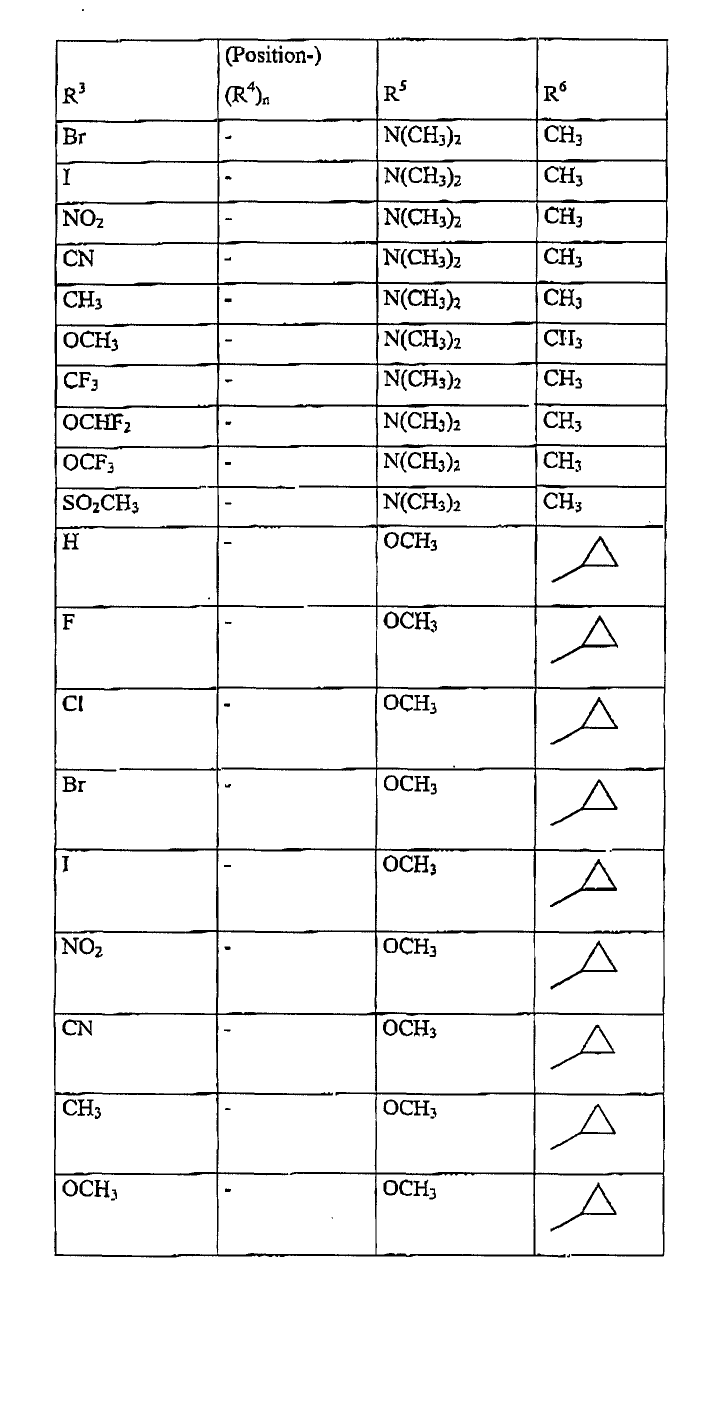

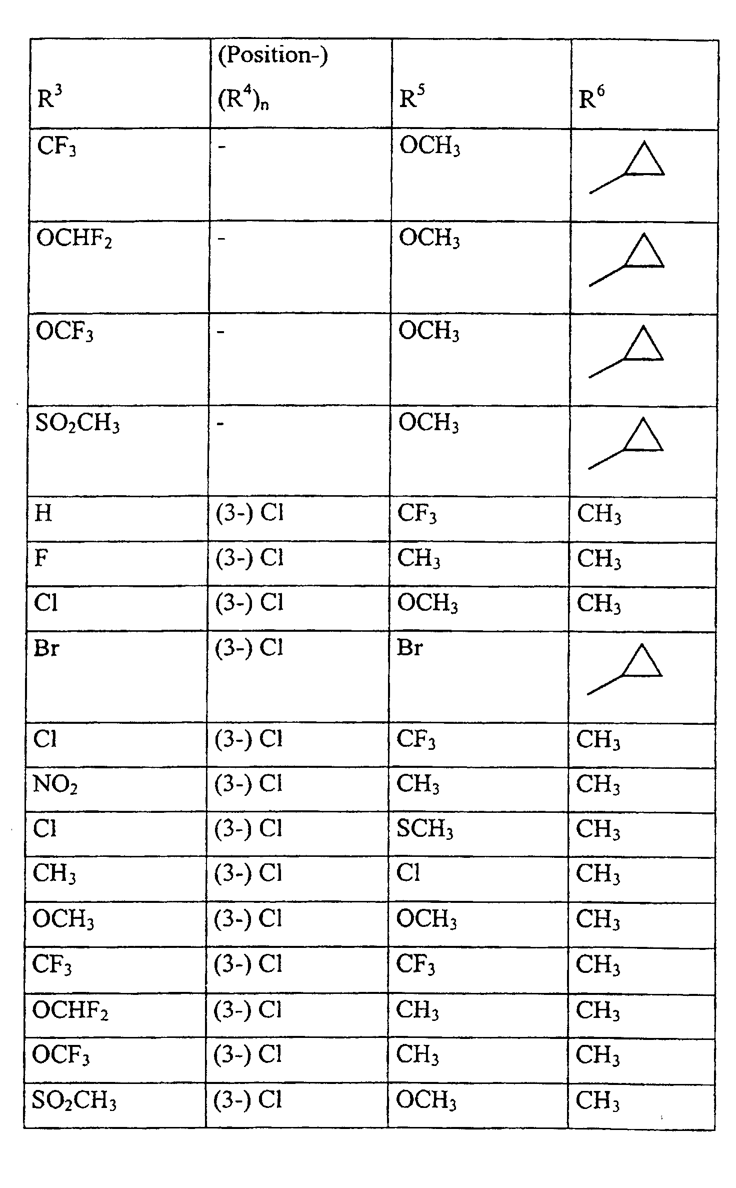

### Gruppe 2

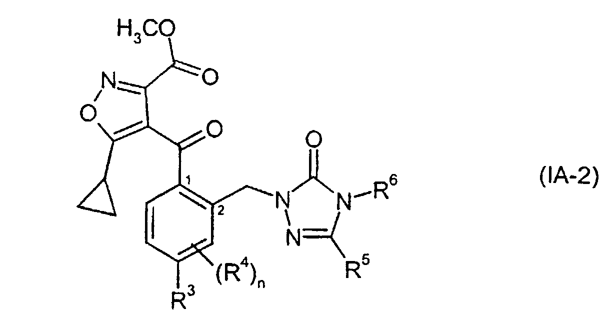

R³, (R⁴)ₙ, R⁵ und R⁶ haben hierbei beispielhaft die für Gruppe 1 angegebenen Bedeutungen.

### Gruppe 3

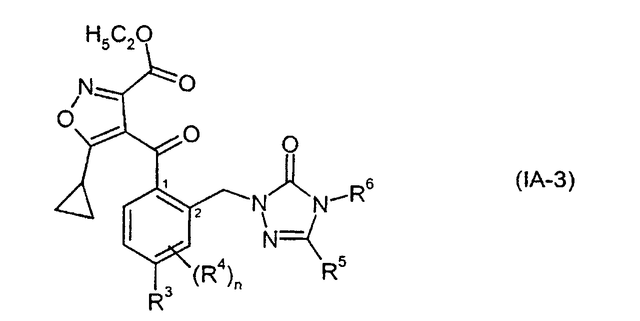

R³, (R⁴)ₙ, R⁵ und R⁶ haben hierbei beispielhaft die für Gruppe 1 angegebenen Bedeutungen.

### Gruppe 4

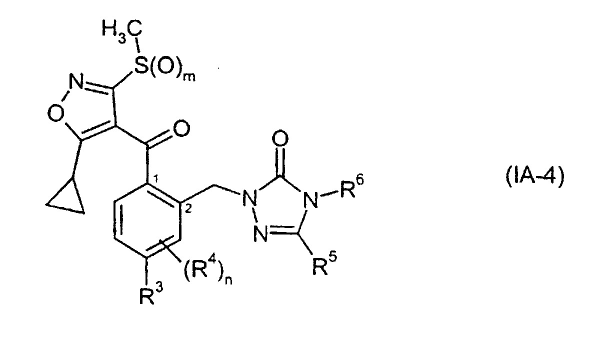

R³, (R⁴)ₙ, R⁵ und R⁶ haben hierbei beispielhaft die für Gruppe 1 angegebenen Bedeutungen und m steht für die Zahlen 0,1 oder 2.

### Gruppe 5

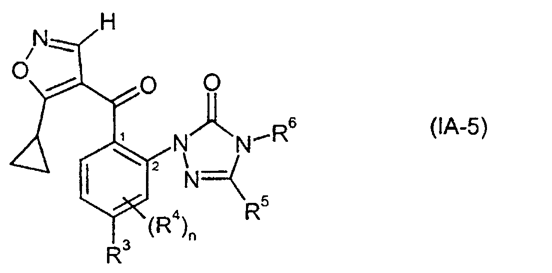

R³, (R⁴)ₙ, R⁵ und R⁶ haben hierbei beispielhaft die für Gruppe 1 angegebenen Bedeutungen.

### Gruppe 6

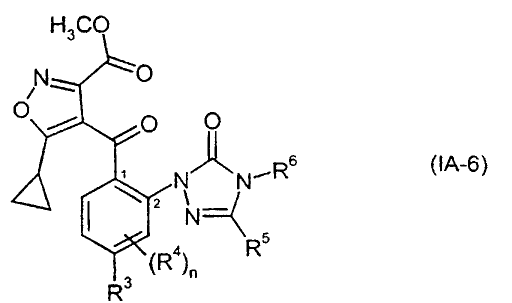

R³, (R⁴)ₙ, R⁵ und R⁶ haben hierbei beispielhaft die für Gruppe 1 angegebenen Bedeutungen.

### Gruppe 7

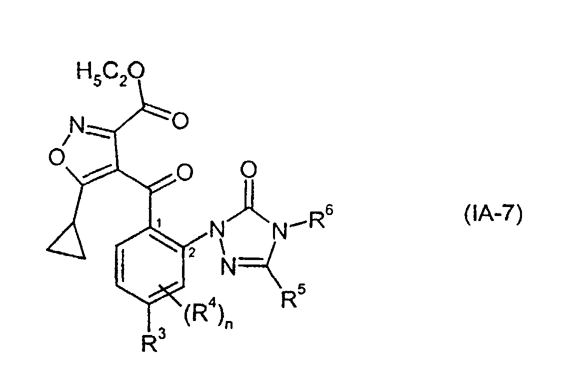

R³, (R⁴)ₙ, R⁵ und R⁶ haben hierbei beispielhaft die für Gruppe 1 angegebenen Bedeutungen.

### Gruppe 8

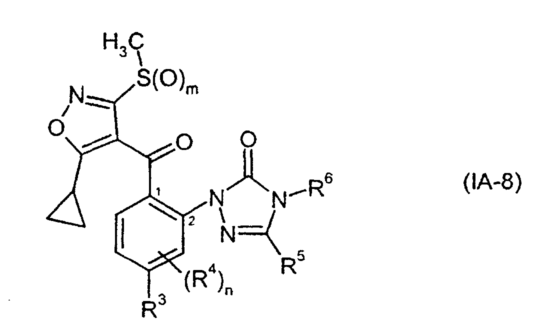

R³, (R⁴)ₙ, R⁵ und R⁶ haben hierbei beispielhaft die für Gruppe 1 angegebenen Bedeutungen und m steht für die Zahlen 0, 1 oder 2.

### Gruppe 9

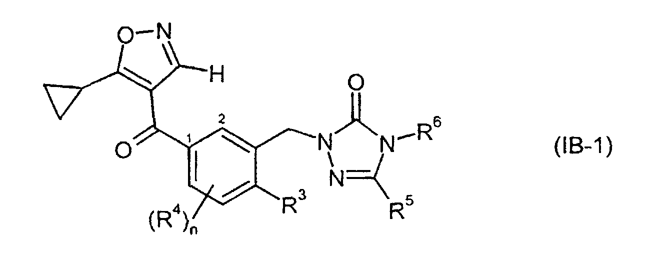

R³, (R⁴)ₙ, R⁵ und R⁶ haben hierbei beispielhaft die in der nachstehenden Tabelle angegebenen Bedeutungen:

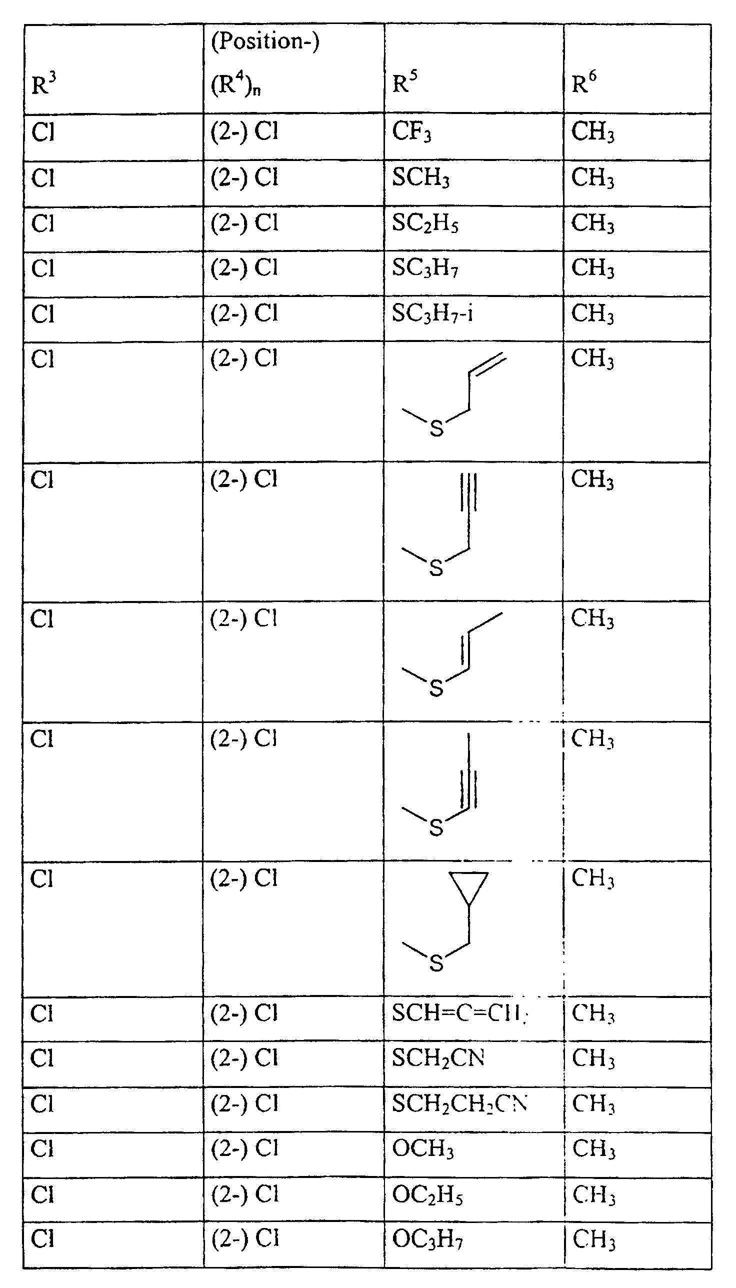

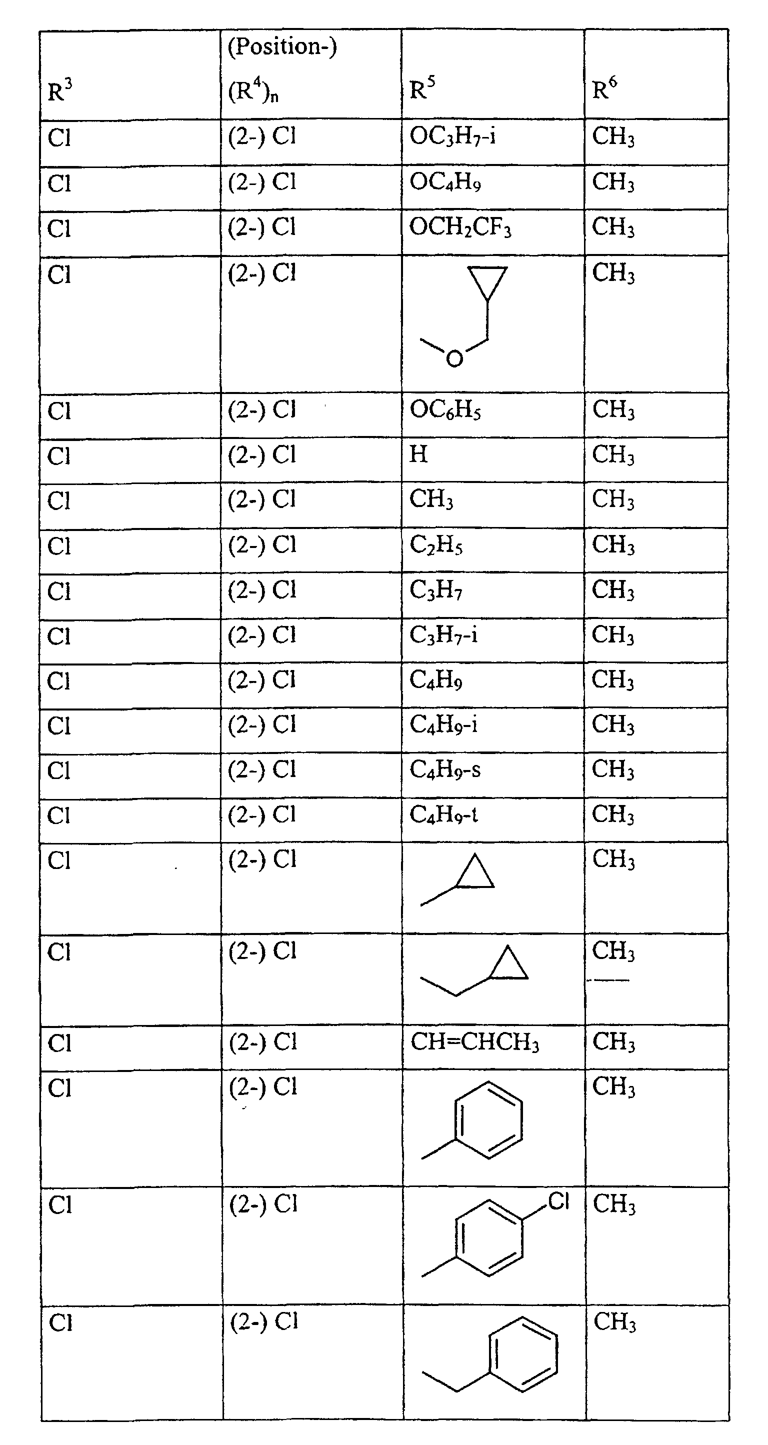

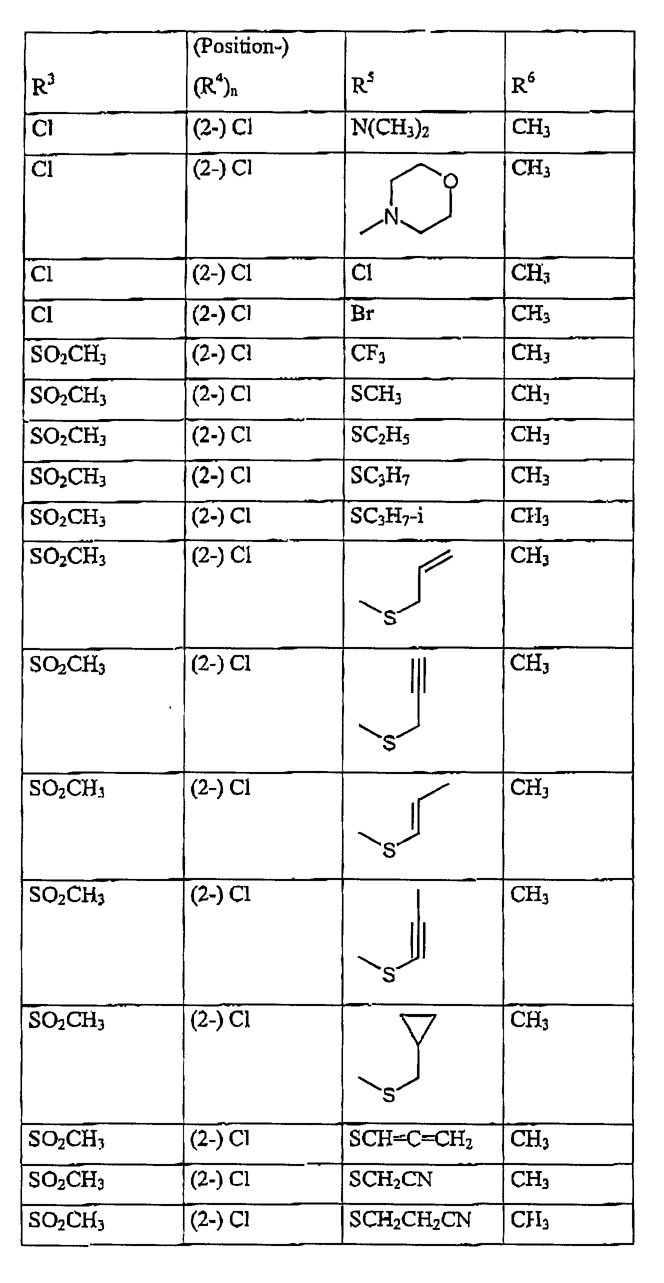

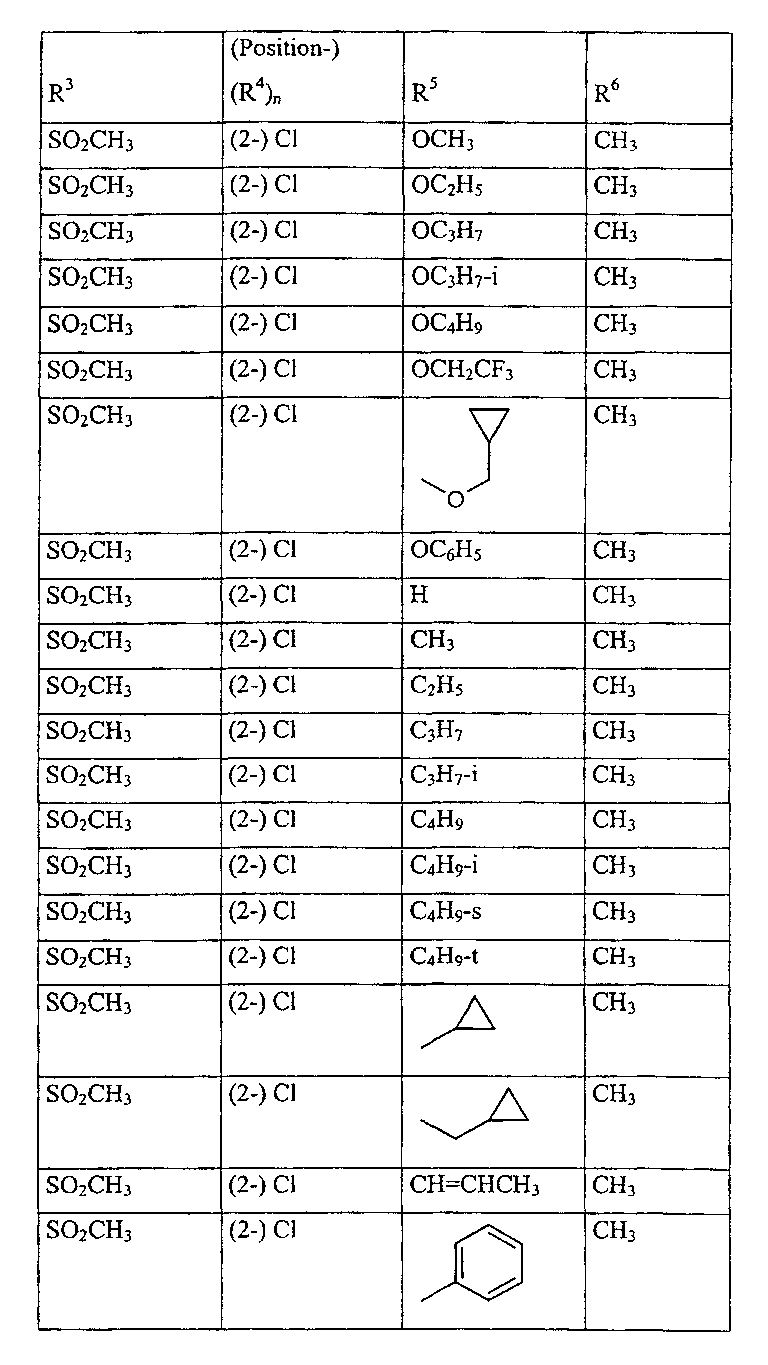

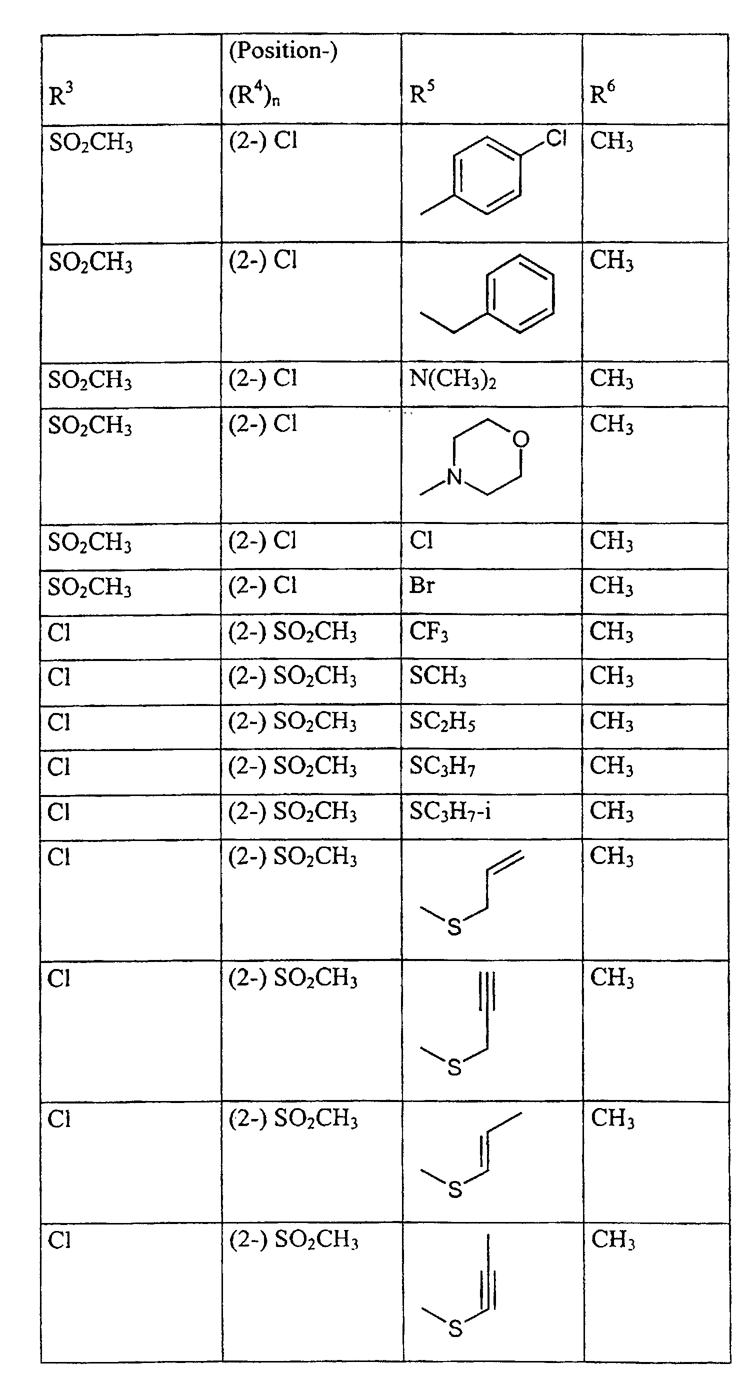

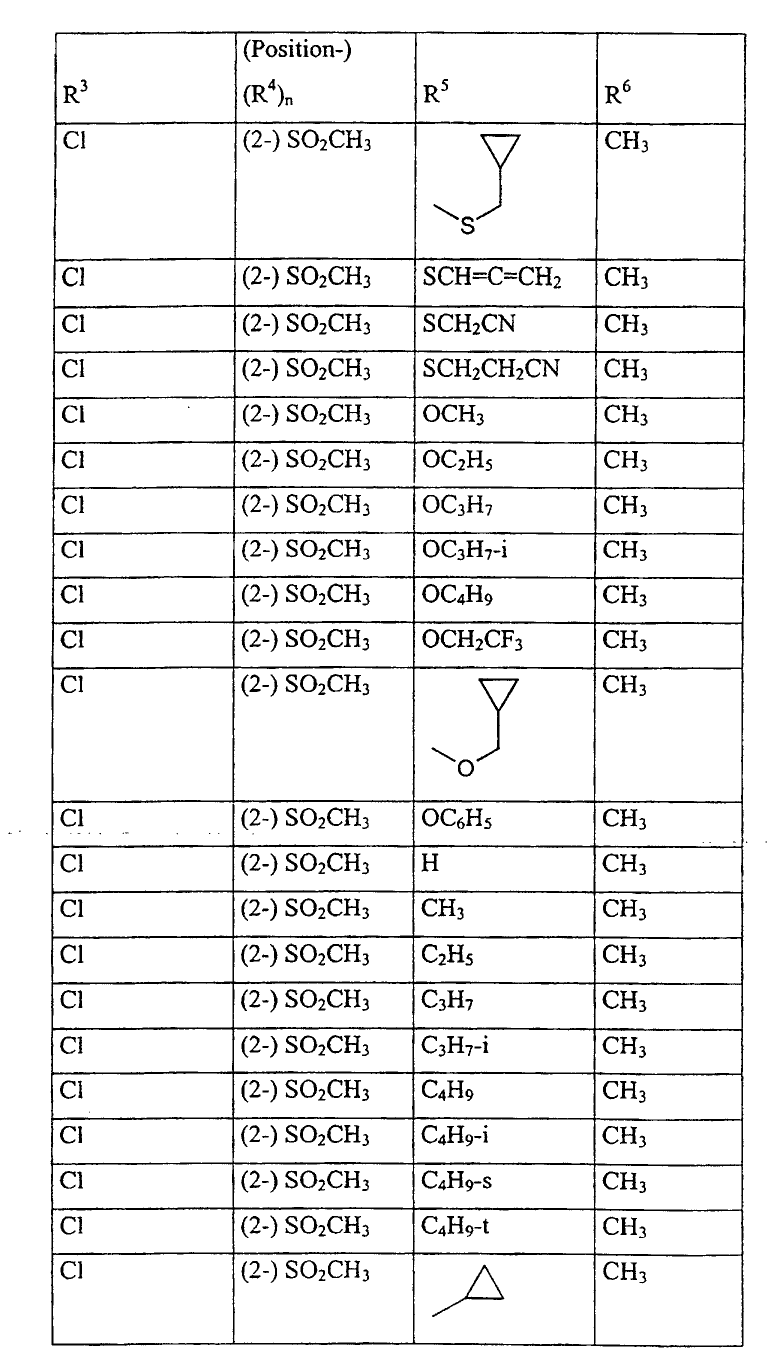

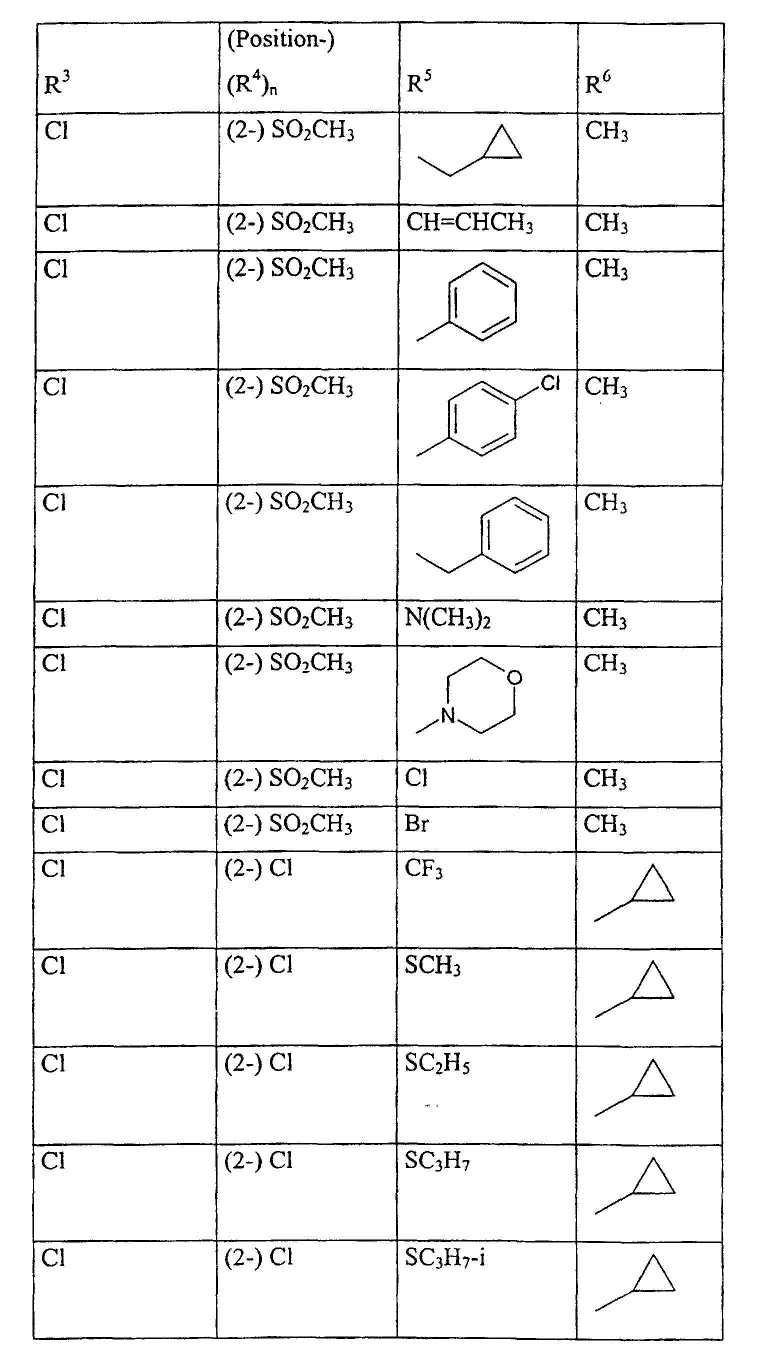

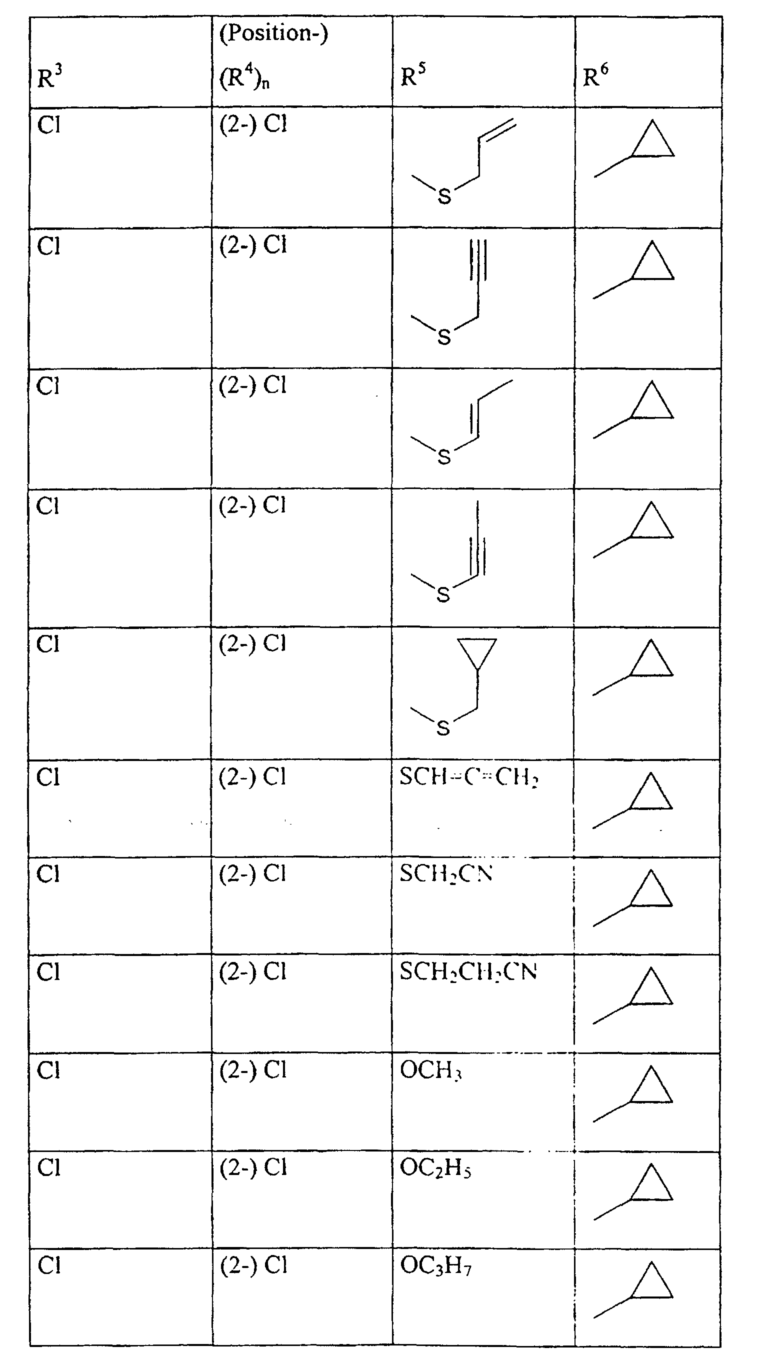

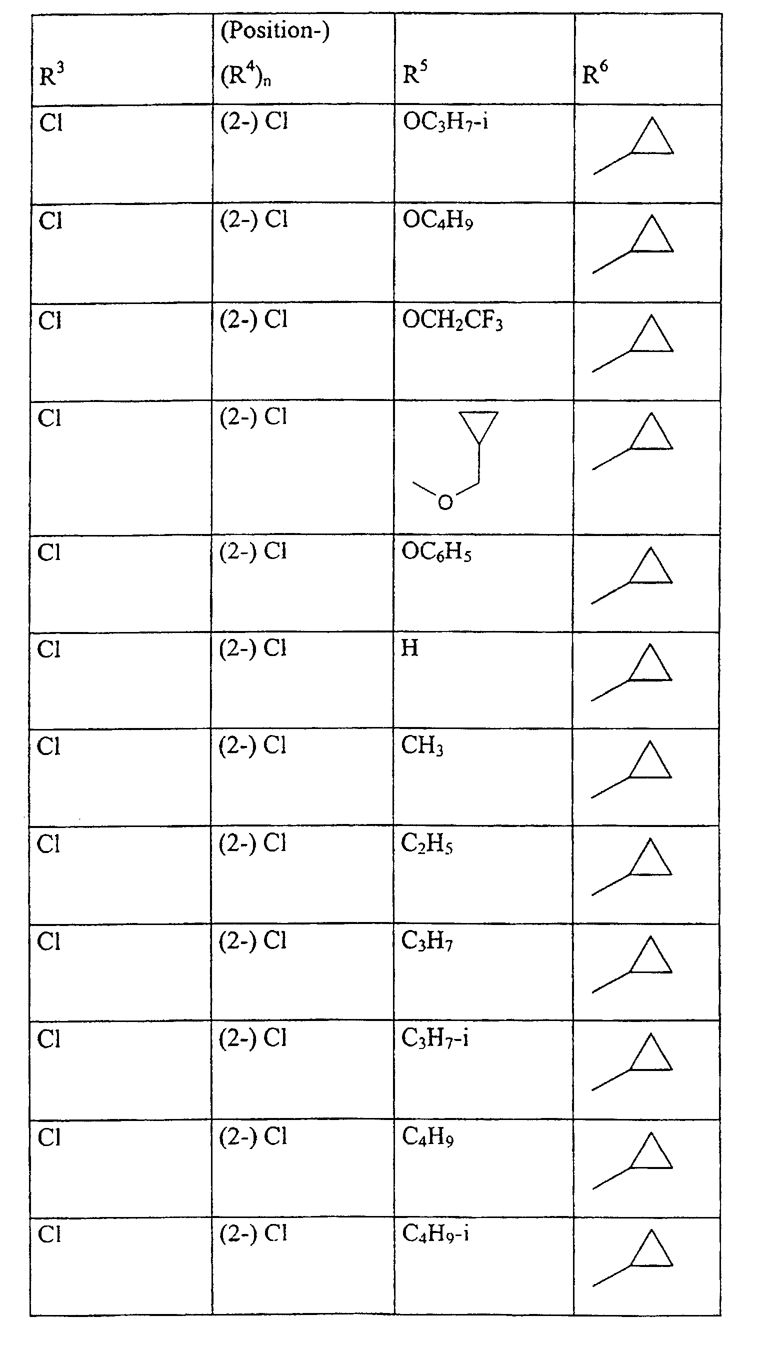

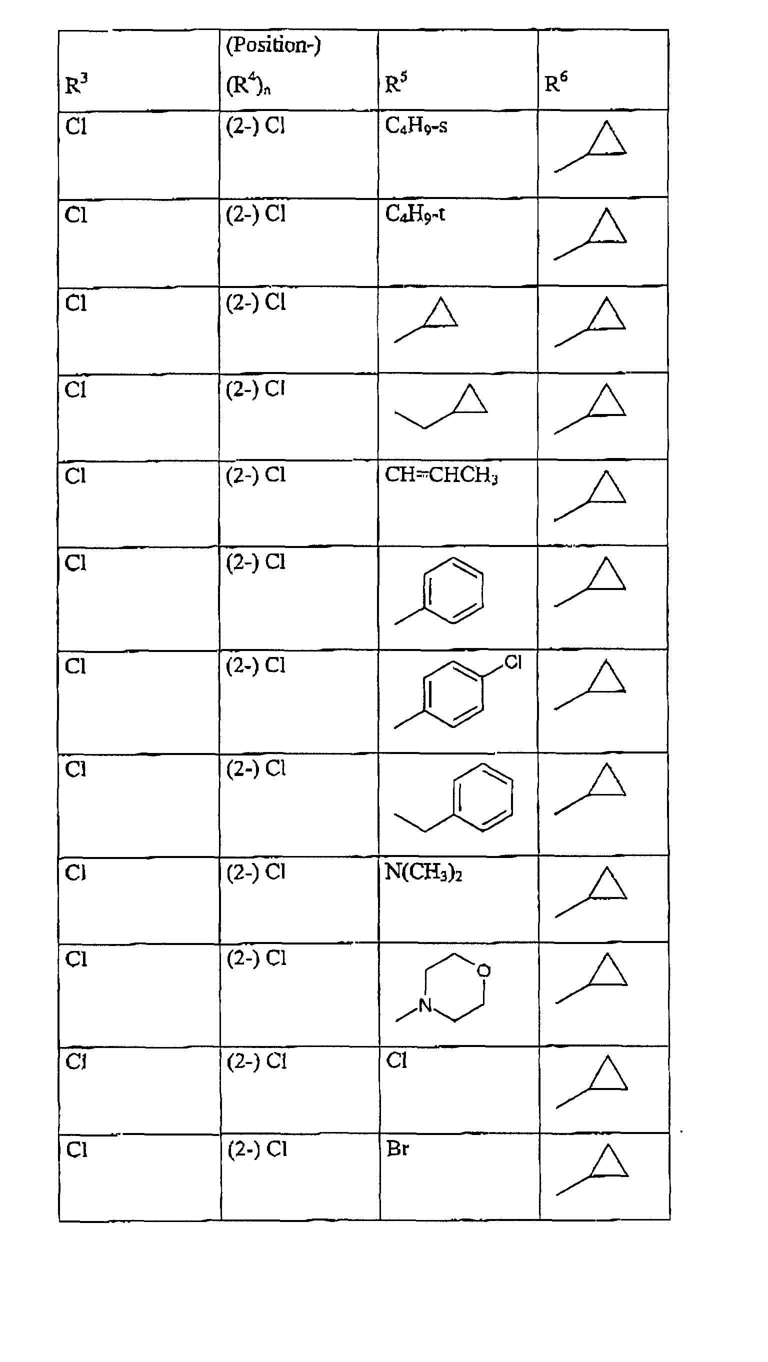

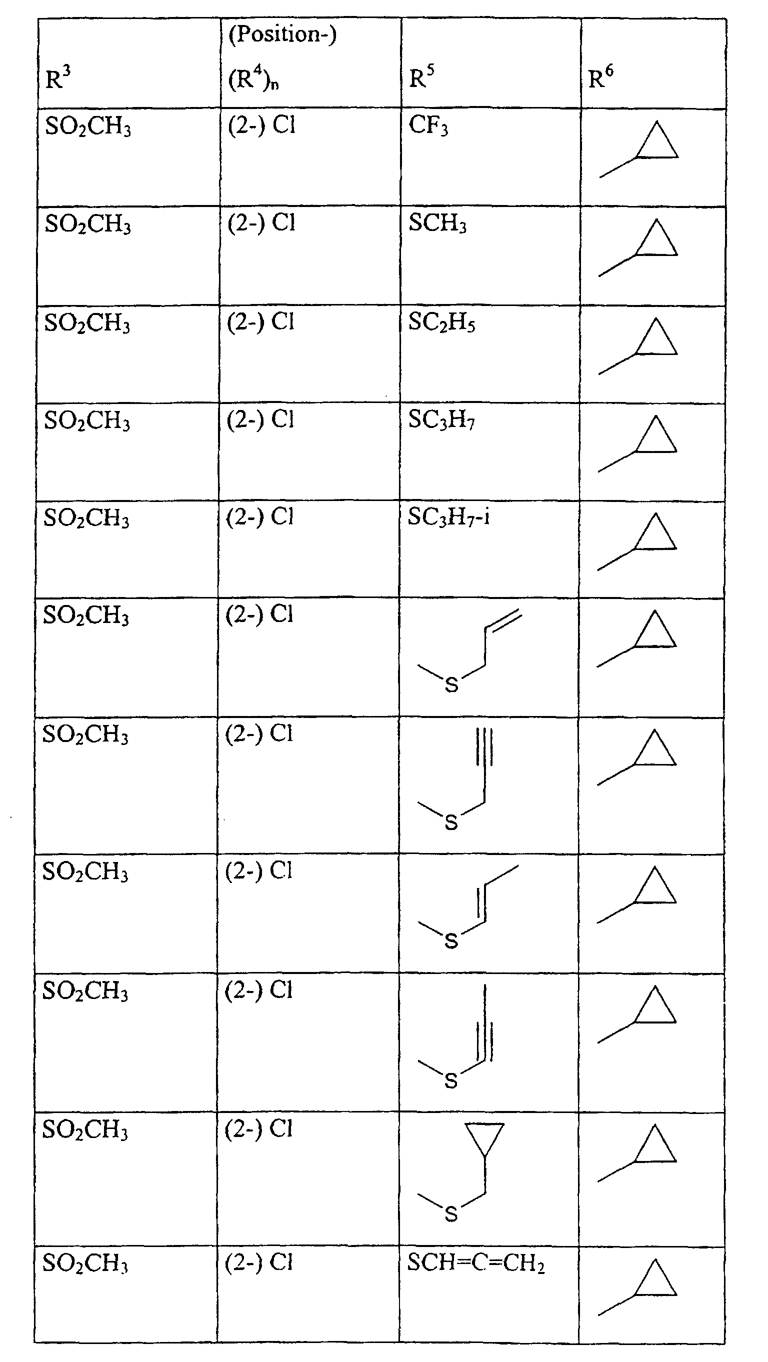

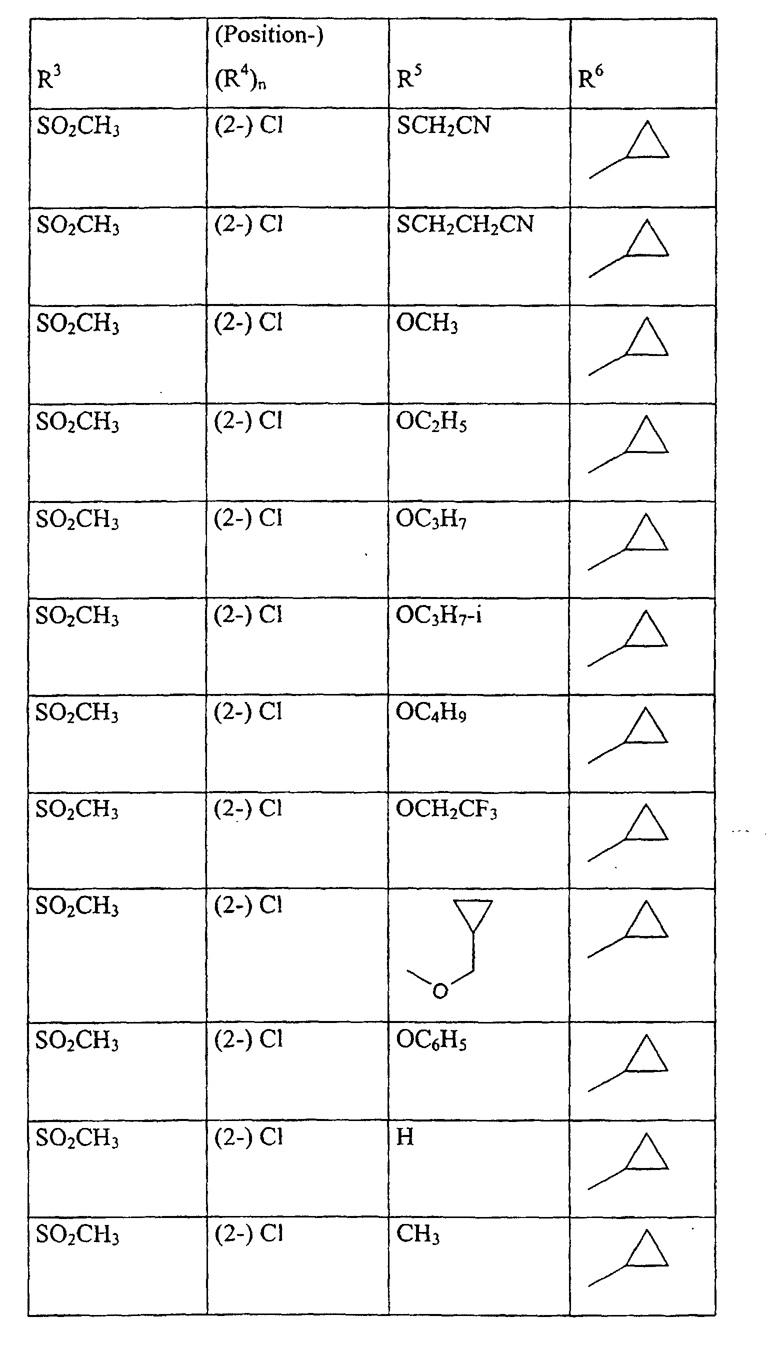

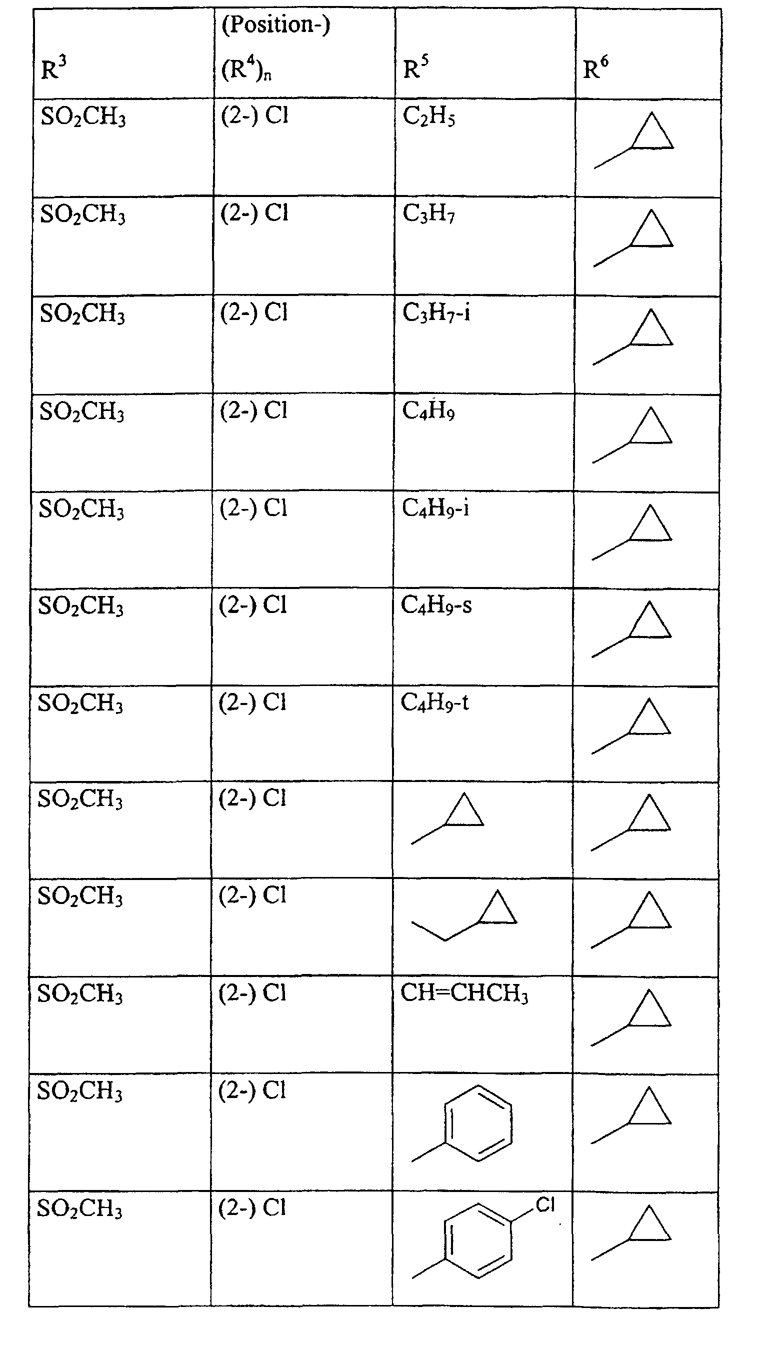

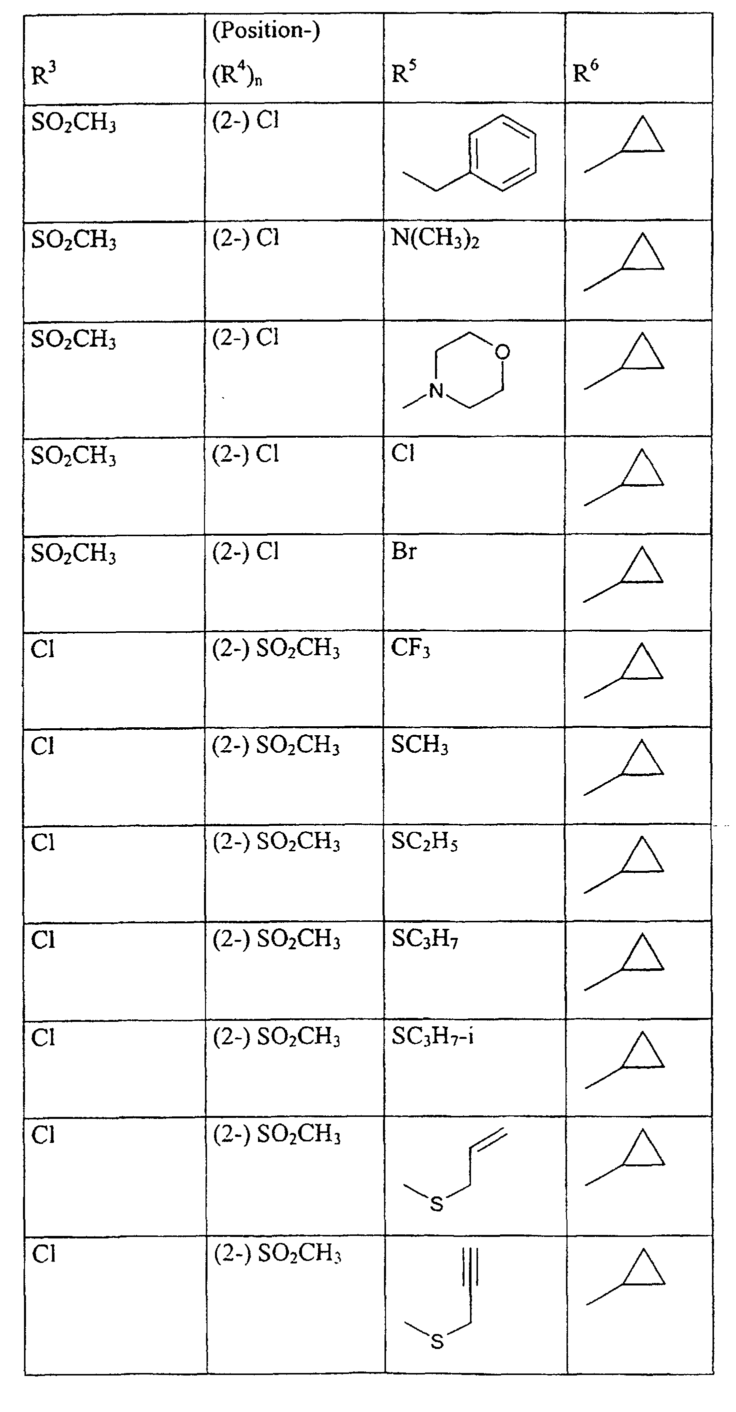

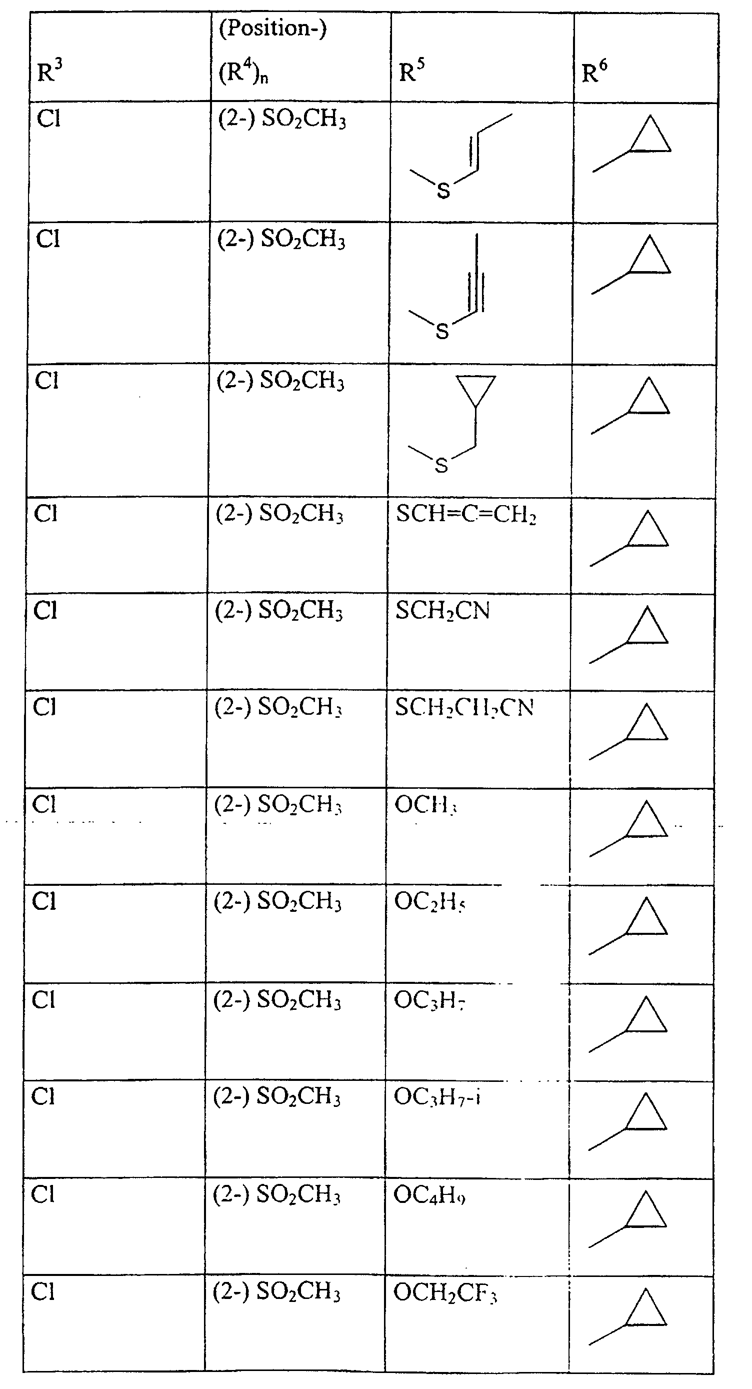

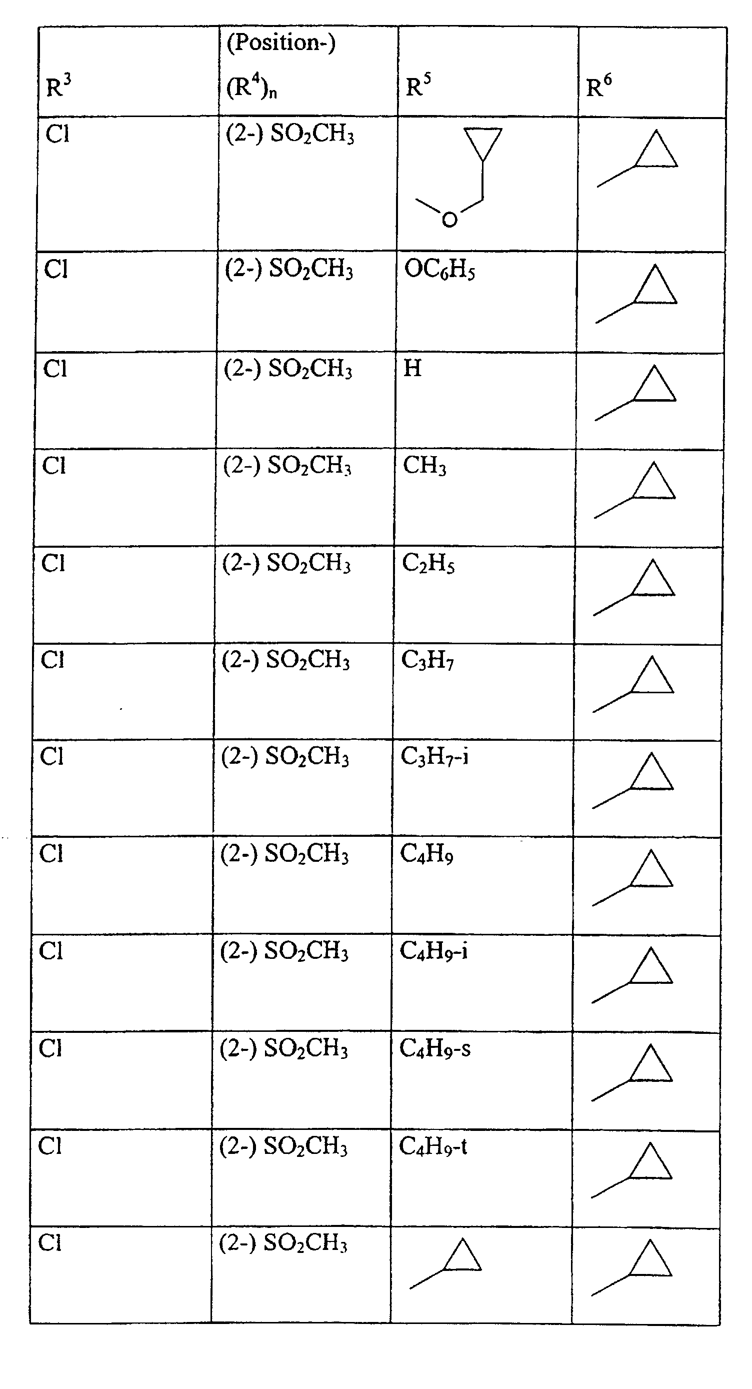

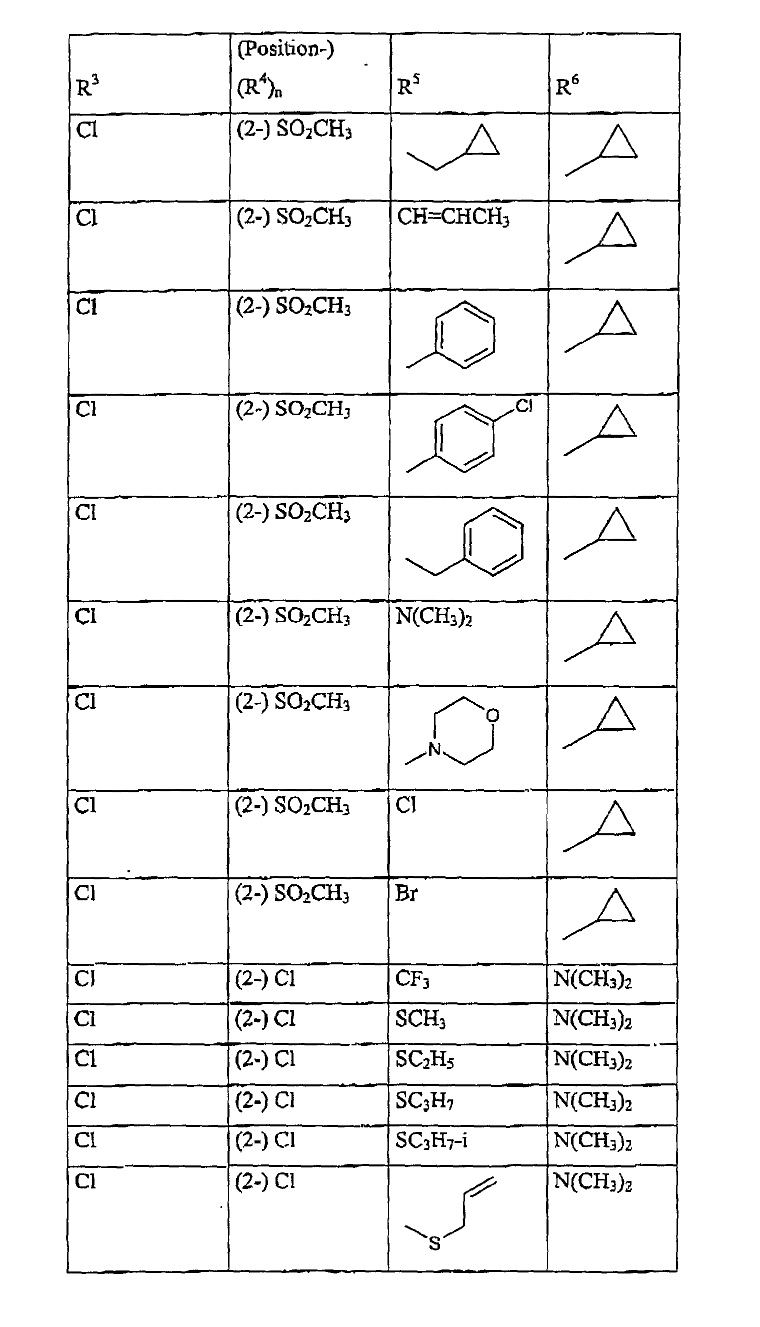

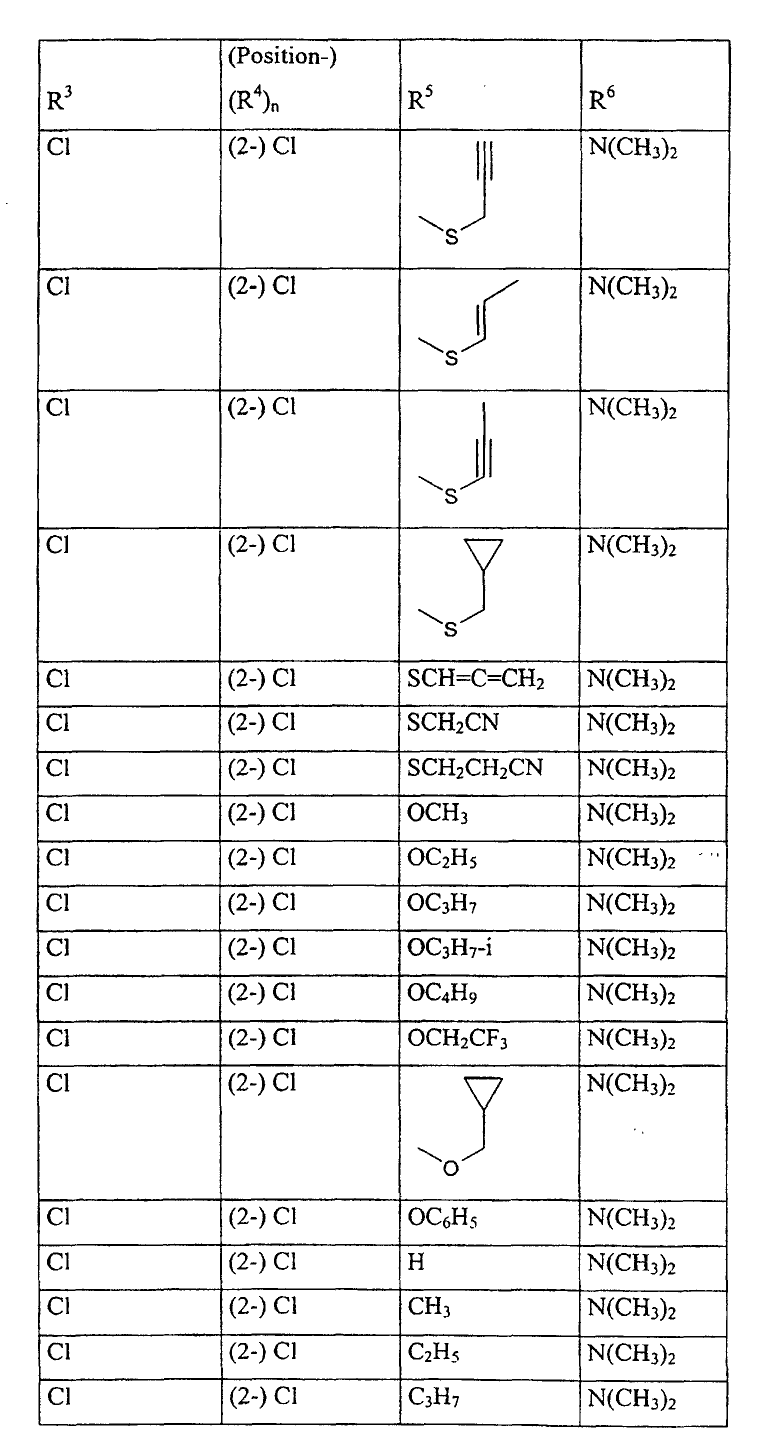

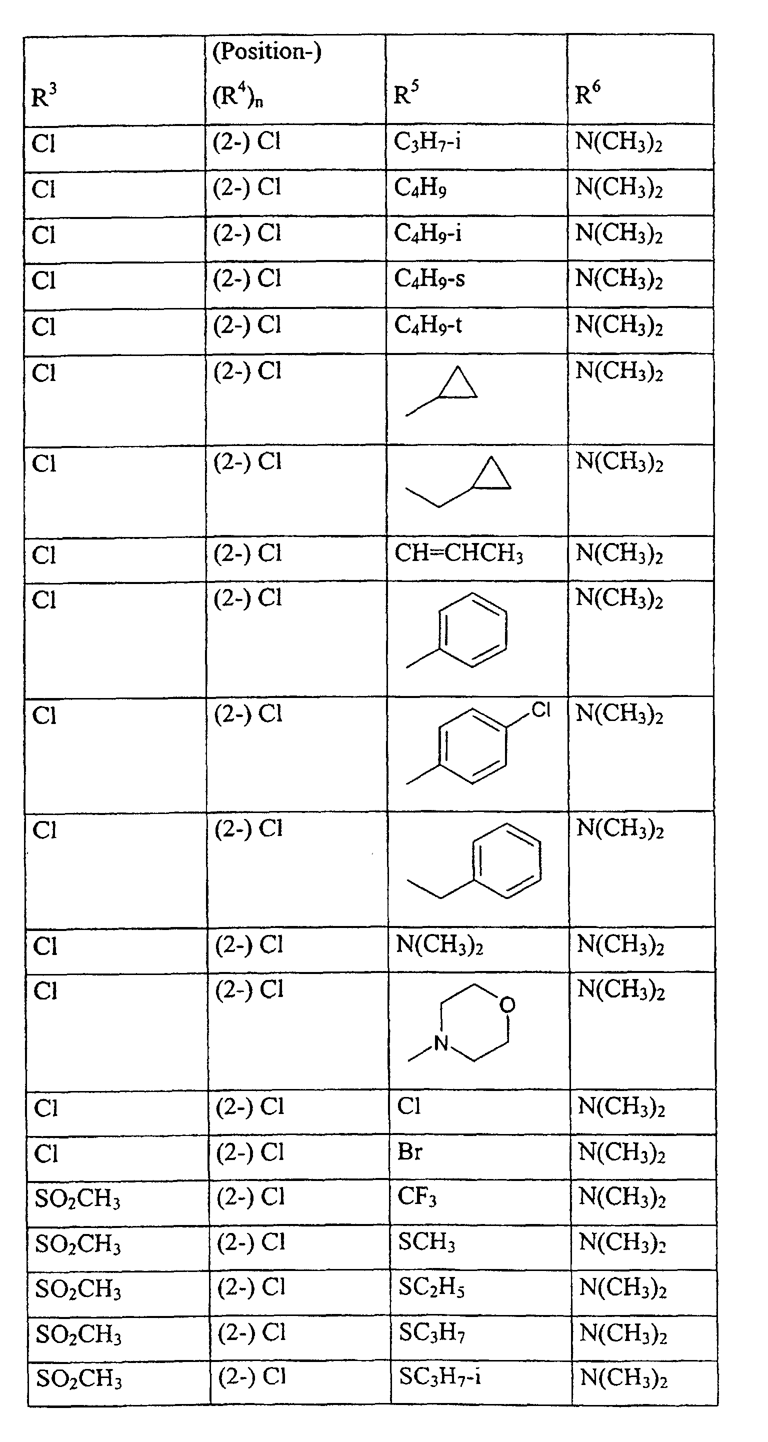

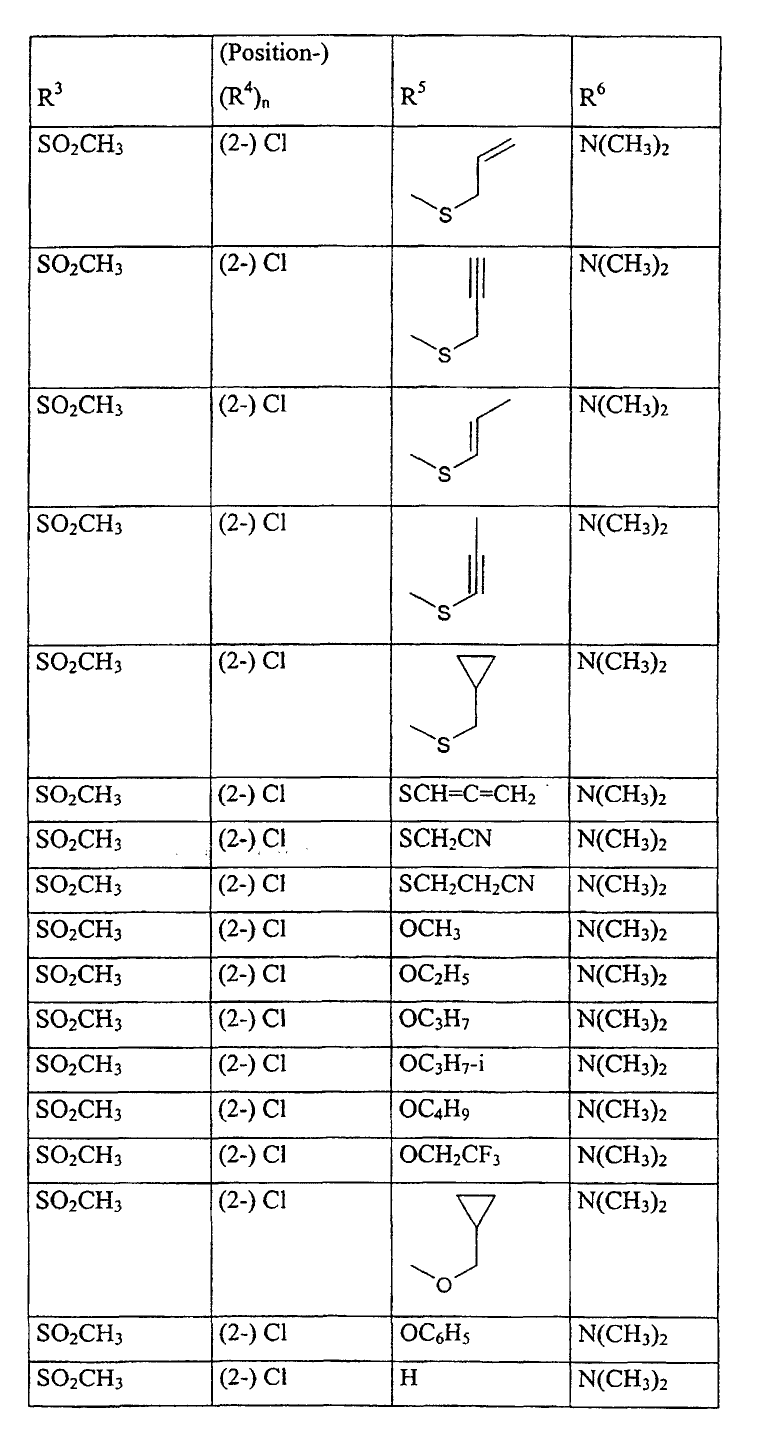

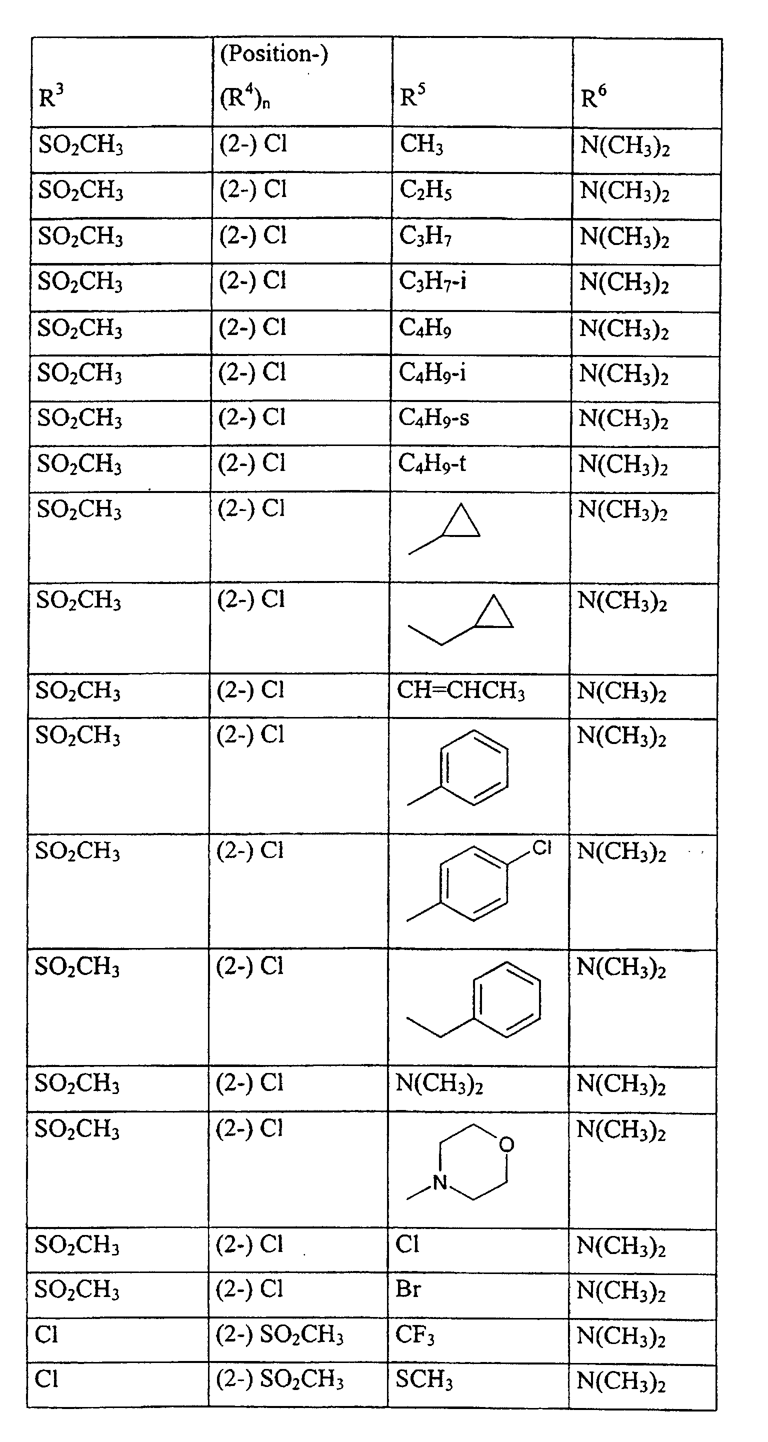

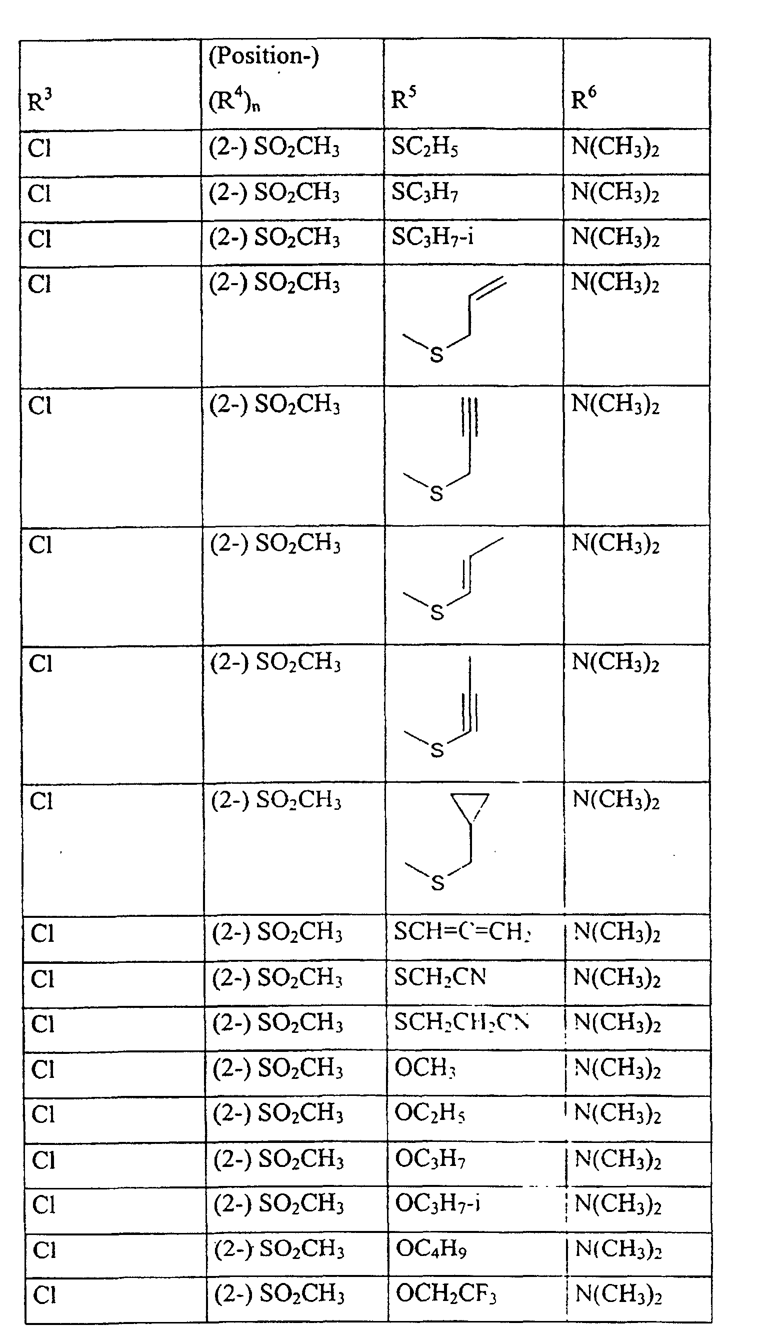

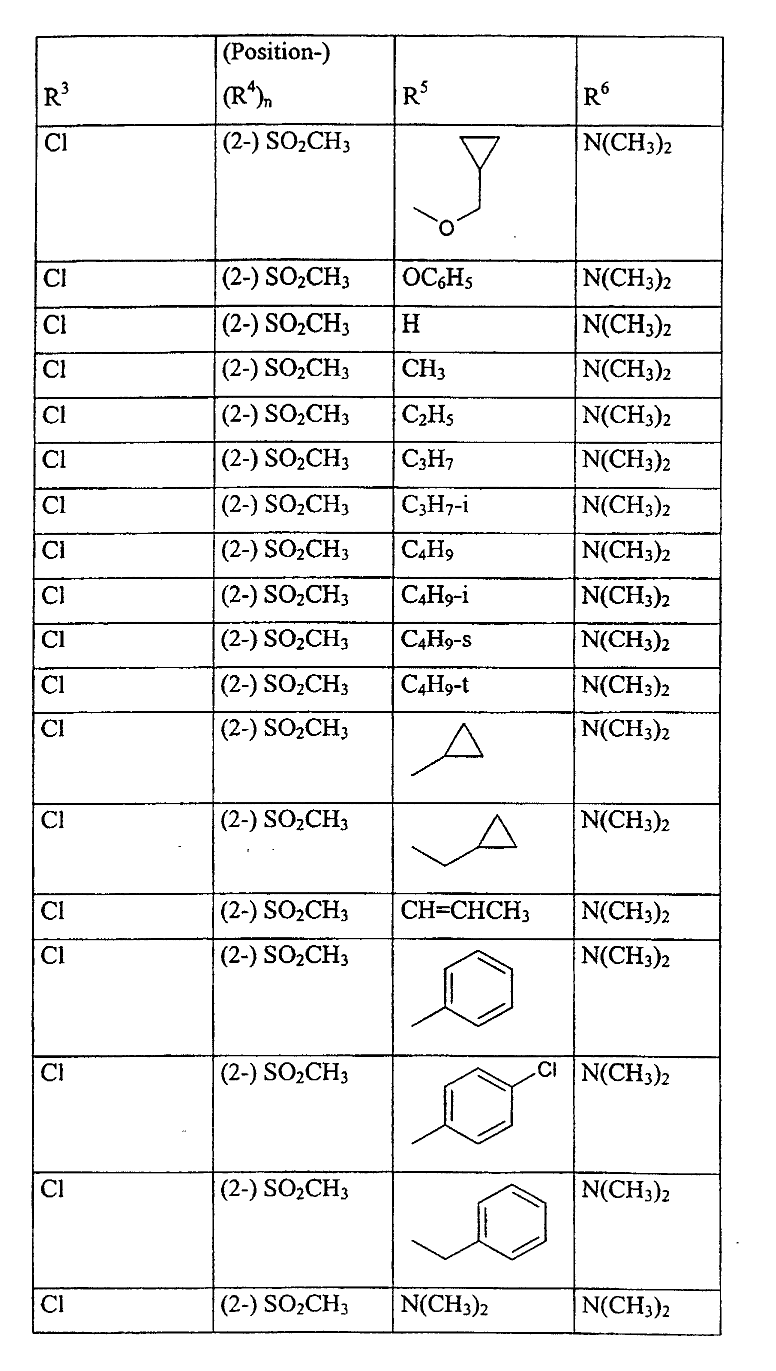

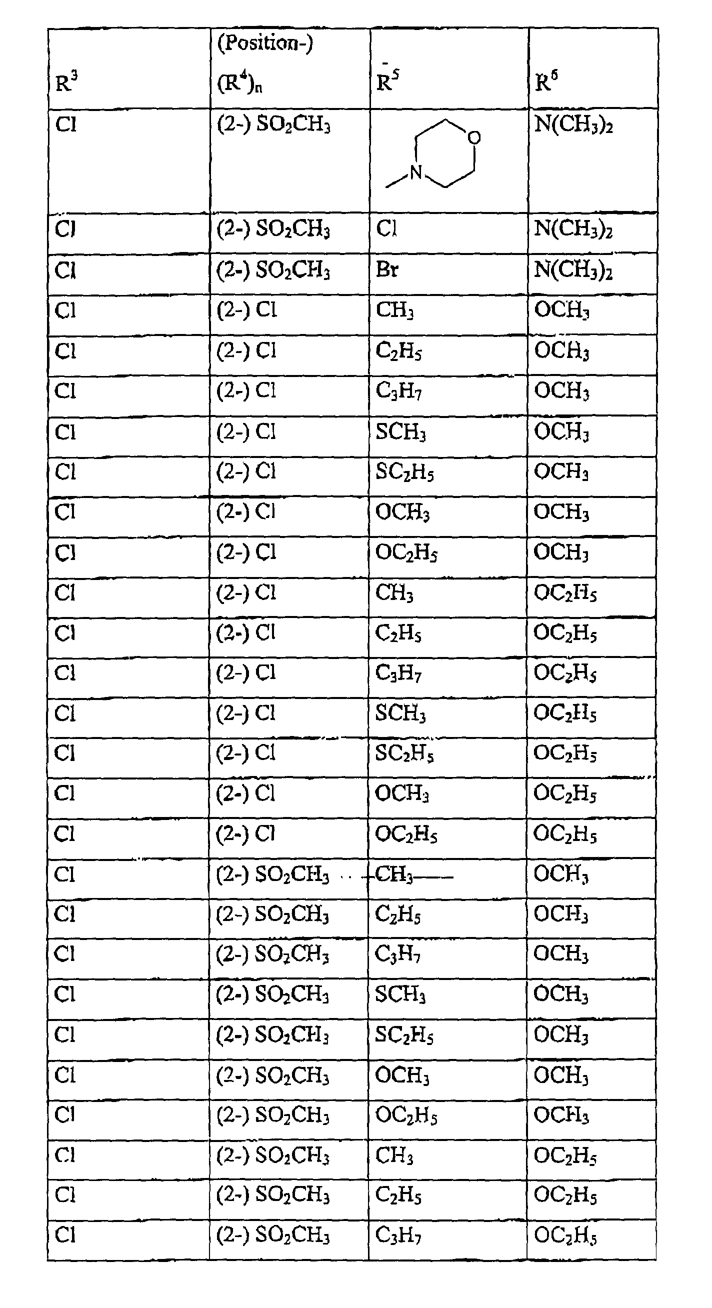

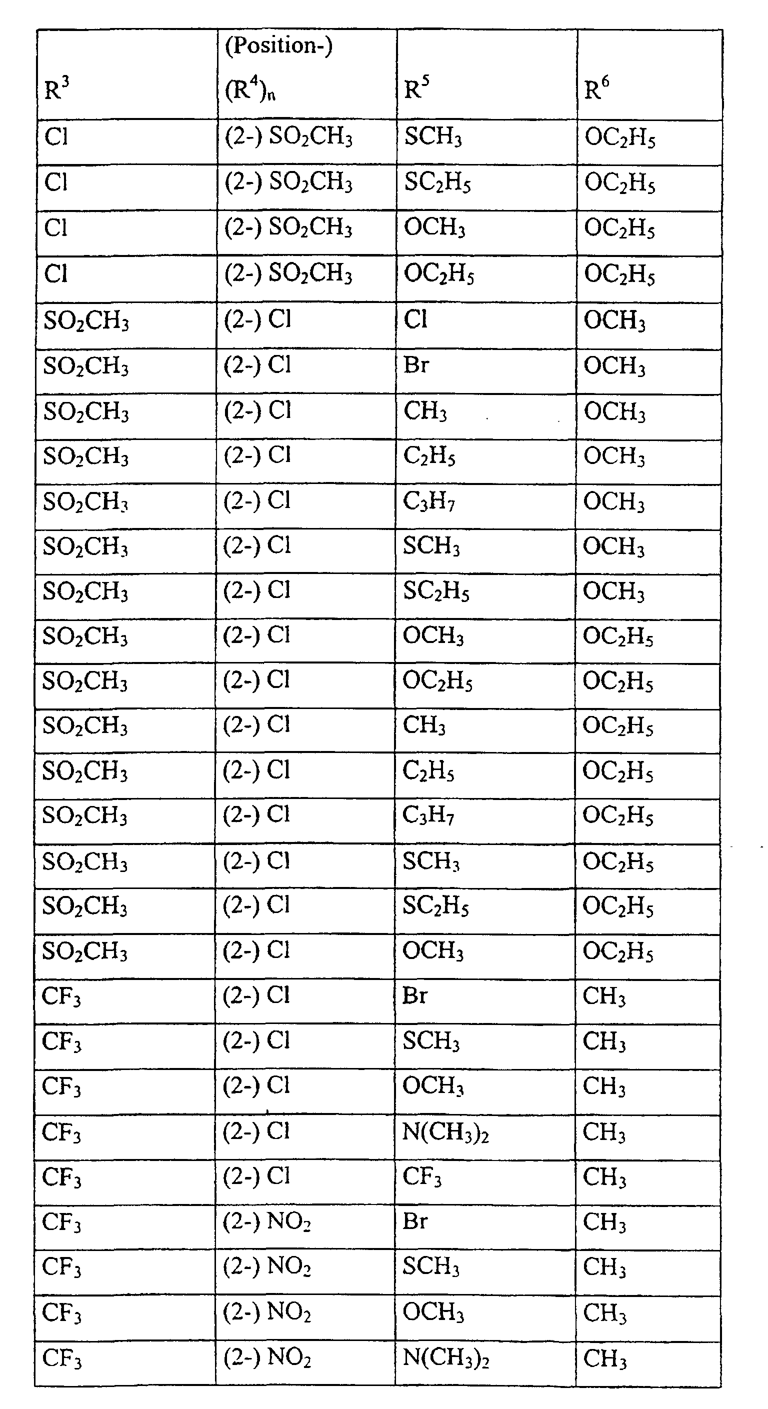

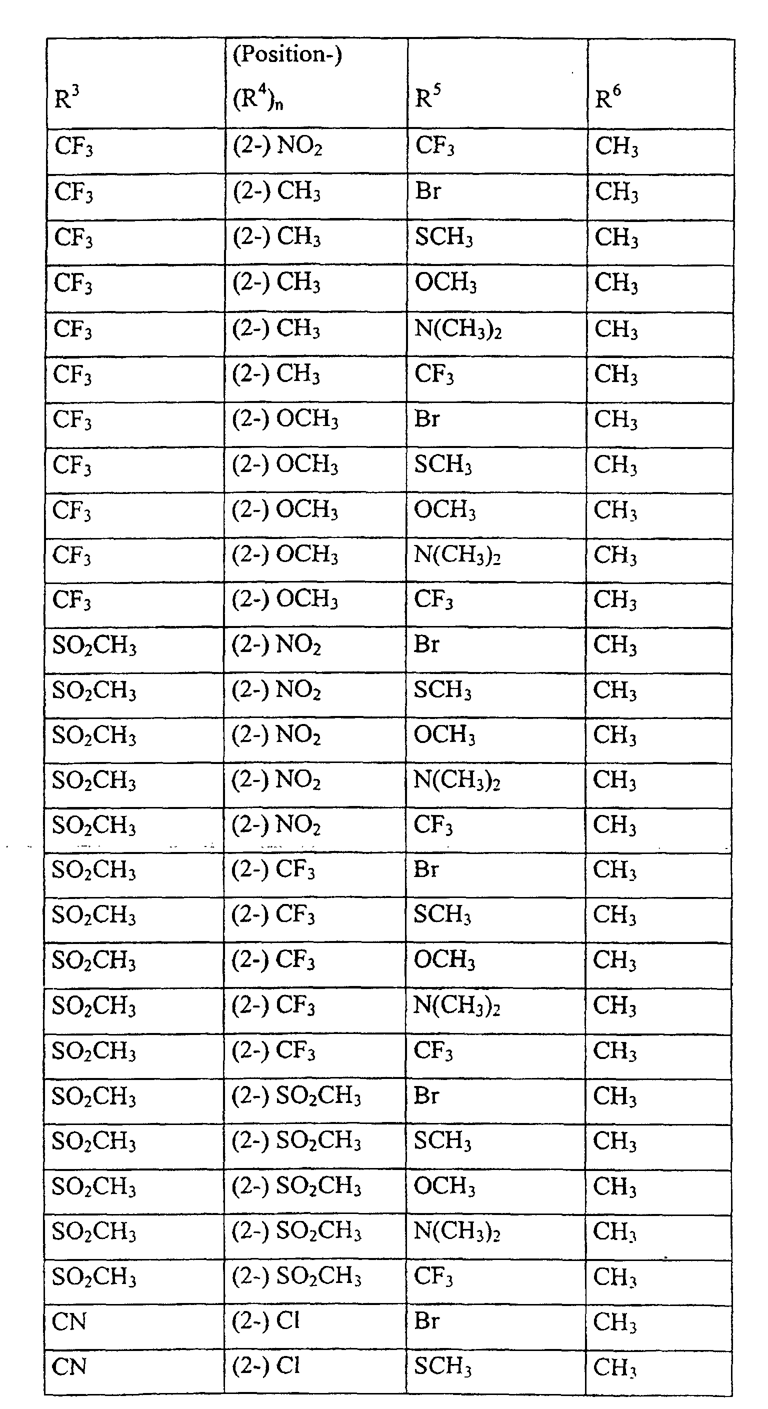

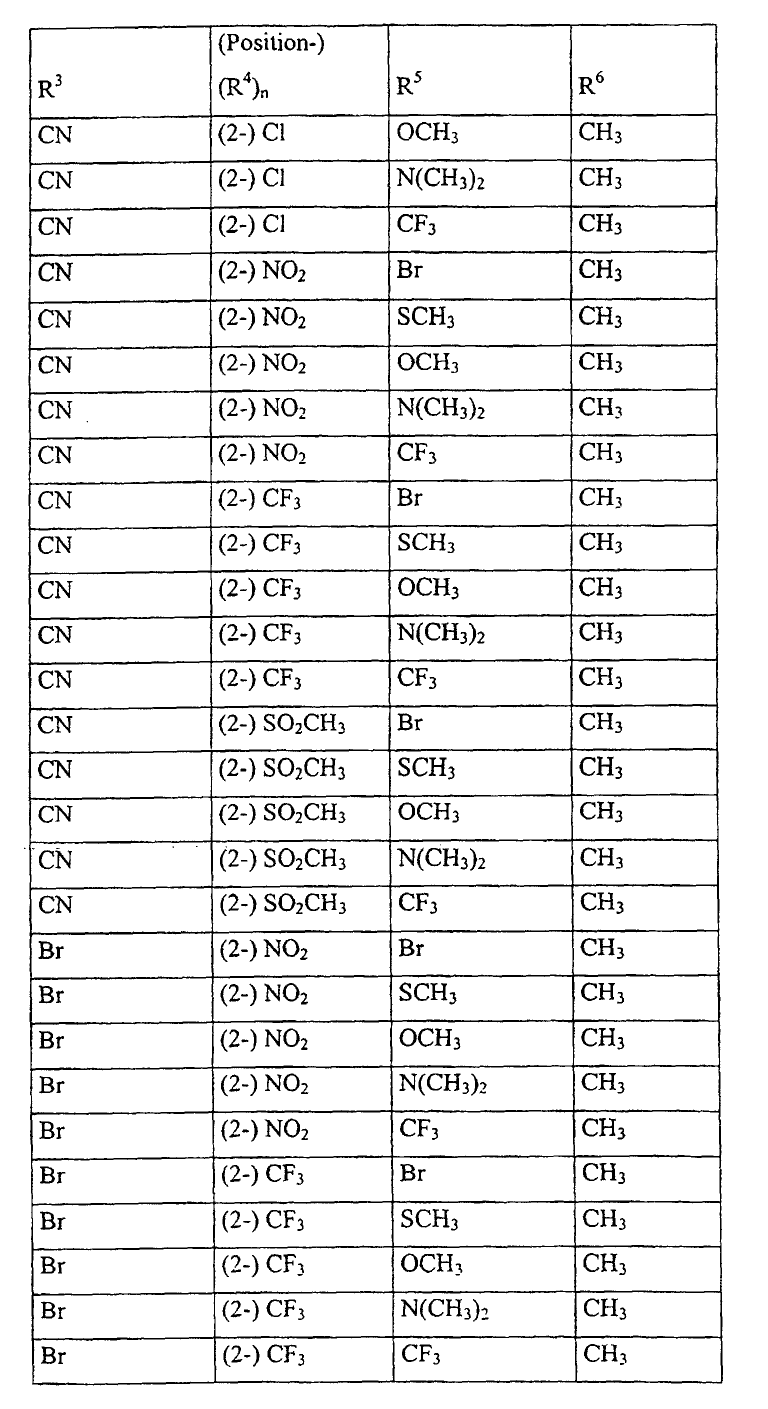

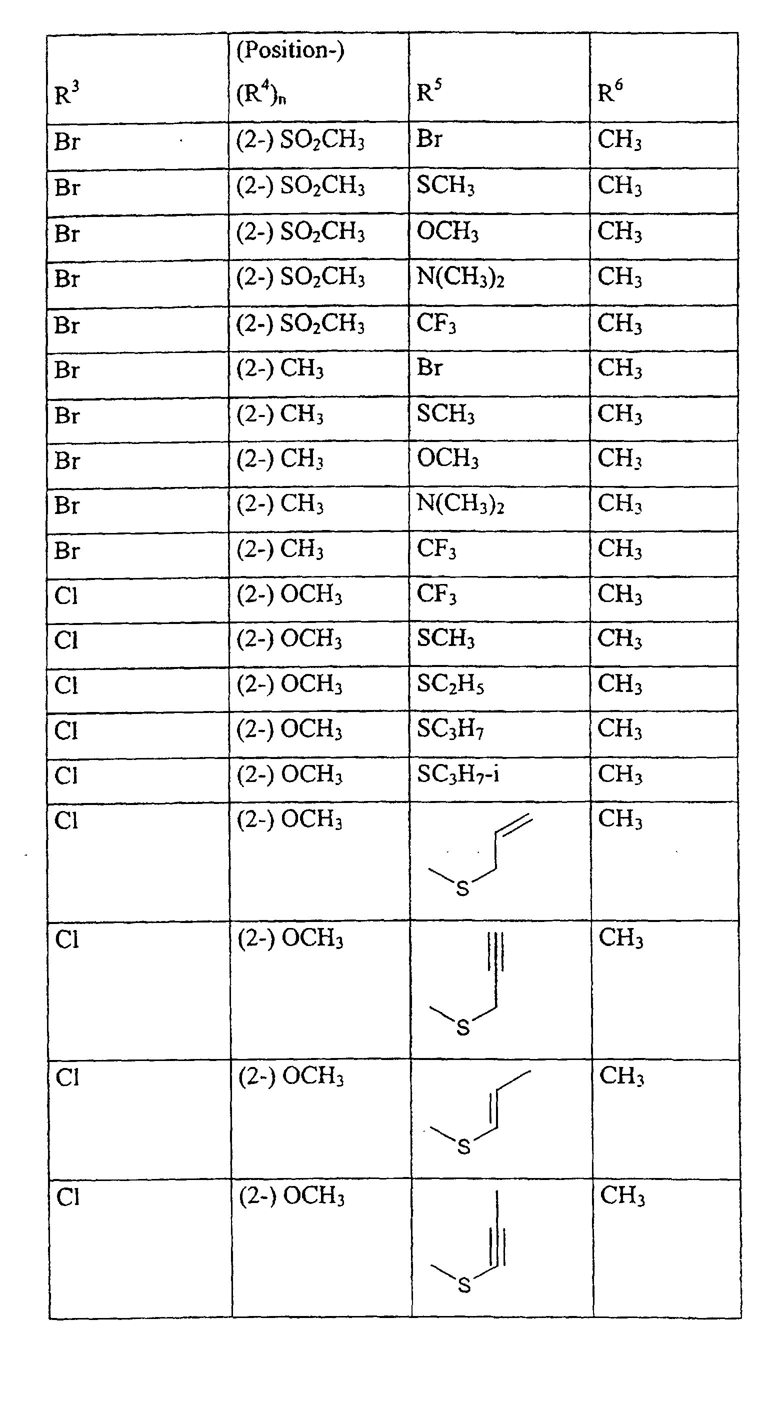

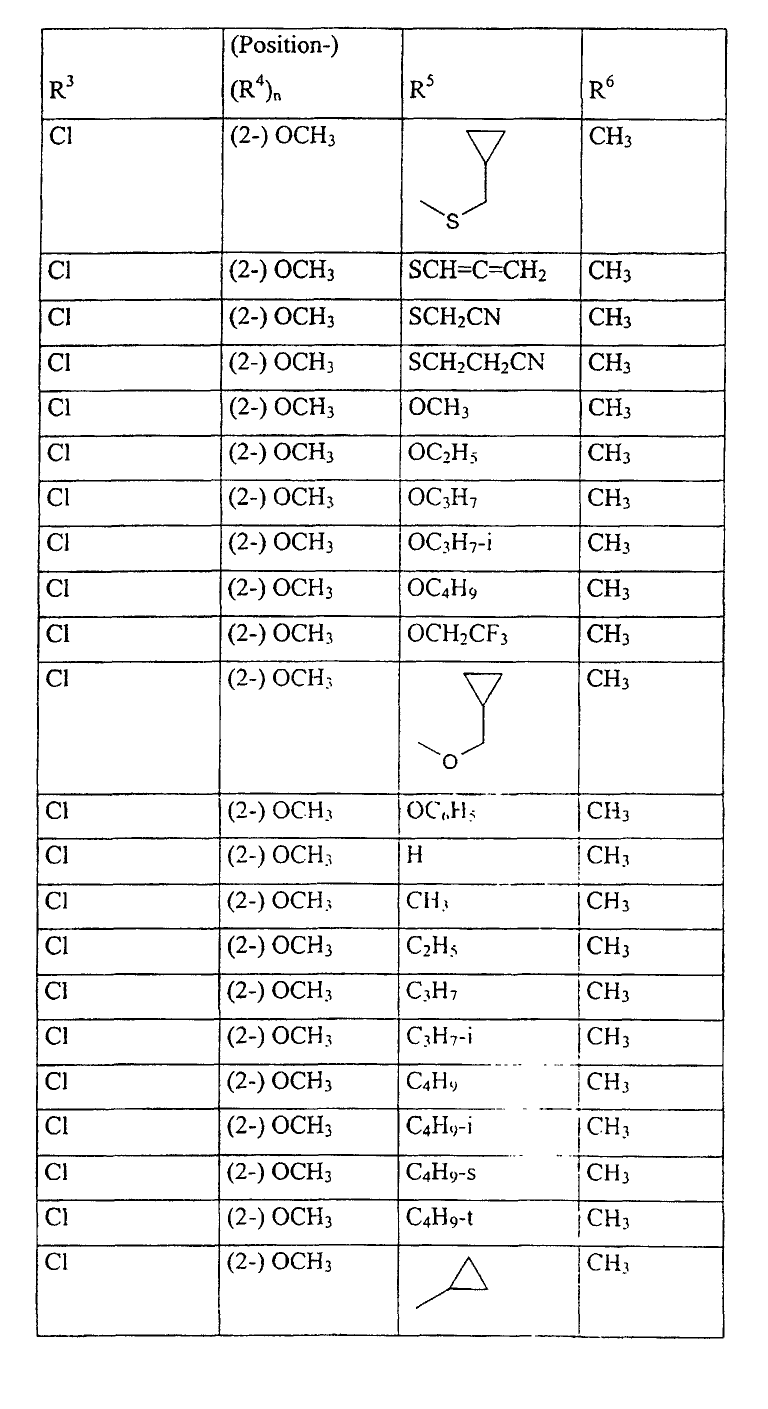

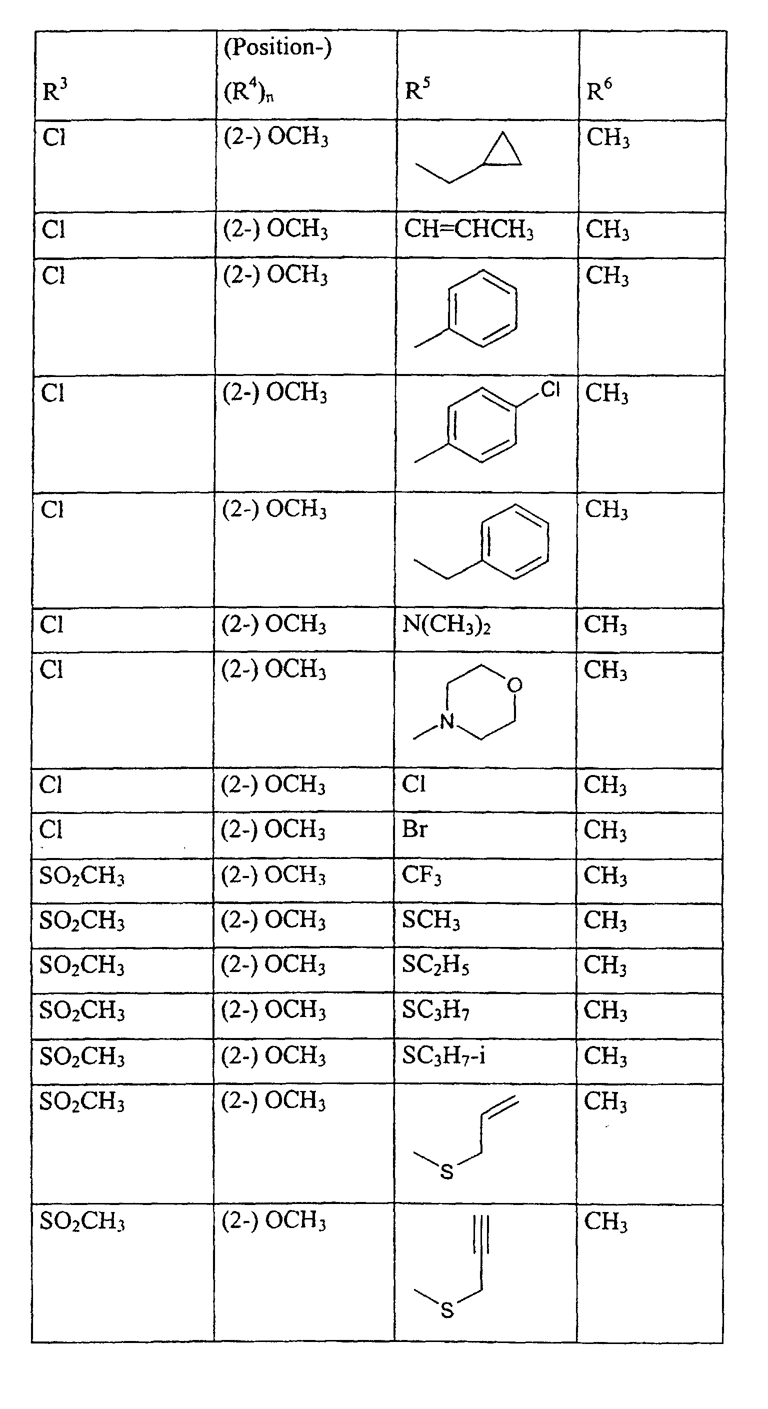

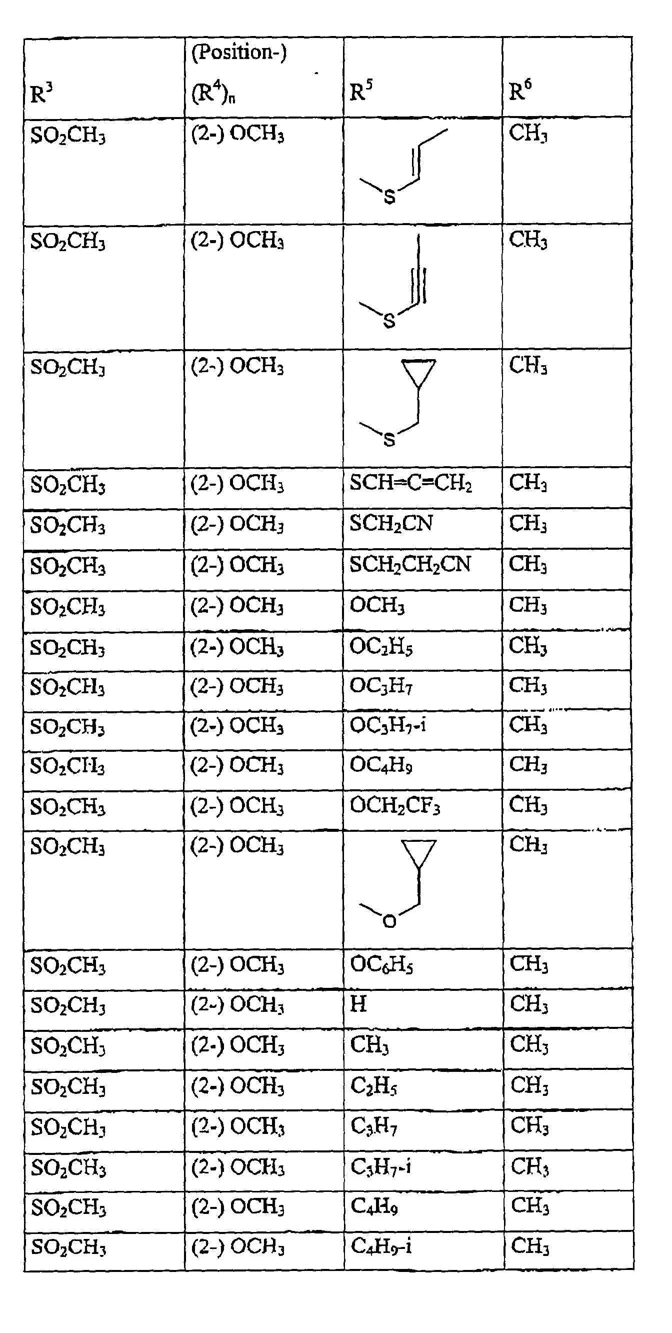

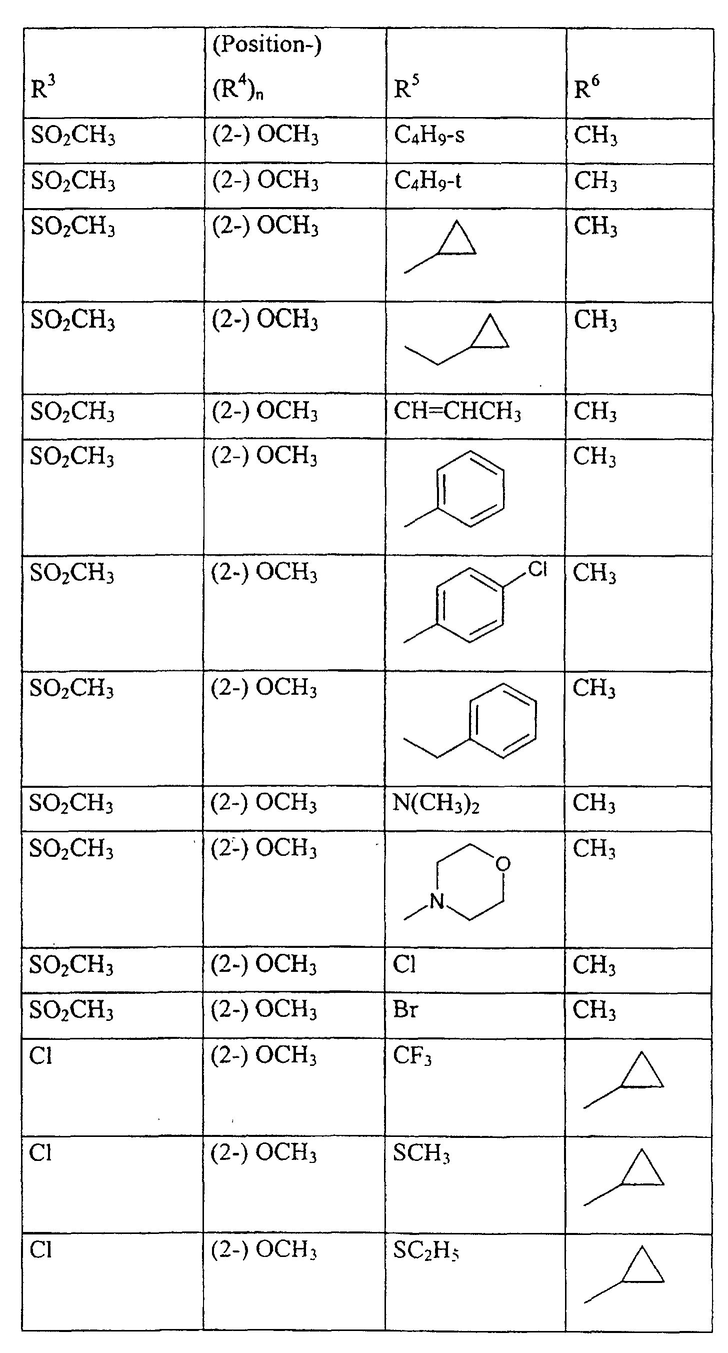

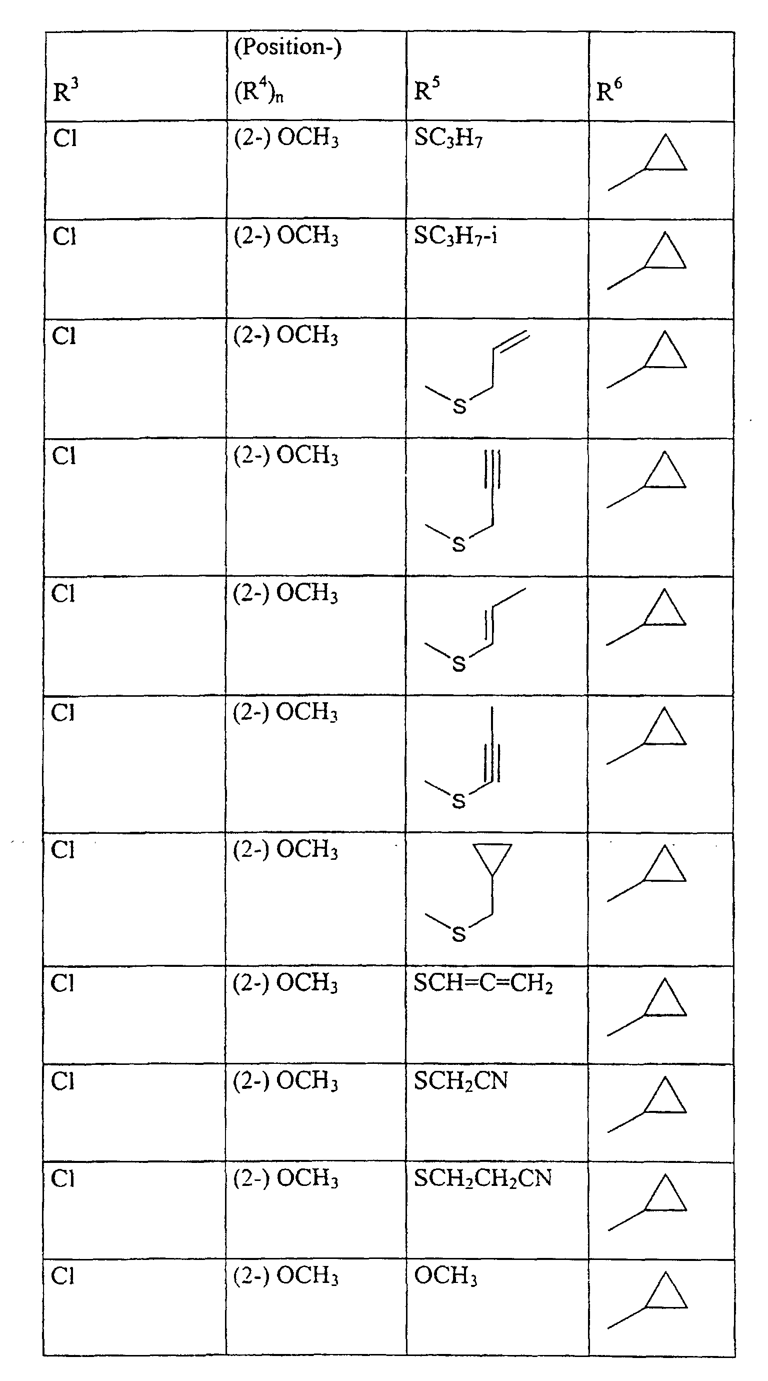

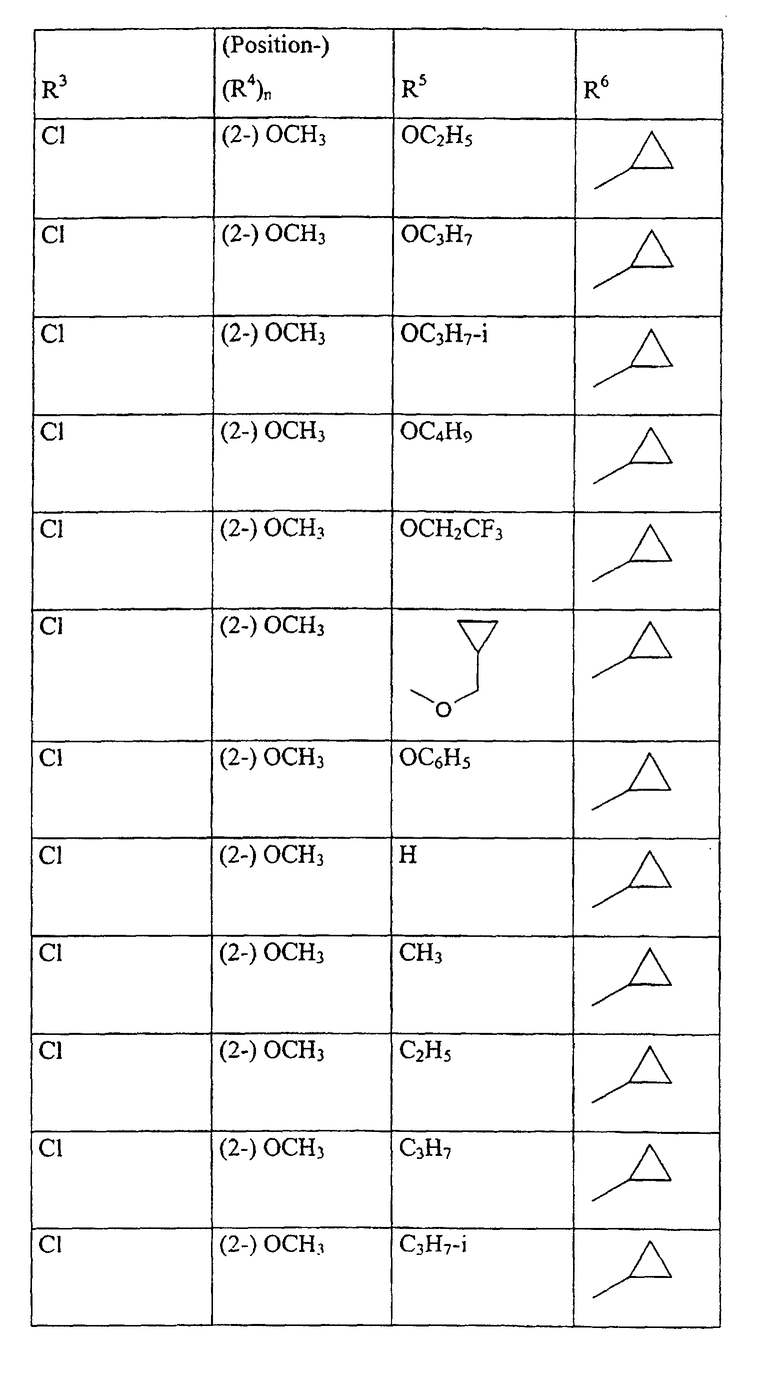

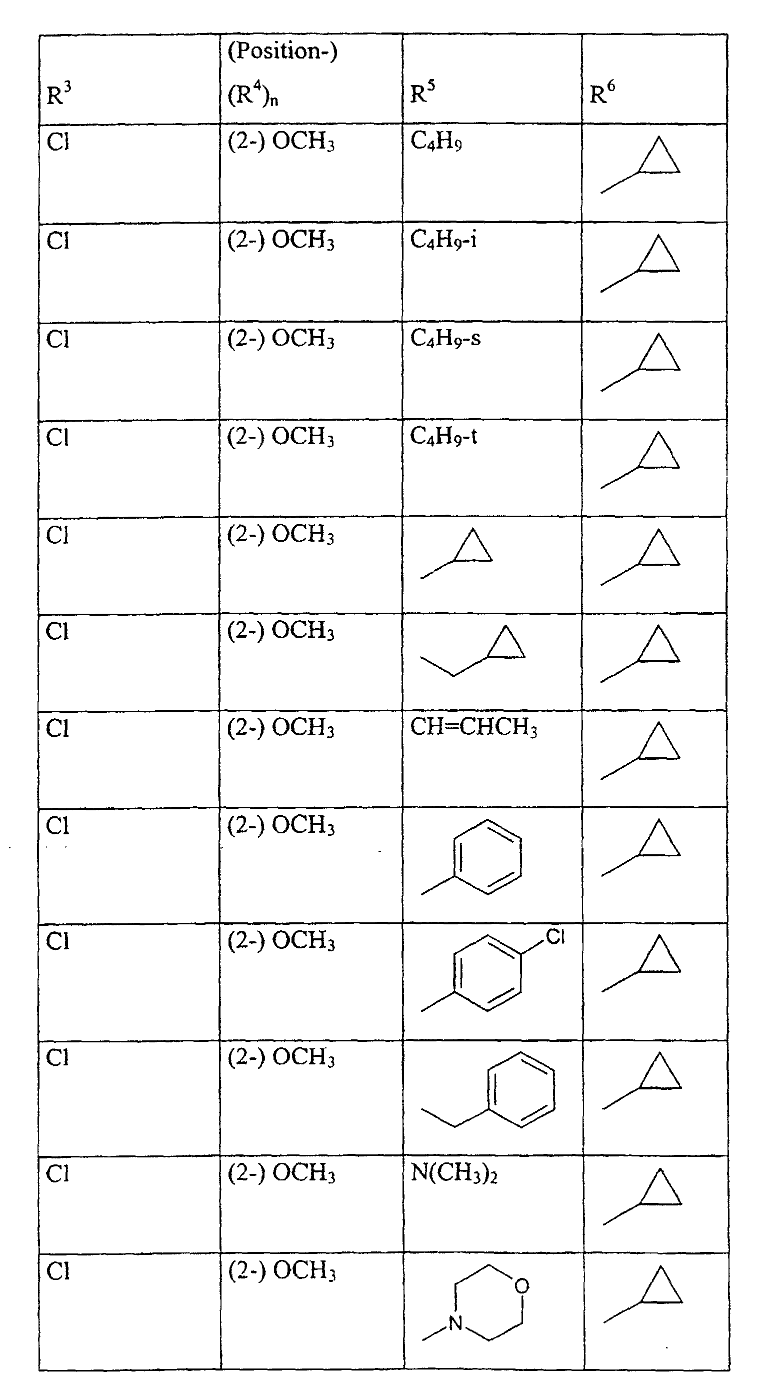

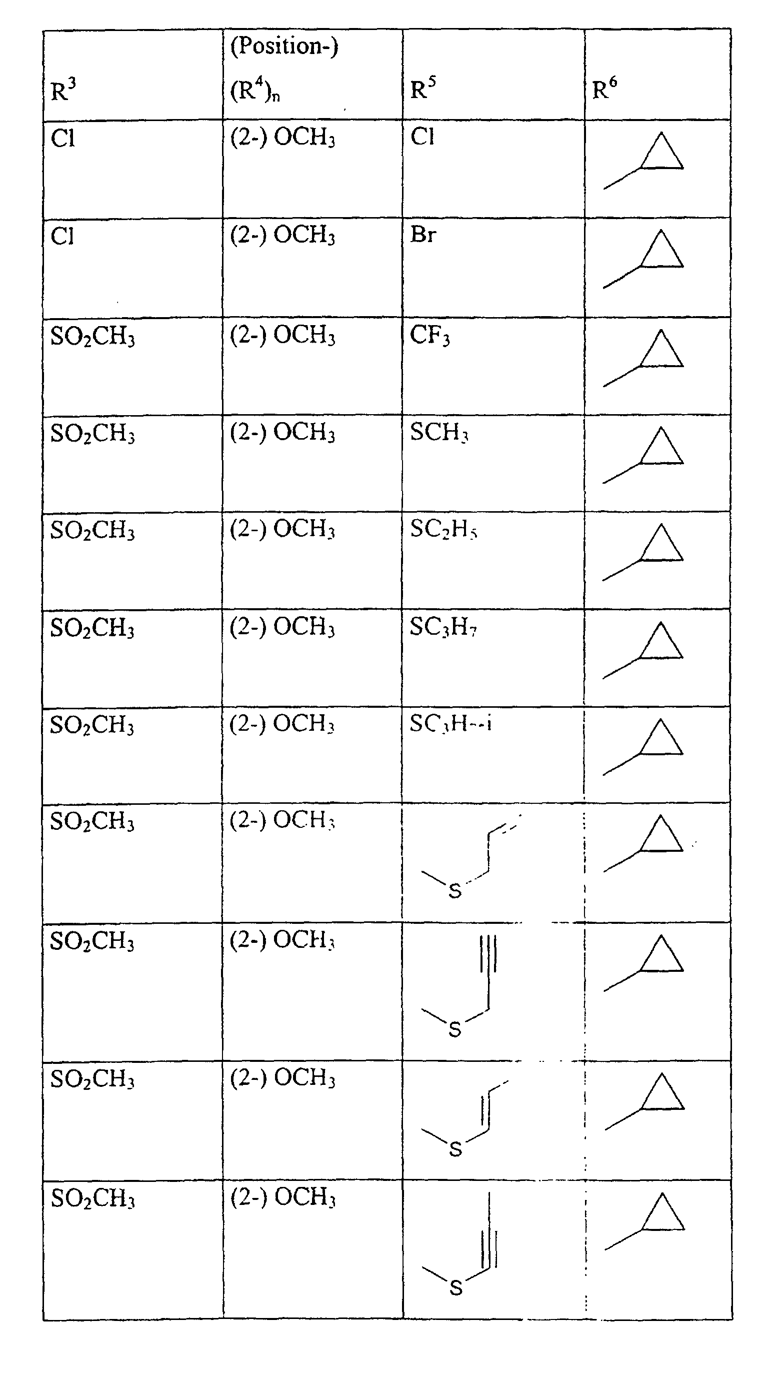

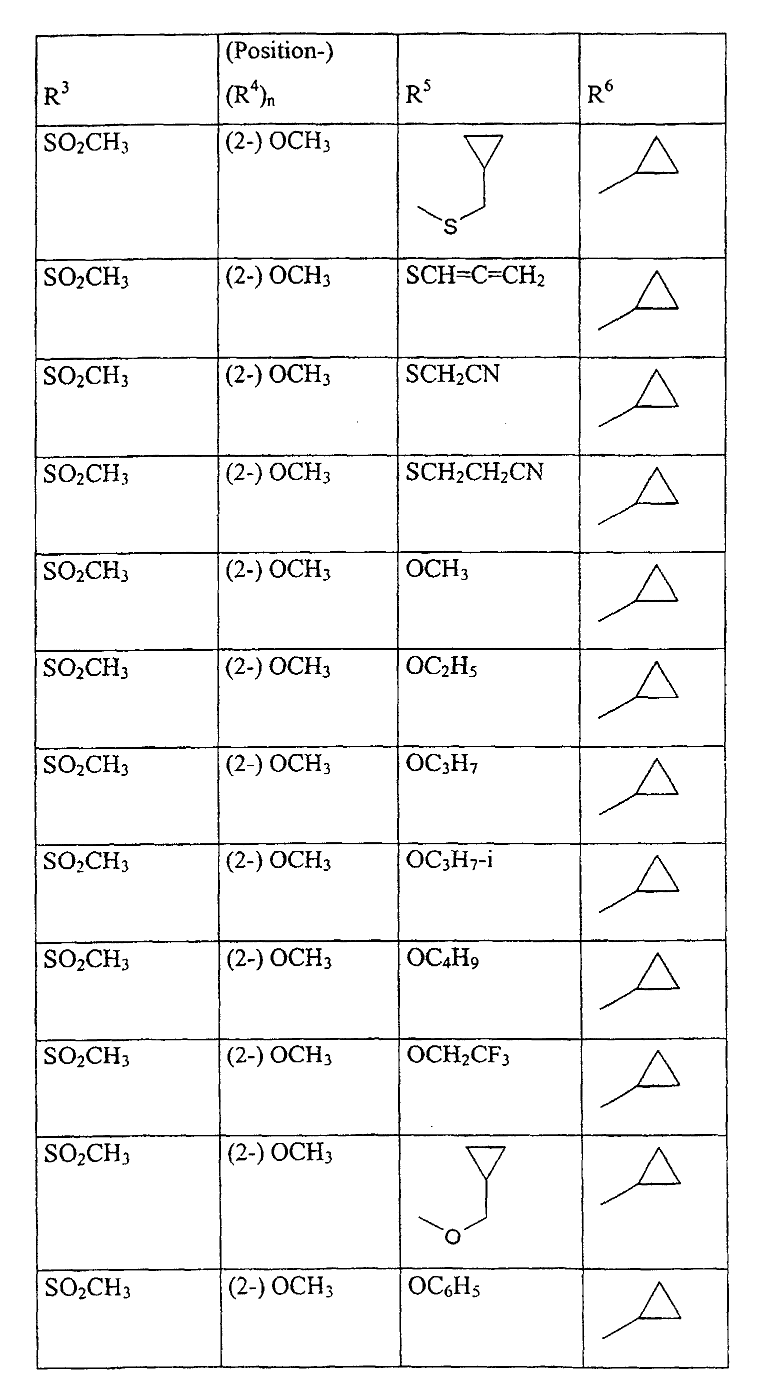

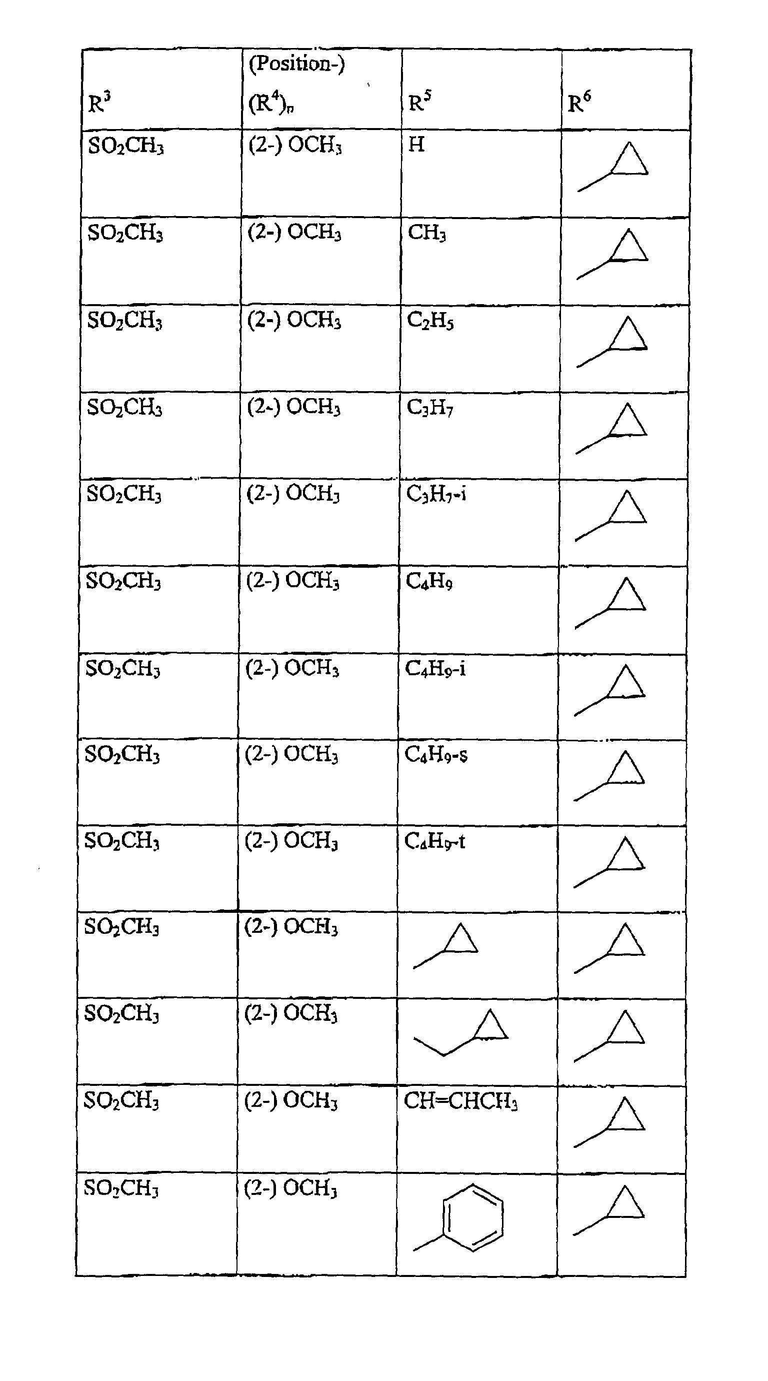

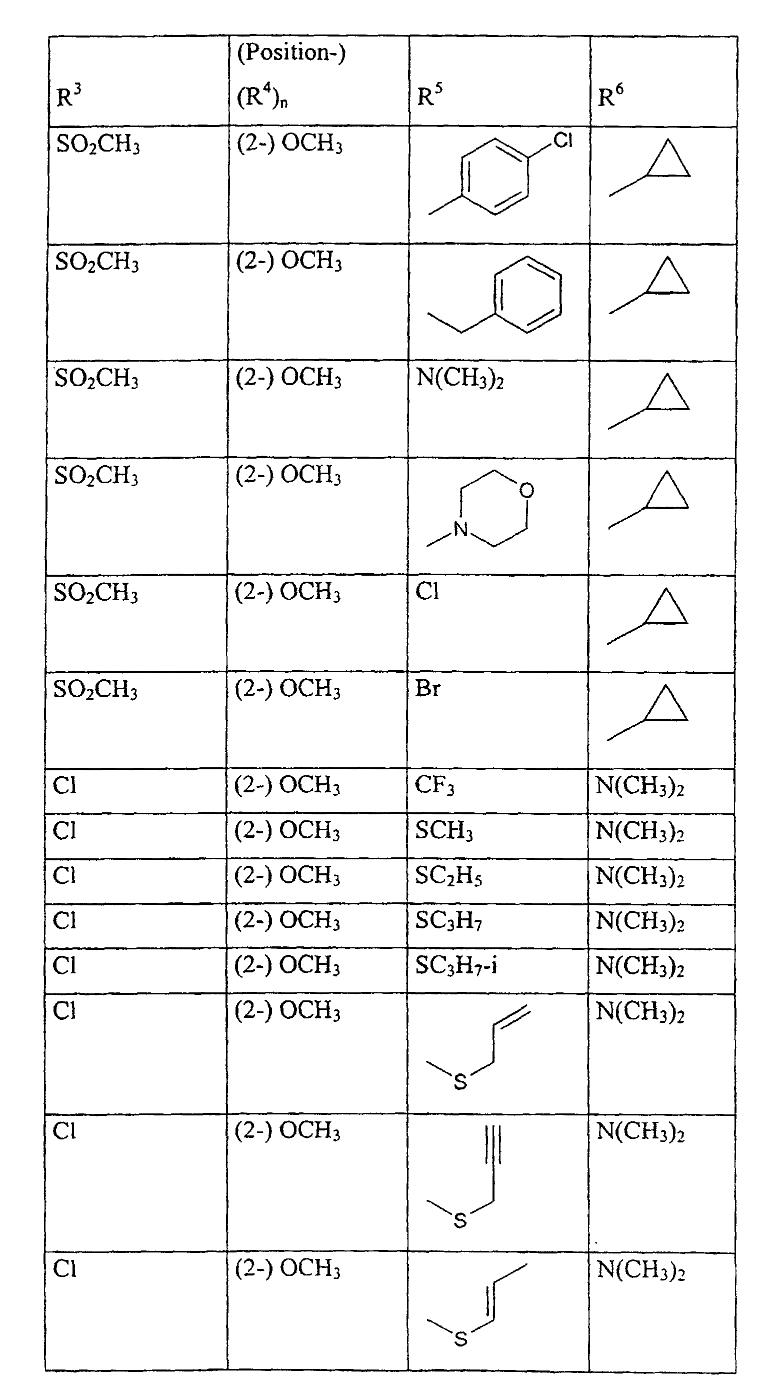

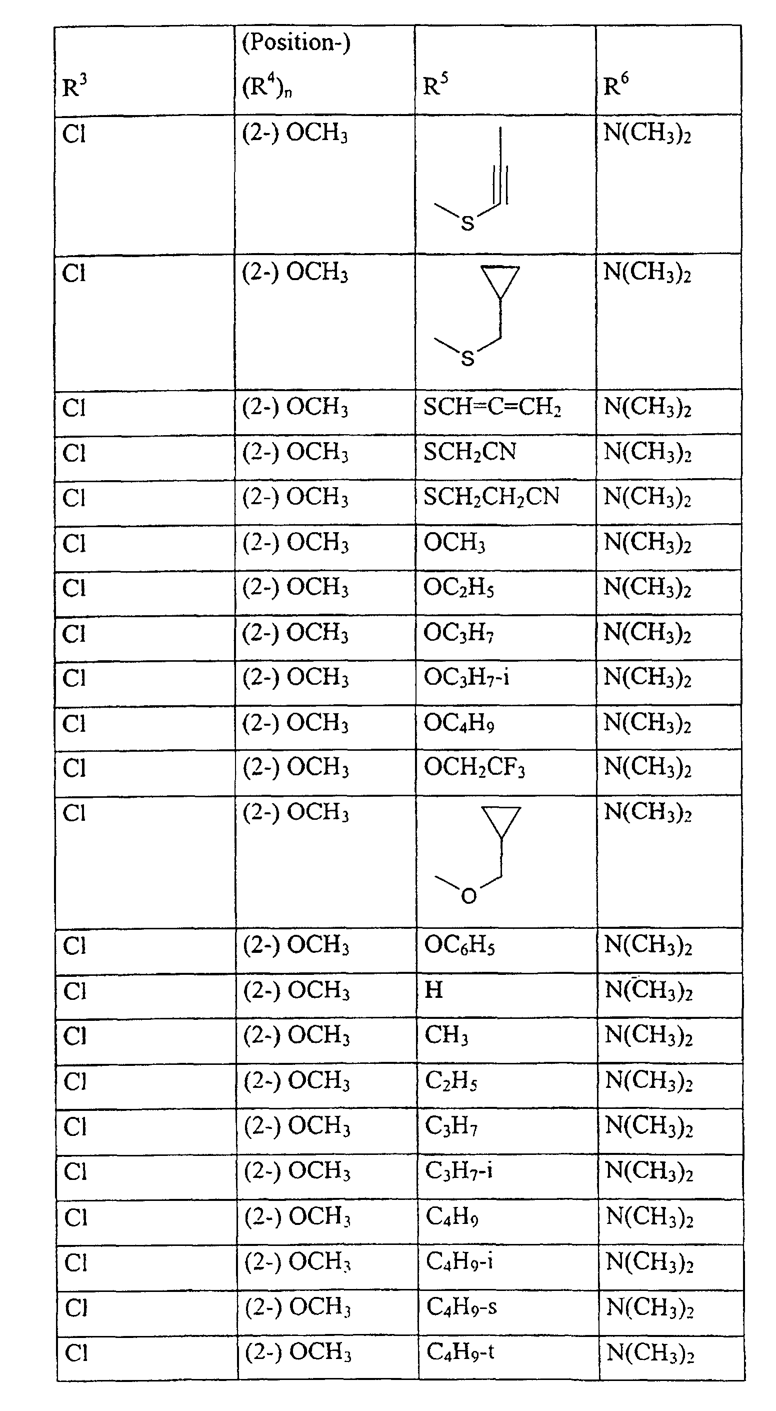

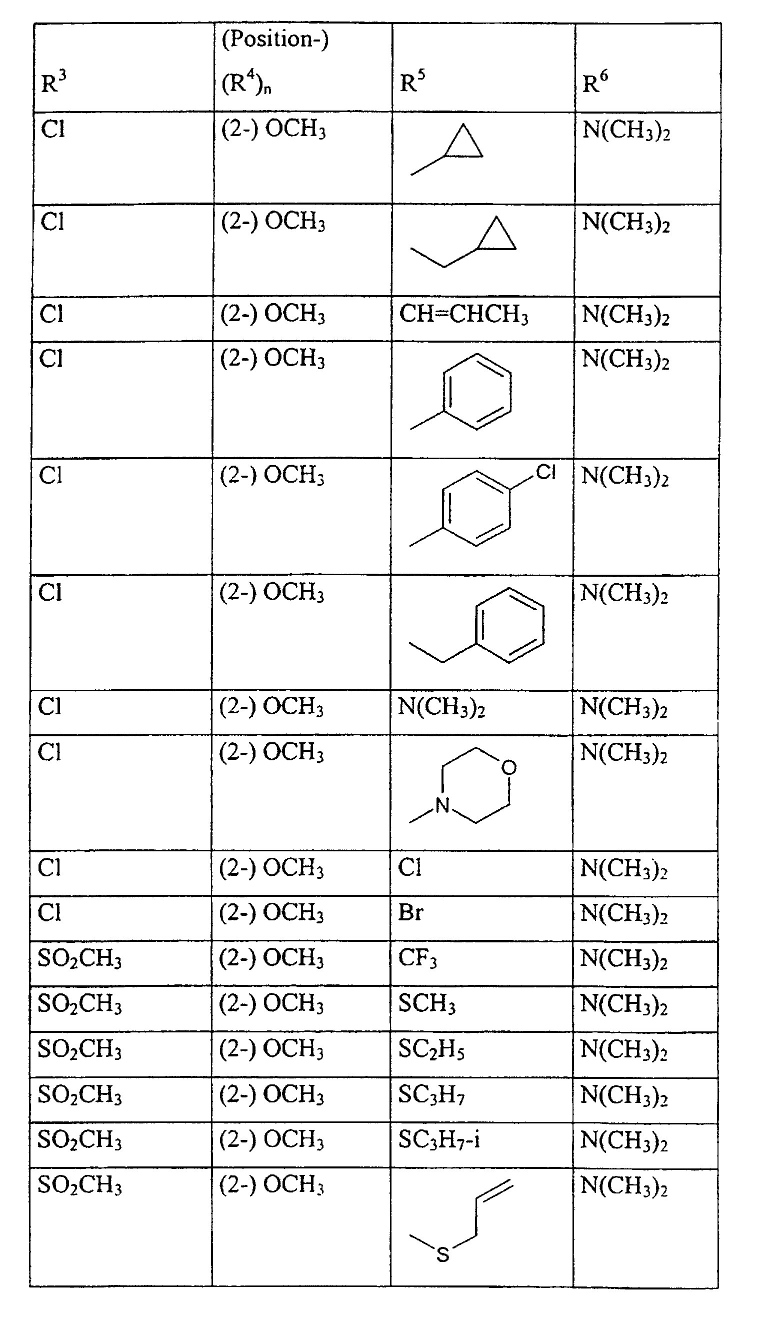

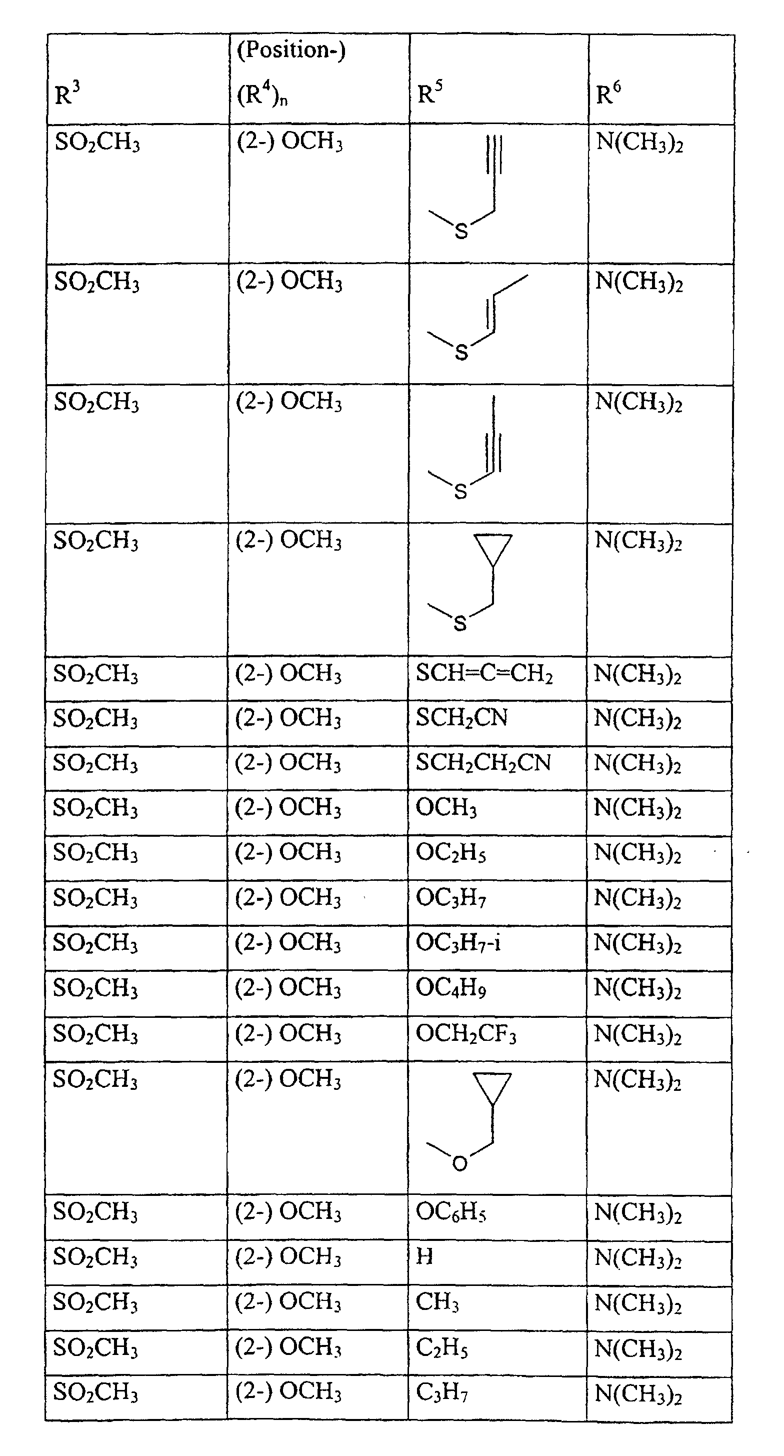

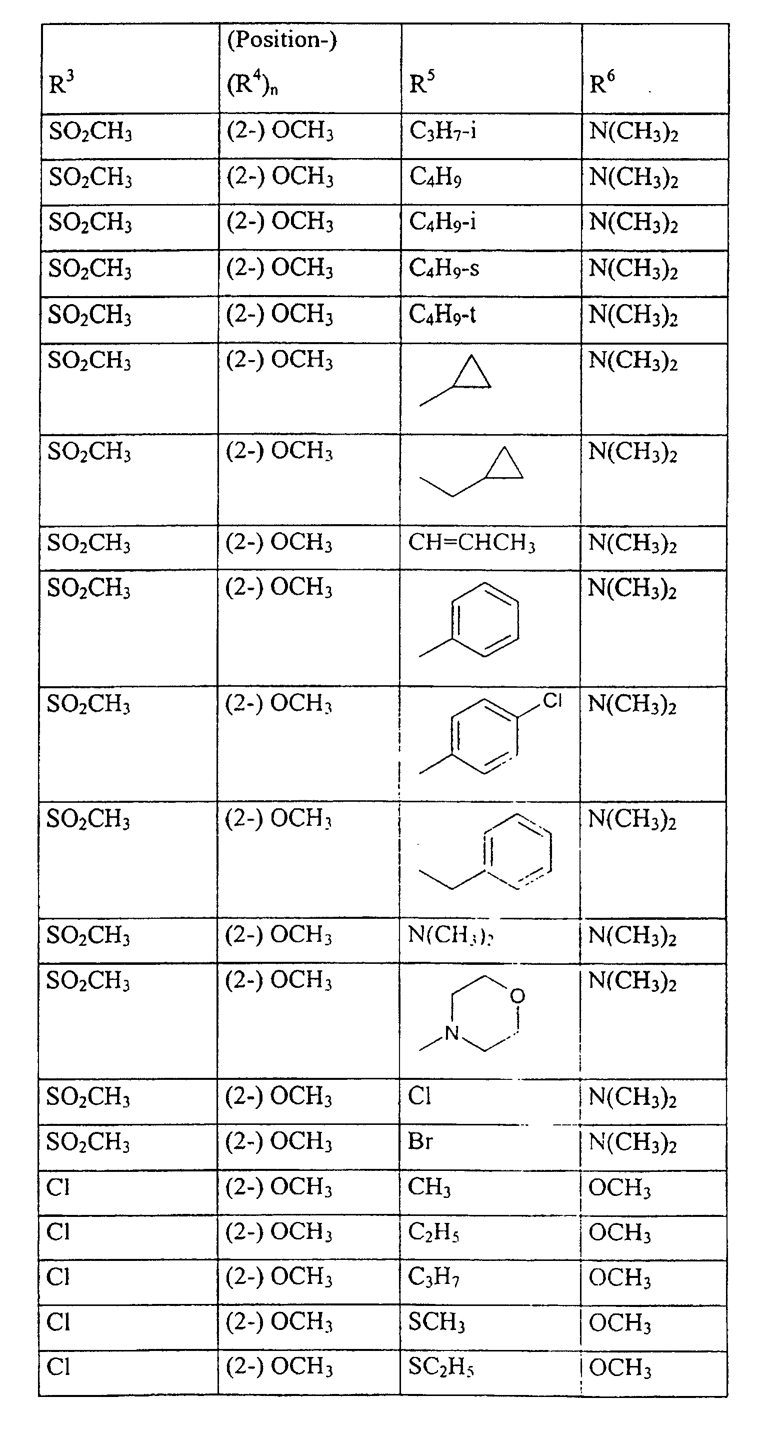

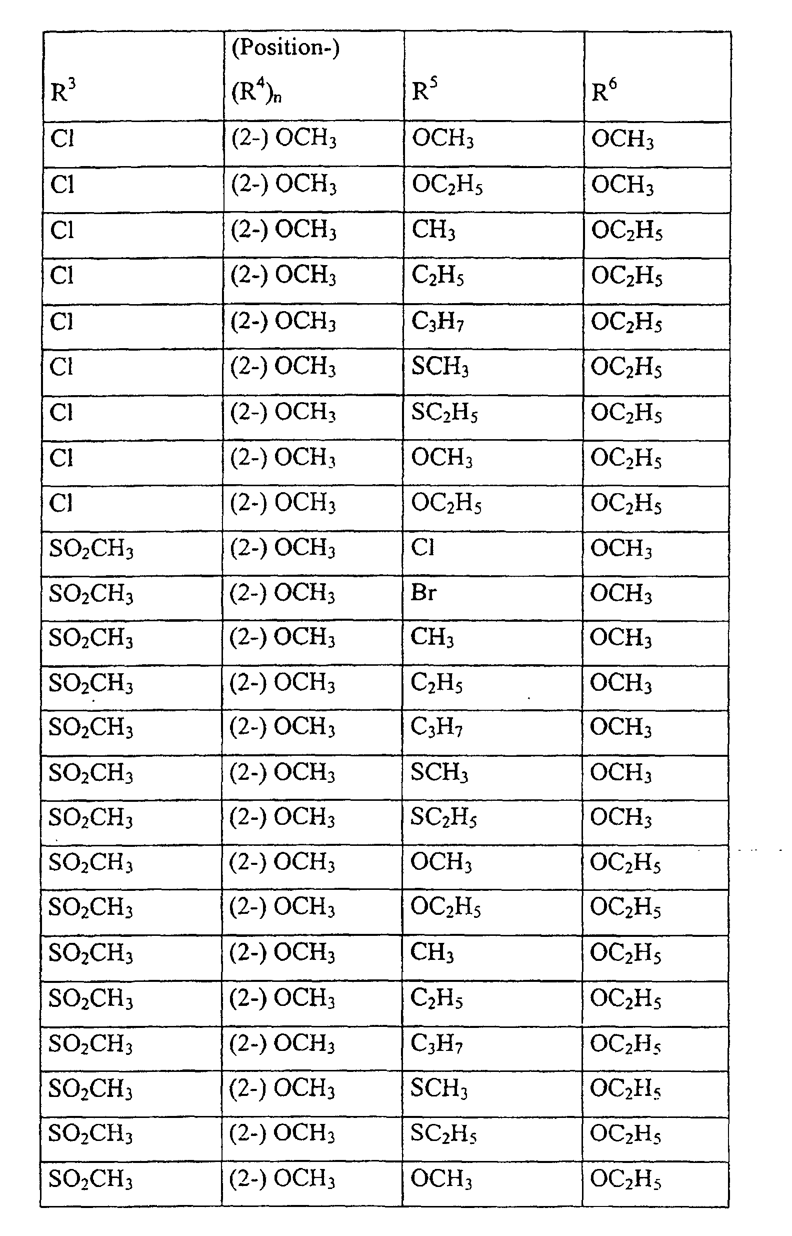

### Gruppe 10

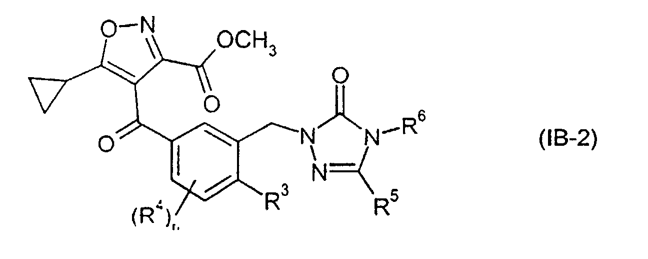

R³, (R⁴)ₙ, R⁵ und R⁶ haben hierbei berspielhaft die für Gruppe 9 angegebenen Bedeutungen.

### Gruppe 11

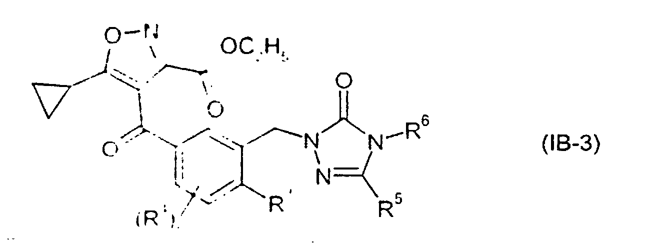

R³, (R⁴)ₙ, R⁵ und R⁶ haben hierbei beispielhaft die für Gruppe 9 angegebenen Bedeutungen.

### Gruppe 12

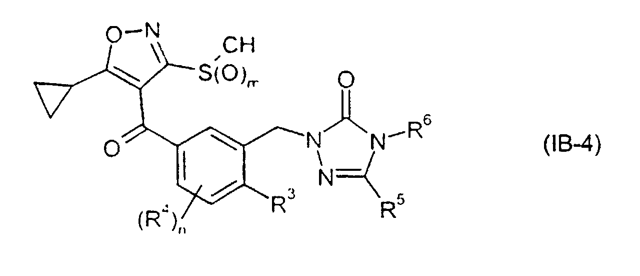

R³, (R⁴)ₙ, R⁵ und R⁶ haben hierbei beispielhaft die für Gruppe 9 angegebenen Bedeutungen und m steht für die Zahlen 0, 1 oder 2.

### Gruppe 13

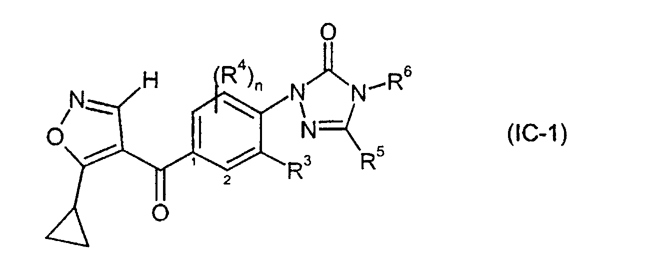

R³, (R⁴)ₙ, R⁵ und R⁶ haben hierbei beispielhaft die für Gruppe 9 angegebenen Bedeutungen.

### Gruppe 14

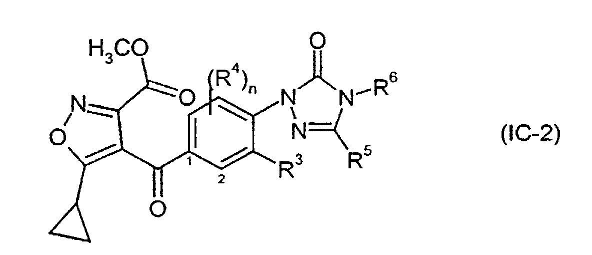

R³, (R⁴)ₙ, R⁵ und R⁶ haben hierbei beispielhaft die für Gruppe 9 angegebenen Bedeutungen.

### Gruppe 15

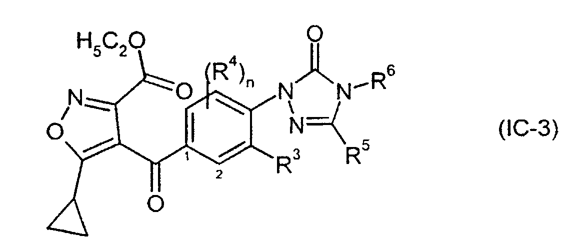

R³, (R⁴)ₙ, R⁵ und R⁶ haben hierbei beispielhaft die für Gruppe 9 angegebenen Bedeutungen.

### Gruppe 16

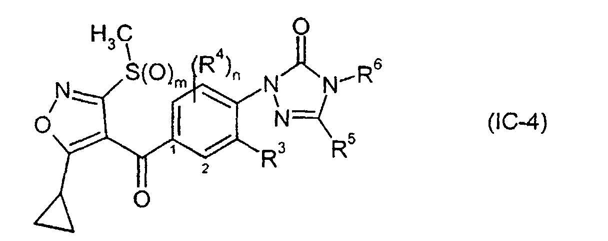

R³, (R⁴)ₙ, R⁵ und R⁶ haben hierbei beispielhaft die für Gruppe 9 angegebenen Bedeutungen und m steht für die Zahlen 0, 1 oder 2.

Die neuen substituierten Benzoylisoxazole der allgemeinen Formel (I) zeichnen sich durch starke und selektive herbizide Wirksamkeit aus.

Man erhält die neuen substituierten Benzoylisoxazole der allgemeinen Formel (I), wenn man
(a) Benzoylisoxazole der allgemeinen Formel (II) 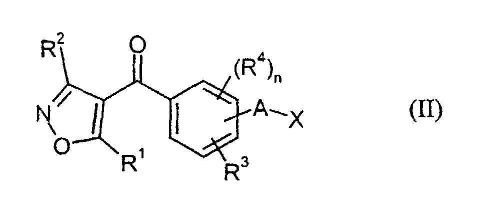 in welcher
   - n, A, R¹, R², R³ und R⁴: die oben angegebene Bedeutung haben und
   - X: für Halogen steht,
   mit Heterocyclen der allgemeinen Formel (III)

   H-Z (III)

   in welcher
   - Z: die oben angegebene Bedeutung hat,
   gegebenenfalls in Gegenwart eines oder mehrerer Reaktionshilfsmittel und gegebenenfalls in Gegenwart eines oder mehrerer Verdünnungsmittel umsetzt,
   oder wenn man
   - für den Fall, daß R² für Wasserstoff steht -
(b) Benzoylketone der allgemeinen Formel (IV) 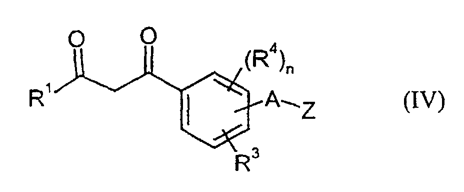 in welcher
   - n, A, R¹, R³, R⁴ und Z: die oben angegebene Bedeutung haben,
   mit einem Orthoameisensäuretrialkylester oder einem N,N-Dimethyl-formamid-dialkylacetal und anschließend mit Hydroxylamin oder einem Säureaddukt hiervon
   gegebenenfalls in Gegenwart eines oder mehrerer Reaktionshilfsmittel und gegebenenfalls in Gegenwart eines oder mehrerer Verdünnungsmittel umsetzt,
   oder wenn man
   - für den Fall, daß R² für gegebenenfalls substituiertes Alkoxycarbonyl steht -
(c) Benzoylketone der allgemeinen Formel (IV) 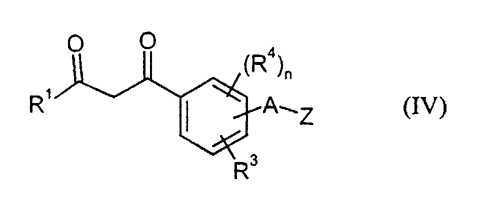 in welcher
   - n, A, R¹, R³, R⁴ und Z: die oben angegebene Bedeutung haben,
   mit einem Cyanoameisensäurealkylester und anschließend mit Hydroxylamin oder einem Säureaddukt hiervon, oder mit einem Chlor-hydroximino-essigsäurealkylester gegebenenfalls in Gegenwart eines oder mehrerer Reaktionshilfsmittel und gegebenenfalls in Gegenwart eines oder mehrerer Verdünnungsmittel umsetzt,
   oder wenn man
   - für den Fall das R² für Alkylthio steht -
(d) Benzoylketone der allgemeinen Formel (IV) 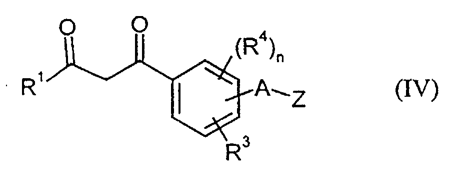
in welcher
- n, A, R¹, R³, R⁴ und Z: die oben angegebene Bedeutung haben,
mit Carbondisulfid (Schwefelkohlenstoff) und mit einem Alkylierungsmittel und anschließend mit Hydroxylamin oder einem Säureaddukt hiervon
gegebenenfalls in Gegenwart eines oder mehrerer Reaktionshilfsmittel und gegebenenfalls in Gegenwart eines oder mehrerer Verdünnungsmittel umsetzt,
und gegebenenfalls im Anschluß daran an den gemäß Verfahren (a) bis (d) erhaltenen Verbindungen der Formel (I) im Rahmen der Substituentendefinition auf übliche Weise elektrophile oder nucleophile Substitutionsreaktionen bzw. Oxidations- oder Reduktionsreaktionen durchführt.

Die Verbindungen der Formel (I) können nach üblichen Methoden in andere Verbindungen der Formel (I) gemäß obiger Definition umgewandelt werden, beispielsweise durch nucleophile Substitution (z.B. R⁵: Cl → OC₂H₅, SCH₃) oder durch Oxidation (z.B. R⁵: CH₂SCH₃ → CH₂S(O)CH₃).

Bei der Herstellung von Verbindungen der allgemeinen Formel (I) können in gewissem Umfang auch Verbindungen der allgemeinen Formel (IE) entstehen 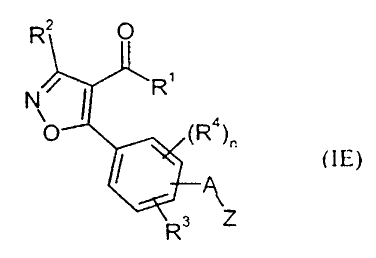 in welcher
- n, A, R¹, R², R³, R⁴ und Z: die oben angegebene Bedeutung haben.
Auch die Verbindungen der allgemeinen Formel (IE) sind als neue Stoffe Gegenstand der vorliegenden Anmeldung.

Verwendet man beispielsweise (3-Chlormethyl-4-trifluormethyl-phenyl)-(3,5-dimethyl-isoxazol-4-yl)-methanon und 4-Methyl-5-trifluormethyl-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

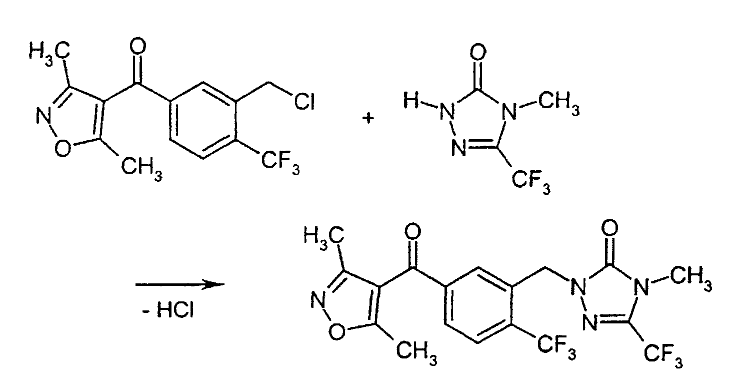

Verwendet man beispielsweise 1-[2-Chlor-3-(3,4-dimethyl-5-oxo-4,5-dihydro-[1,2,4-triazol-1-yl-methyl)-phenyl]-pentan-1,3-dion, N,N-Dimethyl-formamid-diethylacetalund Hydroxylamin als Ausgangsstoffe, so kann der Reaktionsablauf beim erfindungsgemäßen Verfahren (b) durch das folgende Formelschema skizziert werden:

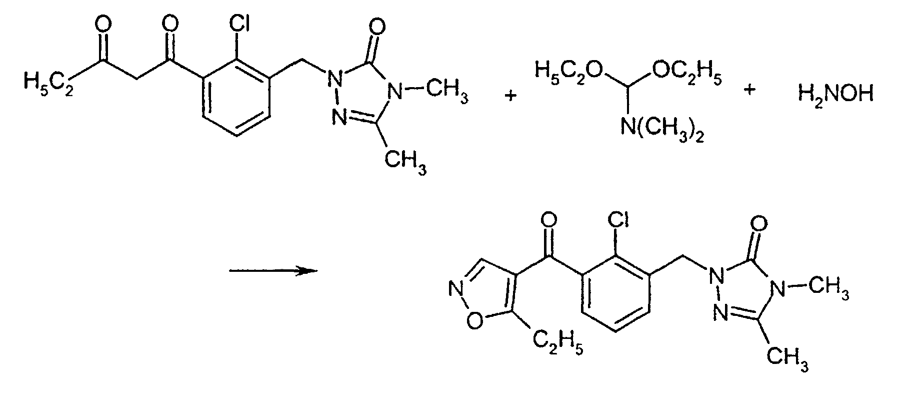

Verwendet man beispielsweise 1-[2-Chlor-3-(4-ethoxy-3-ethyl-5-oxo-4,5-dihydro-1,2,4-triazol-1-yl-methyl)-phenyl)-3-cyclopropyl-propan-1,3-dion, Cyanoameisensäure-ethylester und Hydroxylamin als Ausgangsstoffe, so kann der Reaktionsablauf beim erfindungsgemäßen Verfahren (c) durch das folgende Formelschema skizziert werden:

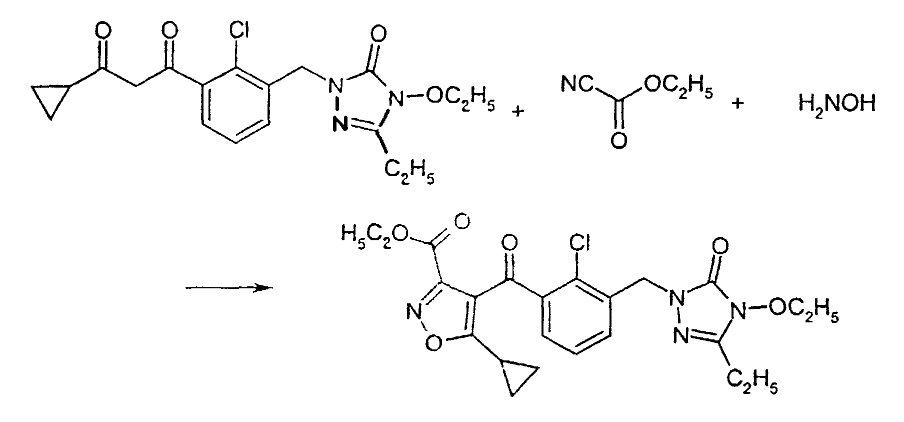

Verwendet man beispielsweise 1-[2-Chlor-3-(4-methyl-3-methylthio-5-oxo-4,5-dihydro-1,2,4-triazol-1-yl-methyl)-phenyl]-3-cyclopropyl-propan-1,3-dion, Carbondisulfid, Methylbromid und Hydroxylamin als Ausgangsstoffe, so kann der Reaktionsablauf beim erfindungsgemäßen Verfahren (d) durch das folgende Formelschema skizziert werden:

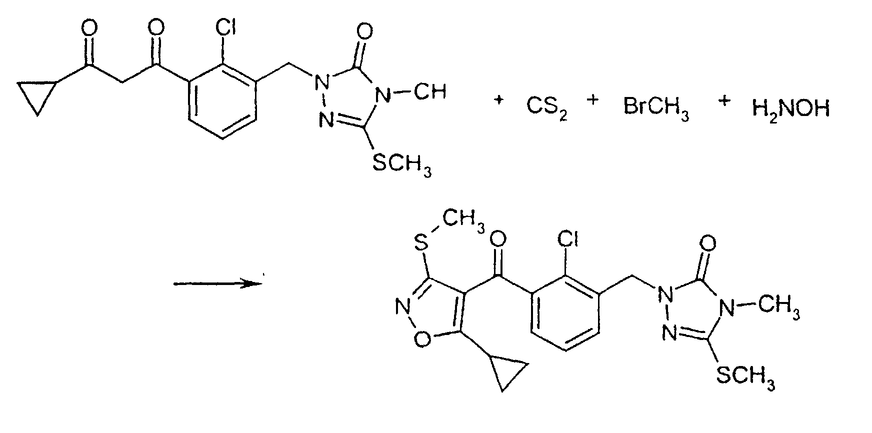

Die beim erfindungsgemäßen Verfahren (a) zur Herstellung von Verbindungen der allgemeinen Formel (I) als Ausgangsstoffe zu verwendenden Benzoylisoxazole sind durch die Formel (II) allgemein definiert. In der allgemeinen Formel (II) haben n, A, R¹, R², R³ und R⁴ vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für n, A, R¹, R², R³ und R⁴ angegeben worden sind; X steht vorzugsweise für Fluor, Chlor, Brom oder Iod, insbesondere fiir Chlor oder Brom.

Die Ausgangsstoffe der allgemeinen Formel (II) sind mit Ausnahme von 4-(2-Brommethyl-benzoyl)-5-cyclopropyl-isoxazol-3-carbonsäure-ethylester (vgl. WO-A-95/31446) noch nicht aus der Literatur bekannt; sie sind unter Ausnahme von 4-(2-Brommethyl-benzoyl)-5-cyclopropyl-isoxazol-3-carbonsäure-ethylester als neue Stoffe auch Gegenstand der vorliegenden Anmeldung.

Man erhält die neuen Benzoylisoxazole der allgemeinen Formel (II), wenn man Benzoylisoxazole der allgemeinen Formel (V) 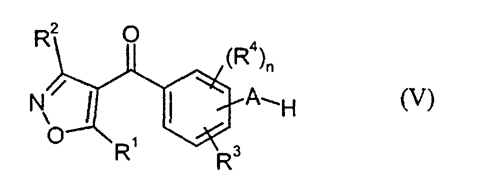 in welcher
- n, A, R¹, R², R³ und R⁴: die oben angegebene Bedeutung haben,
mit einem Seitenketten-Halogenierungmittel, wie z.B. N-Brom-succinimid oder N-Chlor-succinimid, im UV-Licht oder in Gegenwart eines Reaktionshilfsmittels, wie z.B. 2,2'-Azo-bis-isobuttersäurenitril, in Gegenwart eines Verdünnungsmittel, wie z.B. Tetrachlormethan, bei Temperaturen zwischen 0°C und 100°C umsetzt (vgl. WO-A-95/31446; Herstellungsbeispiele).

Die Vorprodukte der allgemeinen Formel (V) sind bekannt und/oder können nach an sich bekannten Verfahren hergestellt werden (vgl. WO-A-95/31446; Herstellungsbeispiele).

Die beim erfindungsgemäßen Verfahren (a) zur Herstellung von Verbindungen der allgemeinen Formel (I) weiter als Ausgangsstoffe zu verwendenden Heterocyclen sind durch die Formel (III) allgemein definiert. In der allgemeinen Formel (III) hat Z vorzugsweise diejenige Bedeutung, die bereits oben im Zusammenhang mit der Beschreibung der erfindungsgemäßen Verbindungen der allgemeinen Formel (I) als bevorzugt für Z angegeben worden ist.

Die Ausgangsstoffe der allgemeinen Formel (III) sind bekannt und/oder können nach an sich bekannten Verfahren hergestellt werden.

Die bei den erfindungsgemäßen Verfahren (b), (c) und (d) zur Herstellung von Verbindungen der allgemeinen Formel (I) als Ausgangsstoffe zu verwendenden Benzoylketone sind durch die Formel (IV) allgemein definiert. In der allgemeinen Formel (IV) haben n, A, R¹, R³, R⁴ und Z vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für n, A, R¹, R³, R⁴ und Z angegeben worden sind.

Die Ausgangsstoffe der allgemeinen Formel (IV) sind noch nicht aus der Literatur bekannt; sie sind als neue Stoffe auch Gegenstand der vorliegenden Anmeldung.

Man erhält die neuen Benzoylketone der allgemeinen Formel (IV), wenn man Ketone der allgemeinen Formel (VI) 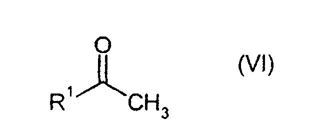 in welcher
- R¹: die oben angegebene Bedeutung hat,
mit Benzoesäurederivaten der allgemeinen Formel (VII) 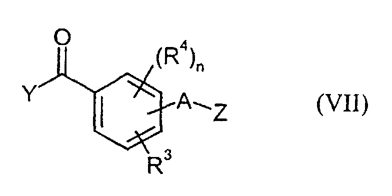 in welcher
- n, A, R³, R⁴ und Z: die oben angegebene Bedeutung haben, und
- Y: für Halogen (insbesondere Fluor, Chlor oder Brom) oder für gegebenenfalls substituiertes Alkoxy (insbesondere Methoxy, Ethoxy oder Ethoxyethoxy) steht,
gegebenenfalls in Gegenwart eines Reaktionshilfsmittels, wie z.B. Natriumhydrid, und gegebenenfalls in Gegenwart eines Verdünnungsmittels, wie z.B. Tetrahydrofuran, bei Temperaturen zwischen 0°C und 100°C umsetzt (vgl. die Herstellungsbeispiele).

Die als Vorprodukte benötigten Benzoesäurederivate der allgemeinen Formel (VII) sind bekannt und/oder können nach an sich bekannten Verfahren hergestellt werden (vgl. DE-A-38 39 480, DE-A-42 39 296, EP-A-597 360, EP-A-609 734, DE-A-43 03 676, EP-A-617 026, DE-A-44 05 614, US-A-5 378 681).

Man erhält die Benzoesäurederivate der allgemeinen Formel (VII), wenn man Halogen(alkyl)benzoesäurederivate der allgemeinen Formel (VIII), 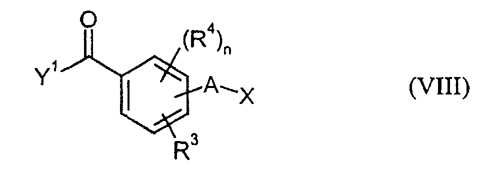 in welcher
- n, A, R³ und R⁴: die oben angegebene Bedeutung haben und
- X: für Halogen (insbesondere Fluor, Chlor oder Brom) steht, und
- Y¹: für gegebenenfalls substituiertes Alkoxy (insbesondere Methoxy, Ethoxy oder Ethoxyethoxy) steht,
mit Verbindungen der allgemeinen Formel (III),

H-Z (III)

in welcher
- Z: die oben angegebene Bedeutung hat,
gegebenenfalls in Gegenwart eines Reaktionshilfsmittel, wie z.B. Natriumhydrid, Triethylamin oder Kaliumcarbonat, und gegebenenfalls in Gegenwart eines Verdünnungsmittels, wie z.B. Aceton, Acetonitril, N,N-Dimethyl-formamid oder N,N-Dimethyl-acetamid, bei Temperaturen zwischen 50°C und 200°C umsetzt (vgl. die Herstellungsbeispiele).

Die als Vorprodukte benötigten Halogen(alkyl)benzoesäurederivate der Formel (VIII) sind bekannt und/oder können nach an sich bekannten Verfahren hergestellt werden (vgl. EP-A-90 369, EP-A-93 488, EP-A-399 732, EP-A-480 641, EP-A-609798, EP-A-763 524, DE-A-21 26 720, WO-A-93/03722, WO-A-97/38977, US-A-39 78 127, US-A-48 37 333).

Das erfindungsgemäße Verfahren (b) zur Herstellung der Verbindungen der Formel (I) wird unter Verwendung von Orthoameisensäureestern oder N,N-Dimethyl-formamid-acetalen durchgeführt. Diese Verbindungen enthalten vorzugsweise Alkylgruppen mit 1 bis 4 Kohlenstoffatomen, insbesondere Methyl oder Ethyl. Als Beispiele seien Orthoameisensäure-trimethylestcr, Orthoameisensäure-triethylester, N,N-Dimethyl-formamid-dimethylacetal und N,N-Dimethyl-formamid-diethylacetal genannt.

Das erfindungsgemäße Verfahren (c) zur Herstellung der Verbindungen der Formel (I) wird unter Verwendung von Cyanoameisensäurealkylestern oder Chlor-hydroximino-essigsäurealkylestern durchgeführt. Diese Verbindungen enthalten vorzugsweise Alkylgruppen mit 1 bis 4 Kohlenstoffatomen, insbesondere Methyl oder Ethyl. Als Beispiele seien Cyanoameisensäure-methylester, Cyanoameisensäureethylester, Chlor-hydroximino-essigsäure-methylester und Chlor-hydroximino-essigsäure-ethylester genannt.

Das erfindungsgemäße Verfahren (d) zur Herstellung der Verbindungen der Formel (I) wird unter Verwendung von (Carbondisulfid und) Alkylierungsmitteln durchgeführt. Diese Verbindungen enthalten vorzugsweise Alkylgruppen mit 1 bis 4 Kohlenstoffatomen, insbesondere Methyl oder Ethyl. Als Beispiele seien Methylchlorid, Methylbromid, Methyliodid, Dimethylsulfat, Ethylchlorid, Ethylbromid, Ethyliodid und Diethylsulfat genannt.

Die erfindungsgemäßen Verfahren (b), (c) und - gegebenenfalls - (d) zur Herstellung der Verbindungen der Formel (I) werden unter Verwendung von Hydroxylamin oder einem Säureaddukt hiervon durchgeführt. Als bevorzugtes Säureaddukt sei Hydroxylamin-Hydrochlorid genannt.

Die erfindungsgemäßen Verfahren zur Herstellung der Verbindungen der allgemeinen Formel (I) werden vorzugsweise unter Verwendung von Verdünnungsmitteln durchgeführt. Als Verdünnungsmittel zur Durchführung der erfindungsgemäßen Verfahren (a), (b), (c) und (d) kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Butyronitril; Amide, wie N,N-Dimethylformamid, N,N-Dimethylacetamid, N-Methyl-formanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Reaktionshilfsmittel für die erfindungsgemäßen Verfahren (a), (b), (c) und (d) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calciumcarbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -soder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen 0°C und 150°C, vorzugsweise zwischen 10°C und 120°C.

Die erfindungsgemäßen Verfahren werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindemia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und-synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Didosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Picolinafen, Piperophos, Pretilachlor, Primisulfuron(-methyl), Procarbazone, Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin, Triflusulfuron, Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver. Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

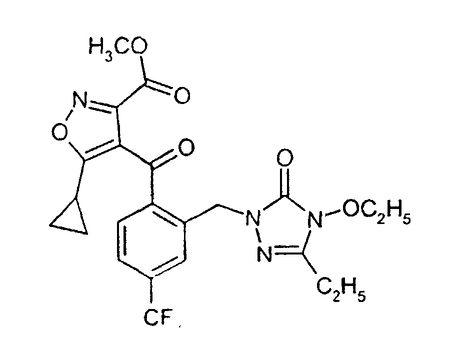

### (Verfahren (a))

Eine Lösung von 1,20 g (33% ig, d.h. 2,8 mMol) 4-(3-Brommethyl-5-trifluormethylbenzoyl)-5-cyclopropyl-isoxazol-3-carbonsäure-methylester in 10 ml N,N-Dimethylformamid wird bei Raumtemperatur (ca. 20 °C) unter Rühren zu einer Mischung aus 0,44 g (2,8 mMol) 4-Ethoxy-5-ethyl-2,4-dihydro-3H-1,2,4-triazol-3-on, 84 mg (2,8 mMol) Natriumhydrid (75% ig) und 20 ml N;N-Dimethyl-formamid tropfenweise gegeben und die Reaktionsmischung wird 30 Minuten bei Raumtemperatur gerührt. Anschließend wird die Mischung mit gesättigter wässriger Natriumchlorid-Lösung auf etwa das doppelte Volumen verdunnt und zweimal mit Essigsäureethylester extrahiert. Die vereinigten organischen Extraktionslösungen werden mit Natriumsulfat getrocknet und filtriert. Das Filtrat wird im Wasserstrahlvakuum eingeengt und der Rückstand durch Säulenchromatografie (Kieselgel, Hexan/Essigsäureethylester, Vol.: 7/3) gereinigt.

Man erhält 0,45 g (96 % der Theorie bezogen auf 33%iges Edukt) (5-Cyclopropyl-3-methoxycarbonyl-isoxazol-4-yl)-[2-(4-ethoxy-3-ethyl-5-oxo-4,5-dihydro-[1,2,4]-triazol-1-yl-methyl)-4-trifluormethyl-phenyl]-methanon als amorphes Produkt.

LogP (bei pH=2,3 bestimmt): 3.56.

### Beispiel 2

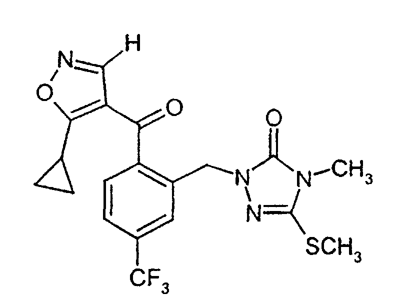

### (Verfahren (b))

Eine Mischung aus 1,5 g (36 mMol) 1-Cyclopropyl-3-[2-(4-methyl-3-methylthio-5-oxo-4,5-dihydro-[1,2,4]-triazol-1-yl-methyl)-4-trifluormethyl-phenyl]-propan-1,3-dion, 0,56 g (46 mMol) N,N-Dimethyl-formamid-dimethylacetal und 15 ml Toluol wird 60 Minuten bei 90°C gerührt. Dann wird im Wasserstrahlvakuum eingeengt, der Rückstand in 15 ml Ethanol aufgenommen und nach Zugabe von 0,25 g (36 mMol) Hydroxylamin-Hydrochlorid zwei Stunden bei Raumtemperatur (ca. 20°C) gerührt. Nach Einengen im Wasserstrahlvakuum wird der Rückstand mit Methylenchlorid/Wasser geschüttelt, die organische Phase abgetrennt, mit gesättigter wässriger Natriumchlorid-Lösung gewaschen, mit Natriumsulfat getrocknet und filtriert. Das Filtrat wird im Wasserstrahlvakuum eingeengt und der Rückstand durch Säulenchromatografie (Kieselgel, Essigsäureethylester/Hexan, Vol.: 1/1) gereinigt.

Man erhält 0,20 g (13 % der Theorie) (5-Cyclopropyl-isoxazol-4-yl)-[2-(4-methyl-3-methylthio-5-oxo-4,5-dihydro-[1,2,4]-triazol-1-yl-methyl)-4-trifluormethyl-phenyl]-methanon als amorphes Produkt.

LogP (bei pH=2,3 bestimmt): 2,94.

Analog zu den Beispielen 1 und 2 sowie entsprechend der allgemeinen Beschreibung der erfindungsgemäßen Herstellungsverfahren können beispielsweise auch die in den nachstehenden Tabellen 1 und 1a aufgeführten Verbindungen der allgemeinen Formel (I) - bzw. der Formeln (IA), (IB), (IC) oder (ID) - hergestellt werden.

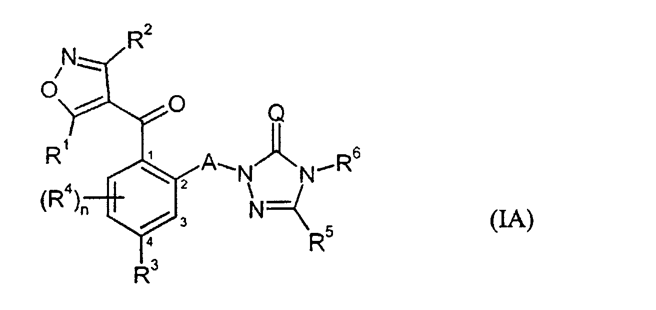

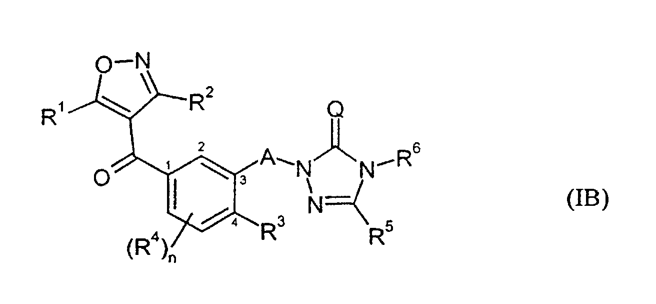

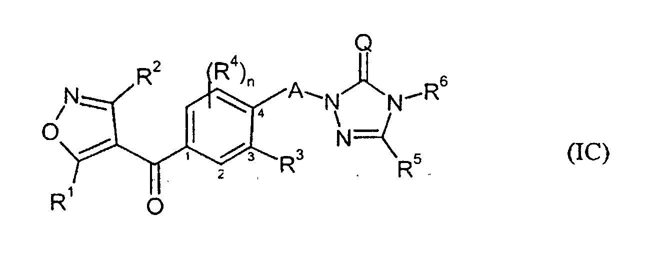

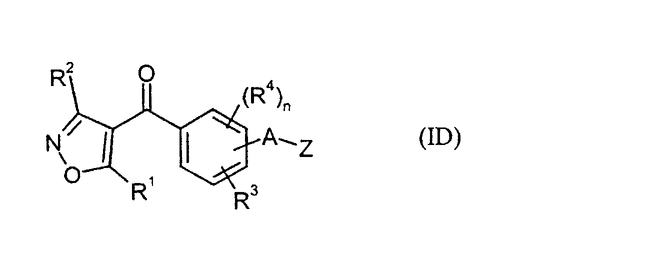

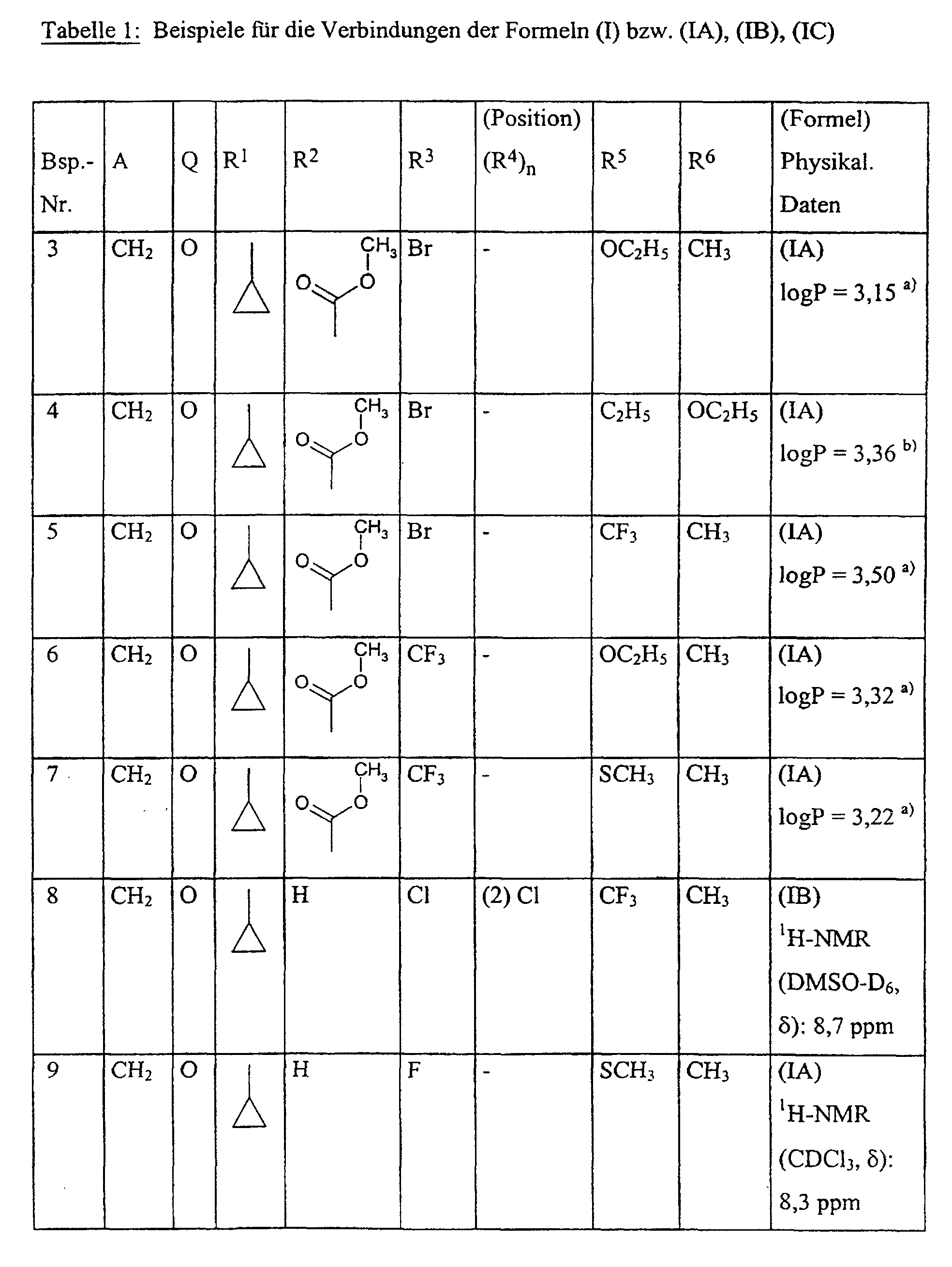

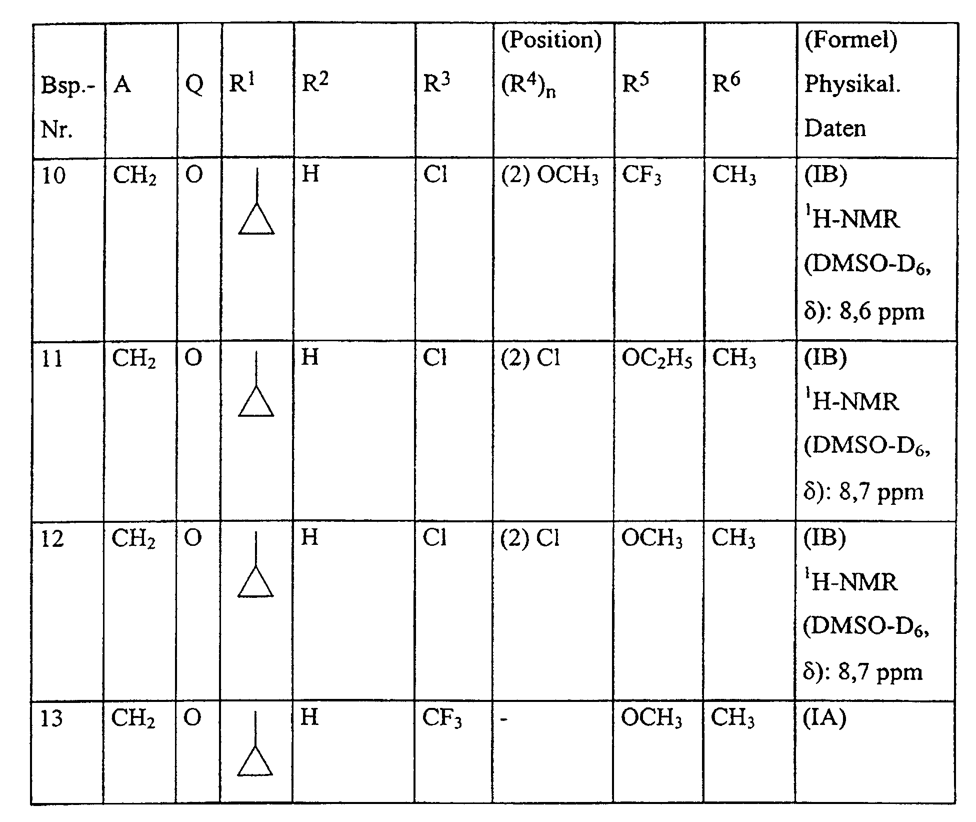

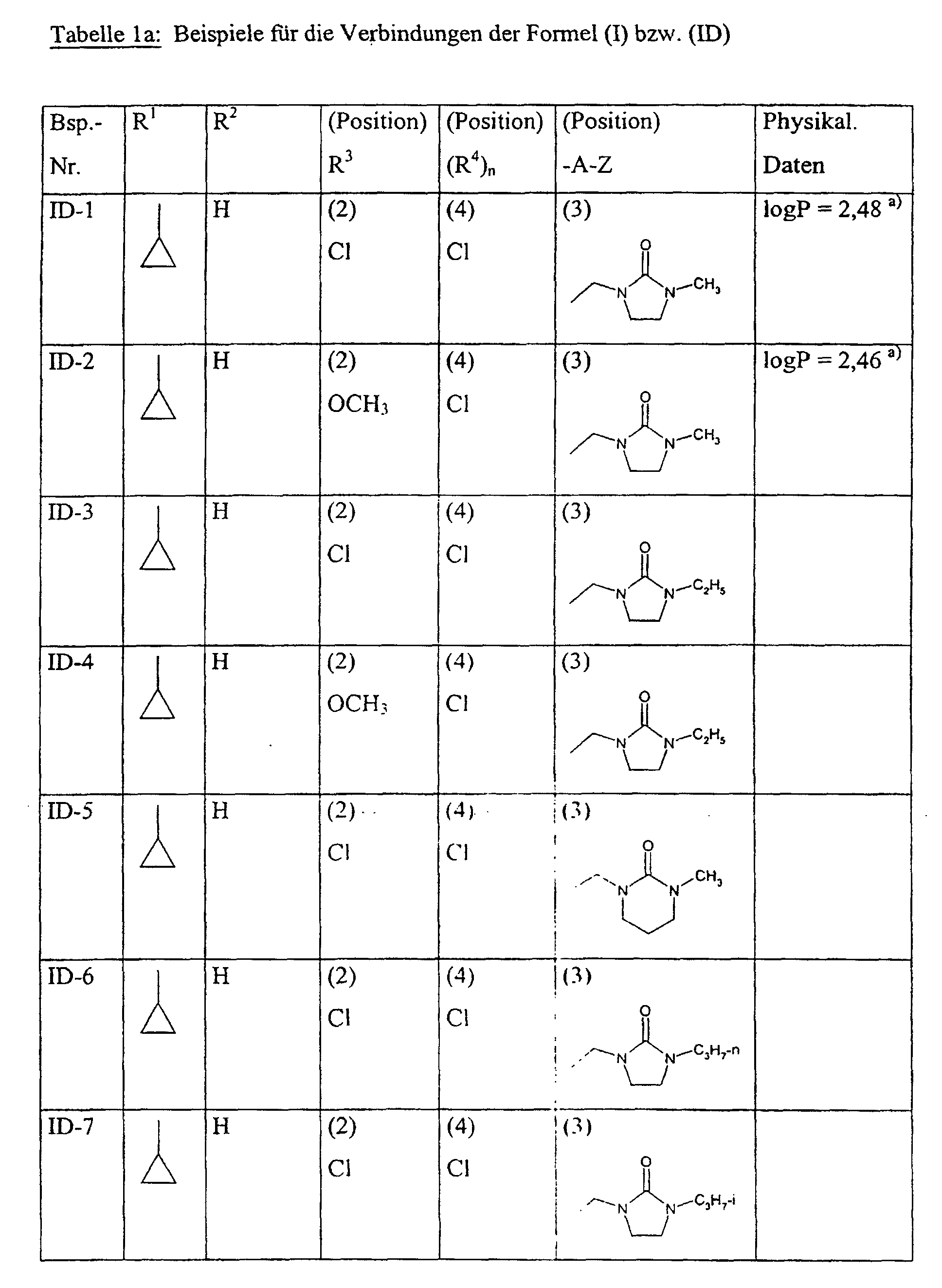

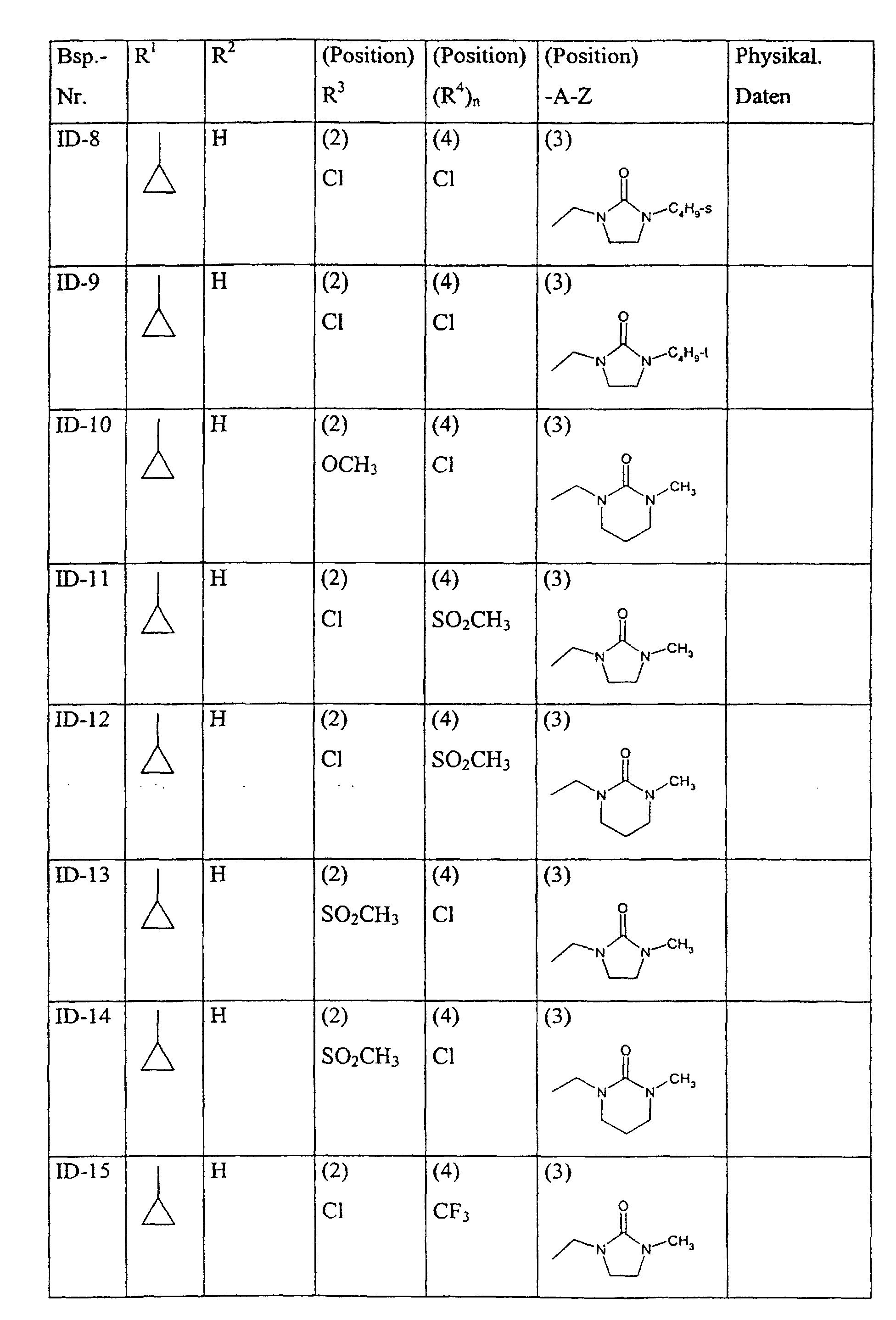

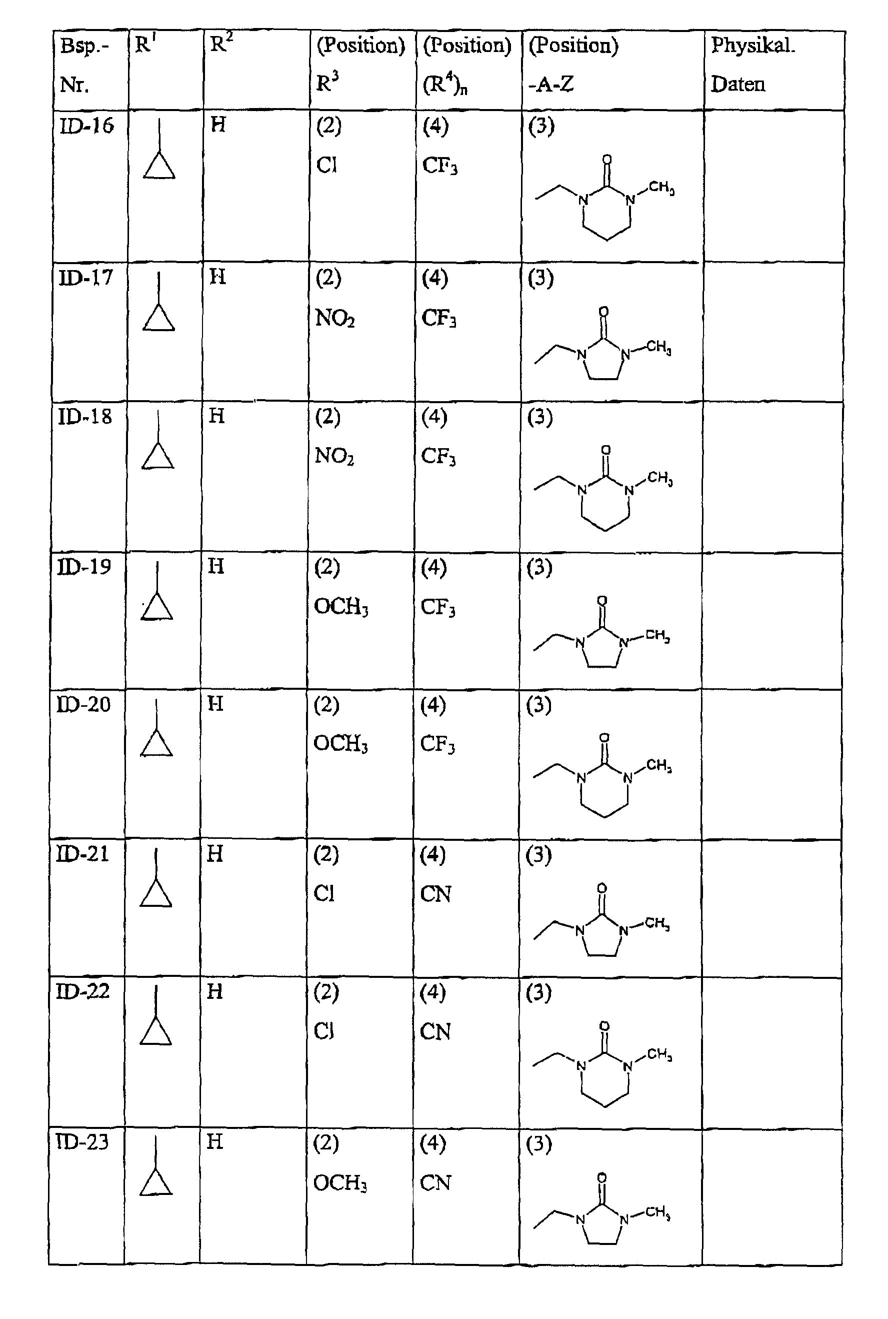

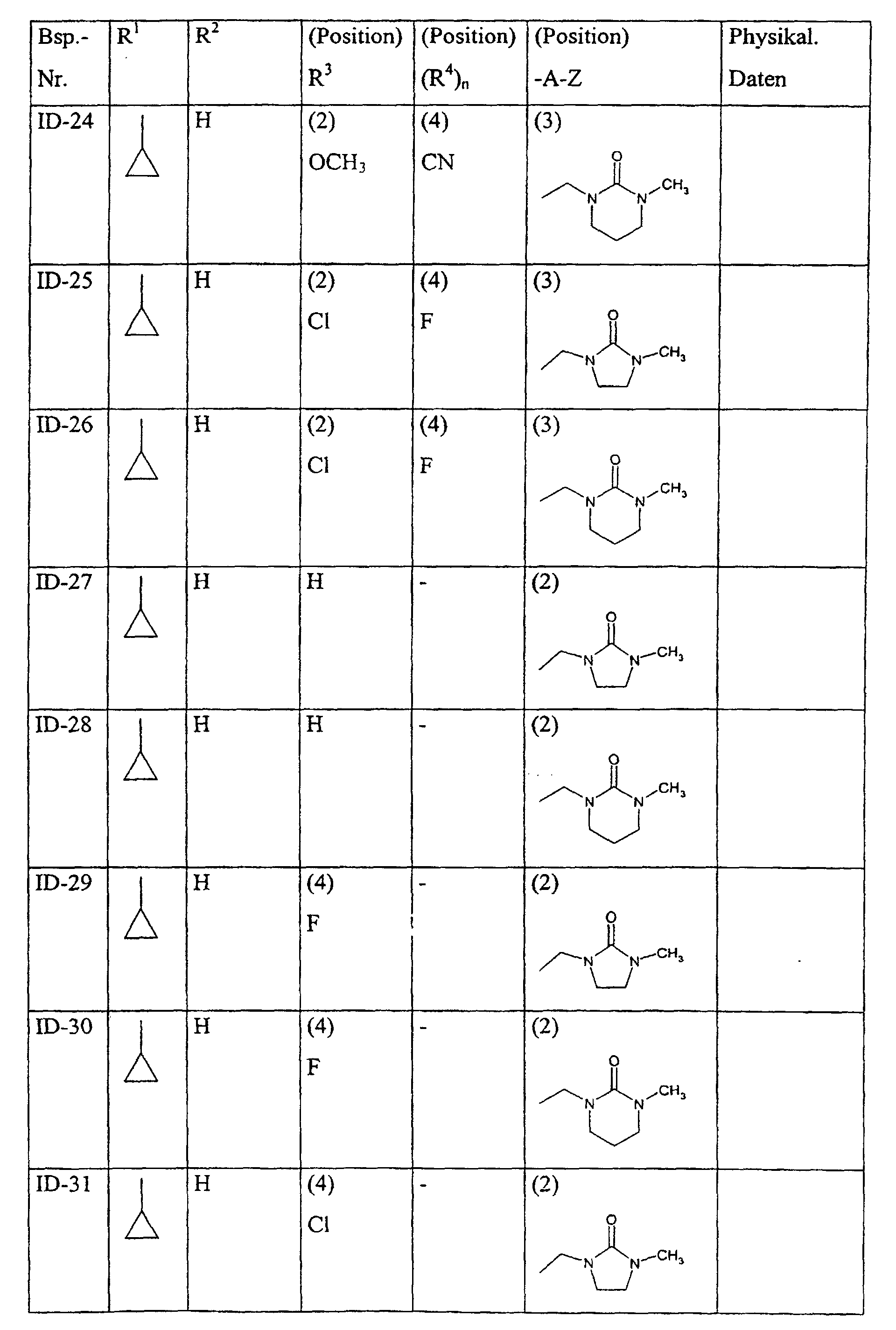

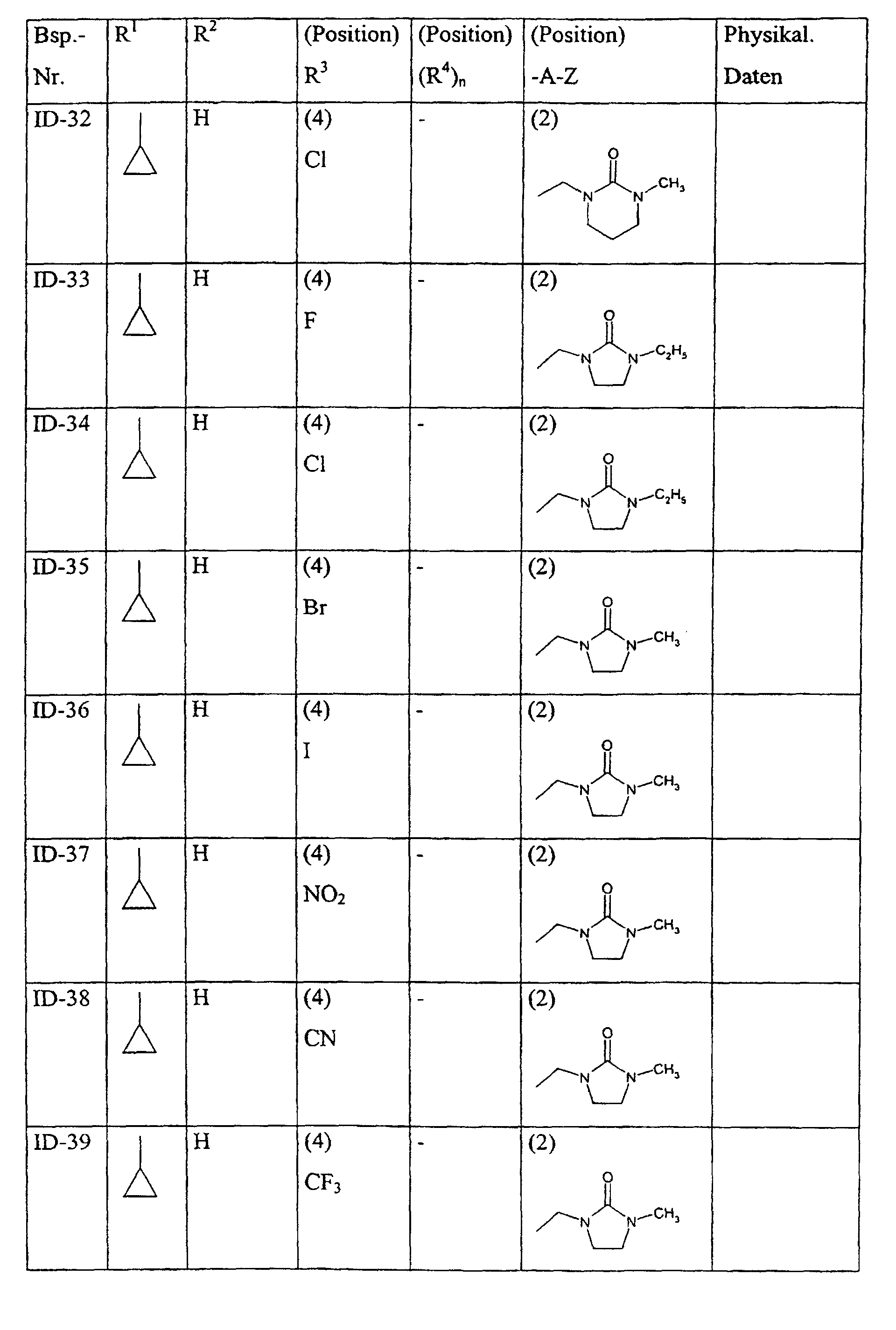

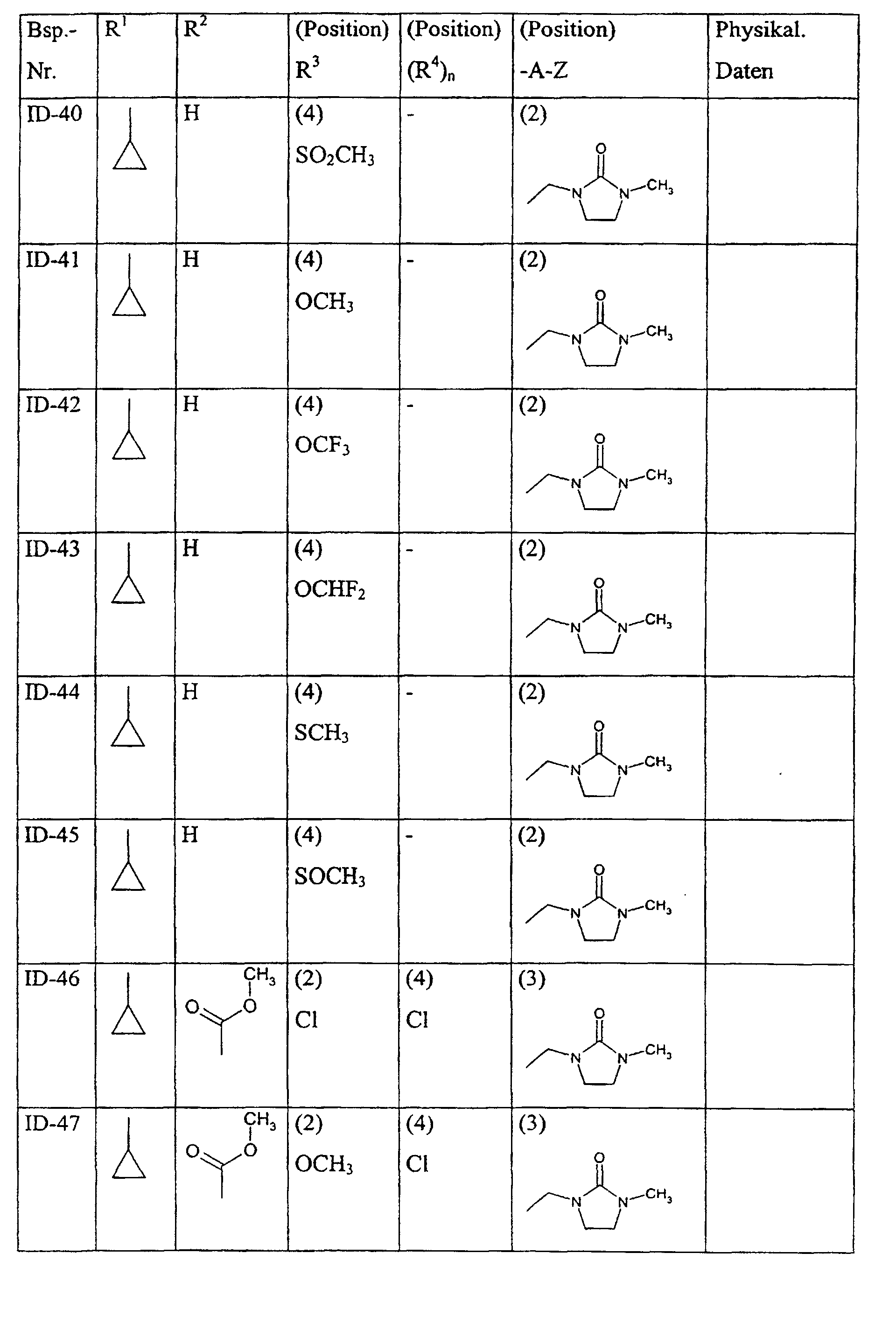

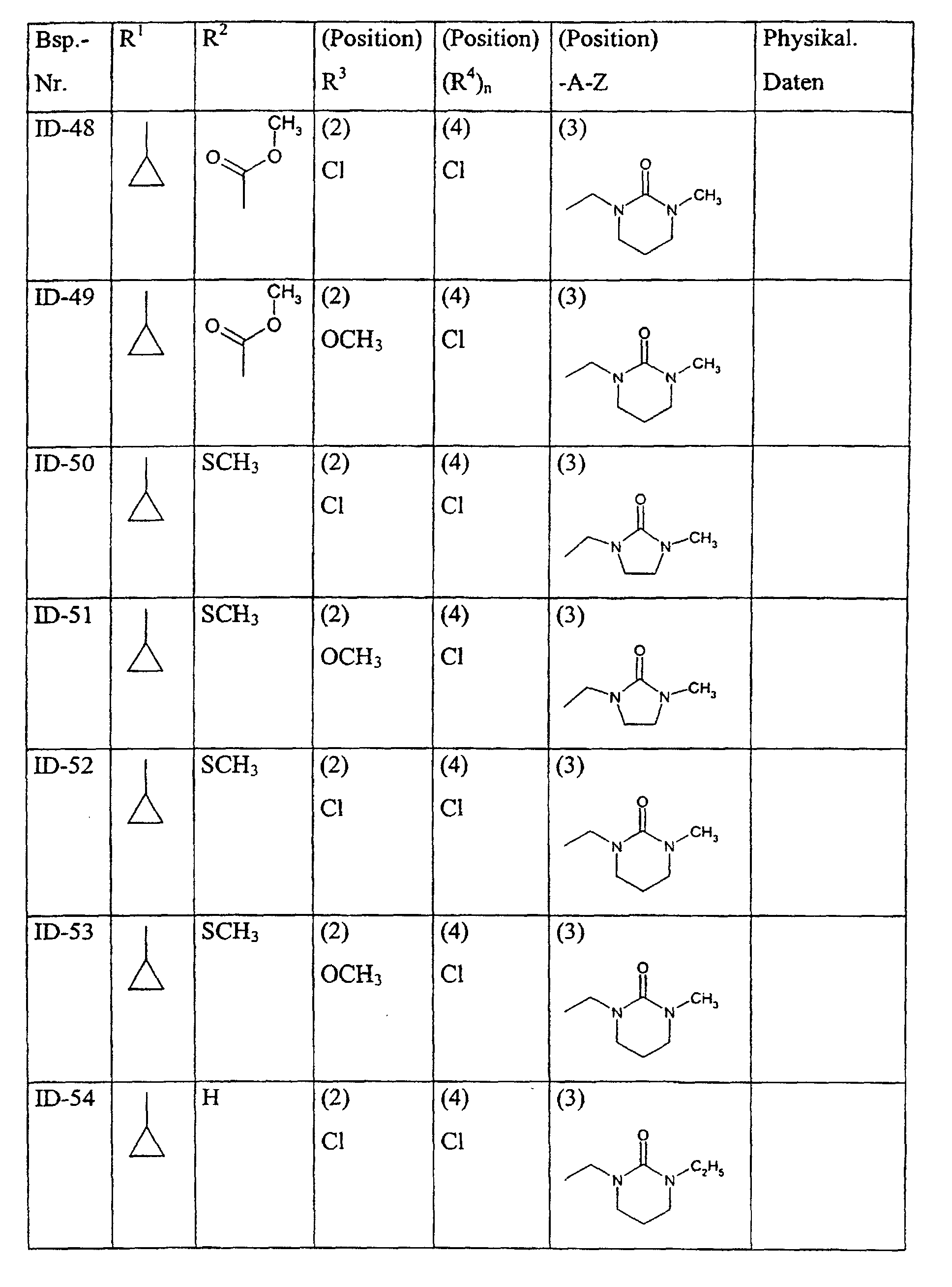

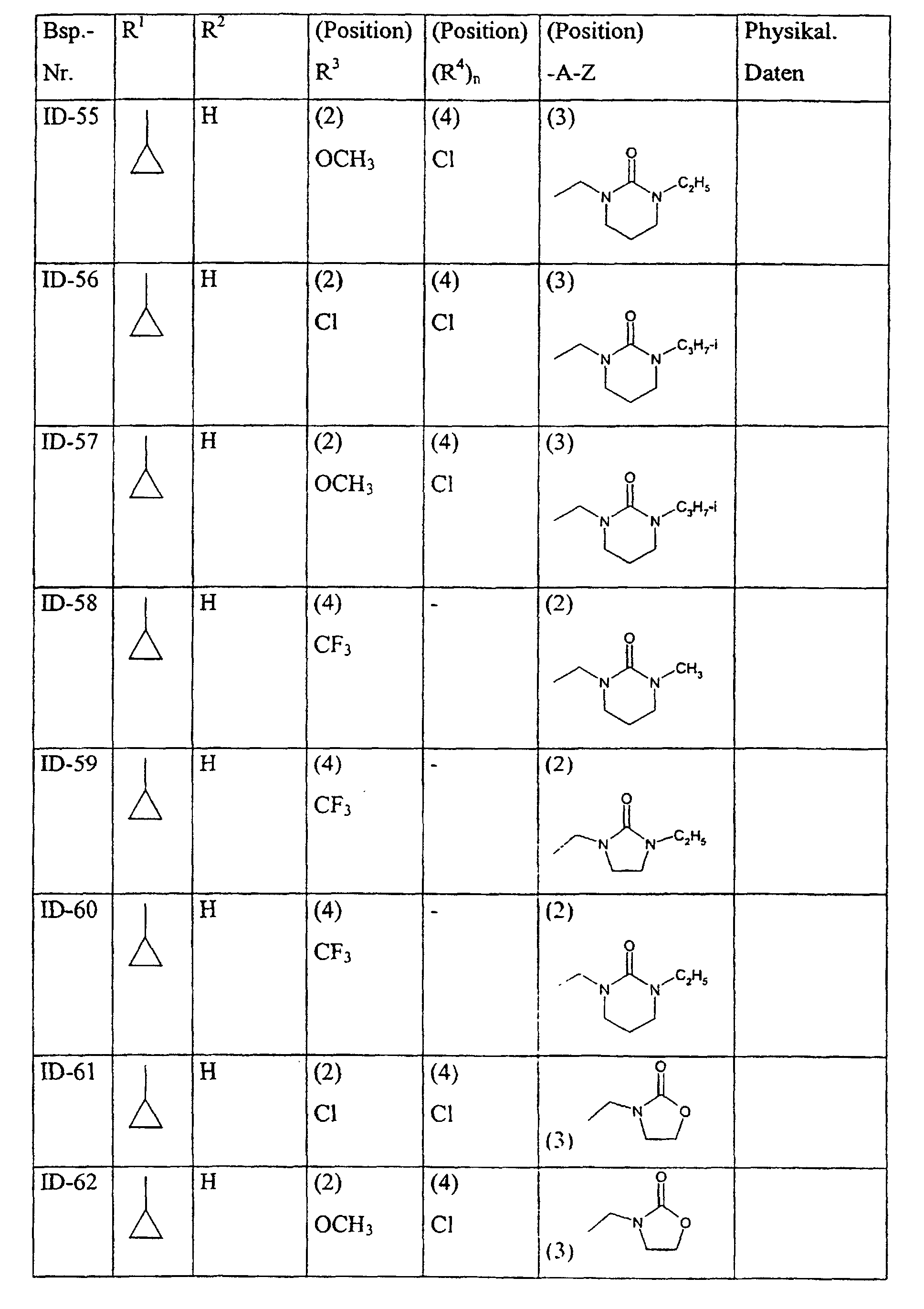

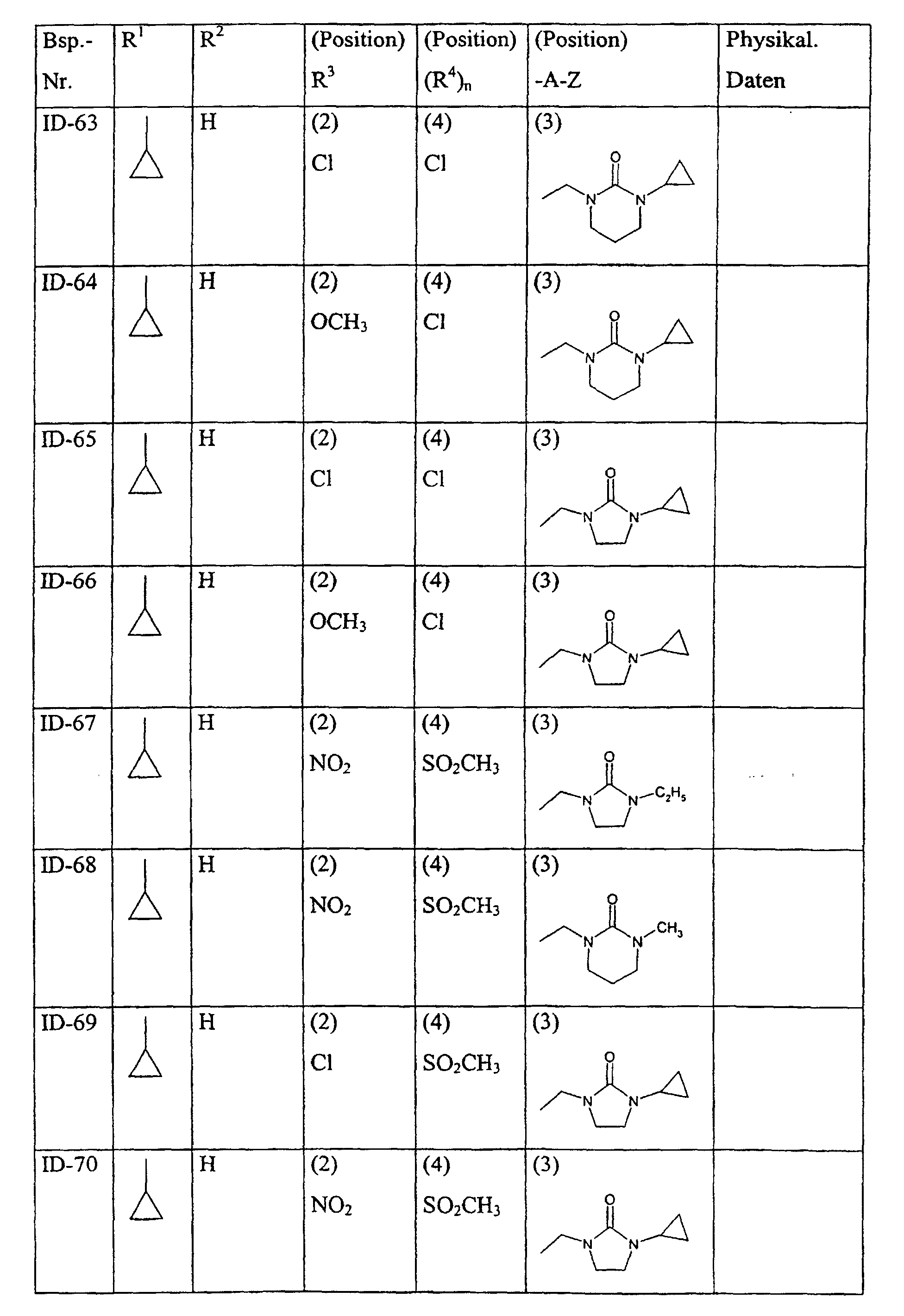

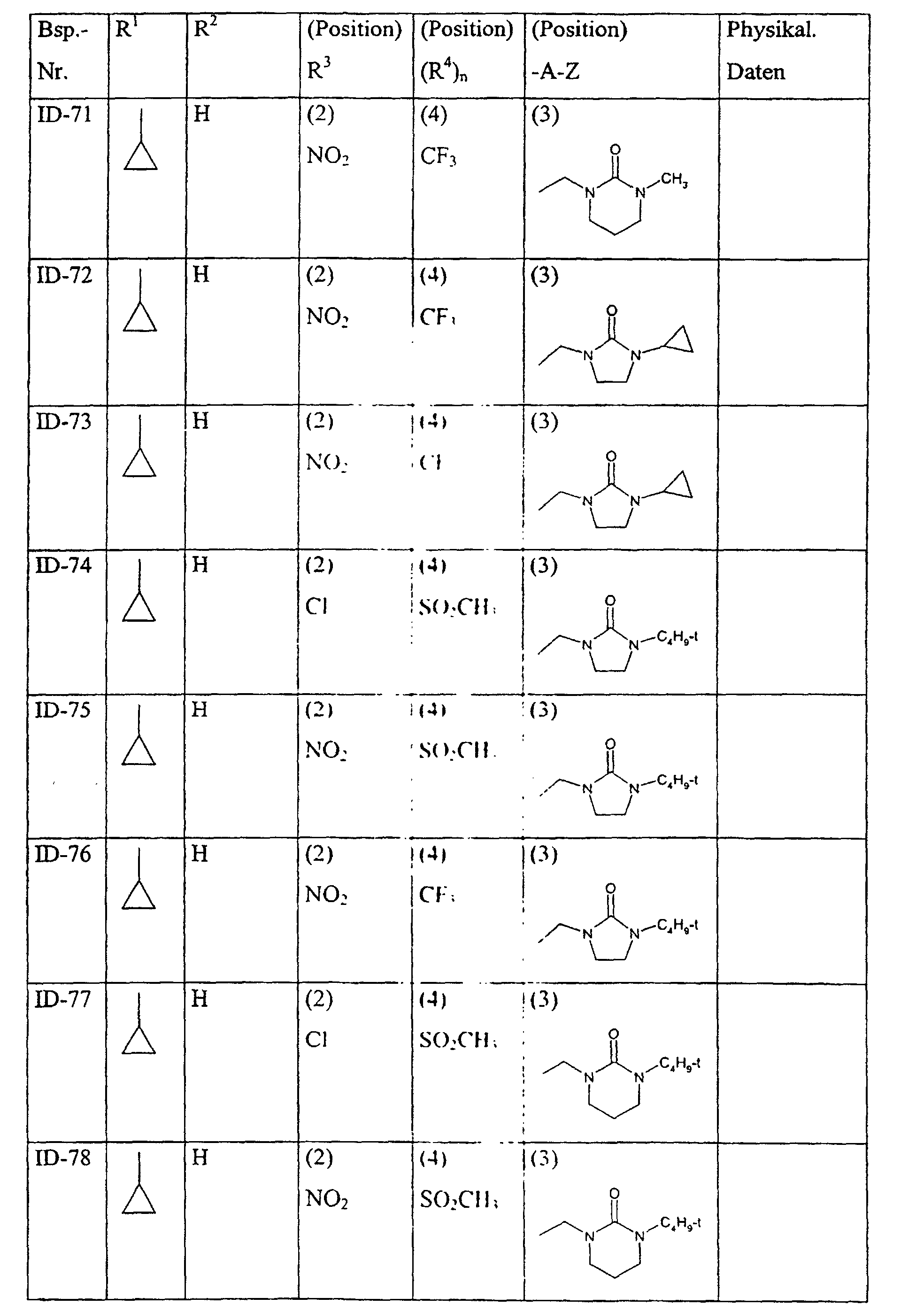

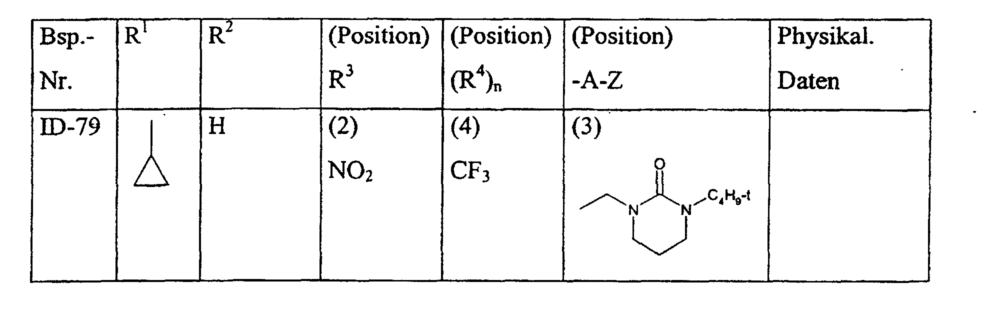

Die Bestimmung der in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10 % Acetonitril bis 90 % Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10 % Acetonitril bis 90 % Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (II):

### Beispiel (II-1)

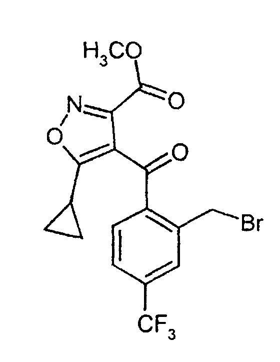

Eine Mischung aus 3,0 g (8,5 mMol) 5-Cyclopropyl-4-(2-methyl-4-trifluormethylbenzoyl)-isoxazol-4-carbonsäure-methylester, 1,5 g (8,5 mMol) N-Brom-succinimid, 0,15 g 2,2'-Azo-bis-isobuttersäurenitril und 45 ml Tetrachlormethan wird zwei Stunden unter Rückfluß erhitzt und nach Abkühlen filtriert. Das Filtrat wird mit Methylenchlorid auf etwa das doppelte Volumen verdünnt, mit 20%iger wässriger Natriumhydrogensulfit-Lösung gewaschen, mit Natriumsulfat getrocknet und filtriert. Vom Filtrat wird das Lösungsmittel im Wasserstrahlvakuum sorgfältig abdestilliert.

Man erhält 2,5 g (68 % der Theorie) 5-Cyclopropyl-4-(2-brommethyl-4-trifluormethyl-benzoyl)-isoxazol-4-carbonsäure-methylester als amorphes Produkt, welches ohne Reinigung weiter umgesetzt werden kann.

Analog Beispiel (II-1) können beispielsweise auch die in der nachstehenden Tabelle 2 aufgeführten Verbindungen der Formel (II) hergestellt werden.

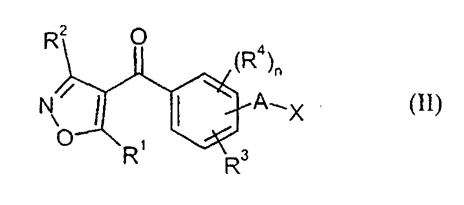

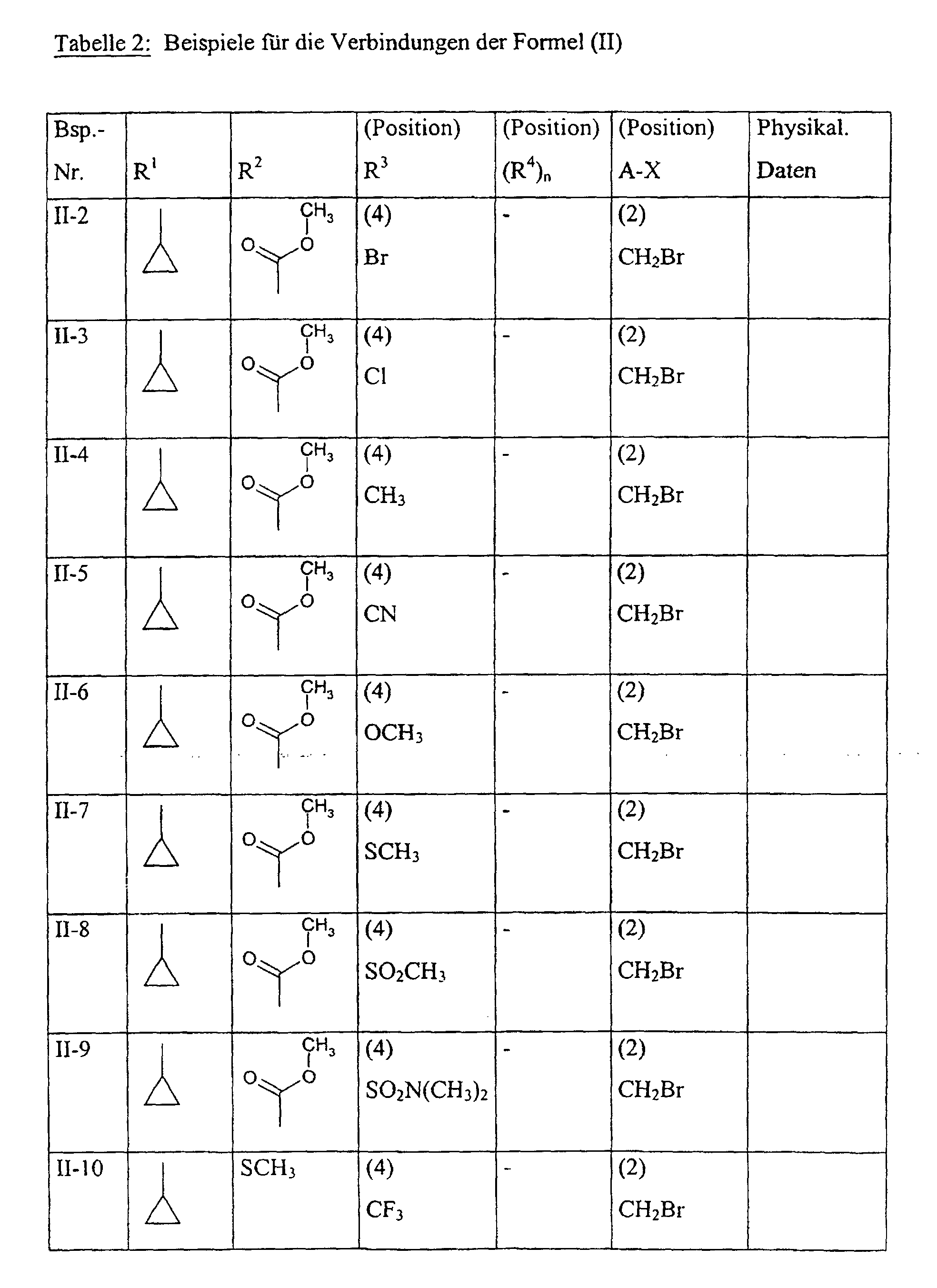

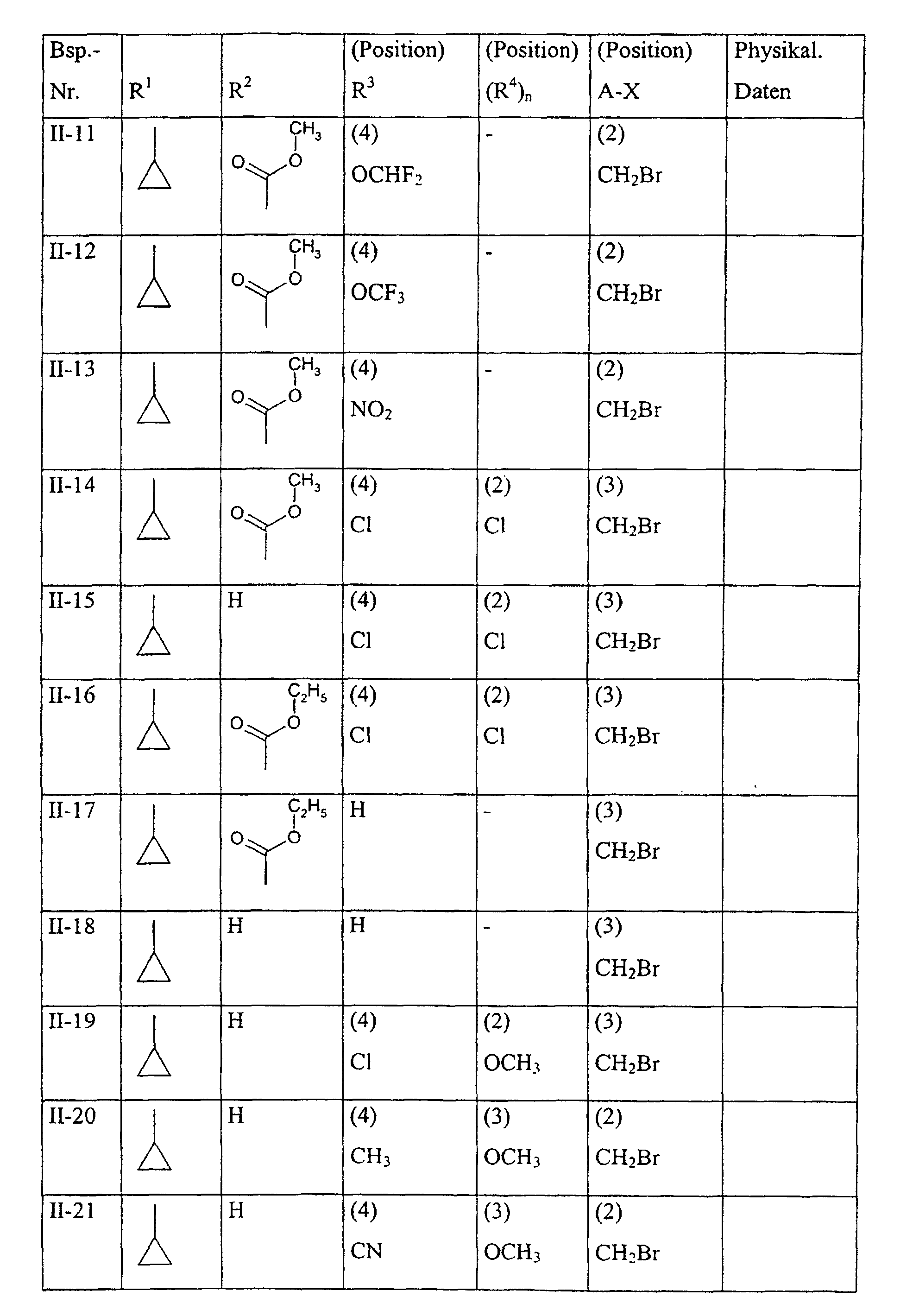

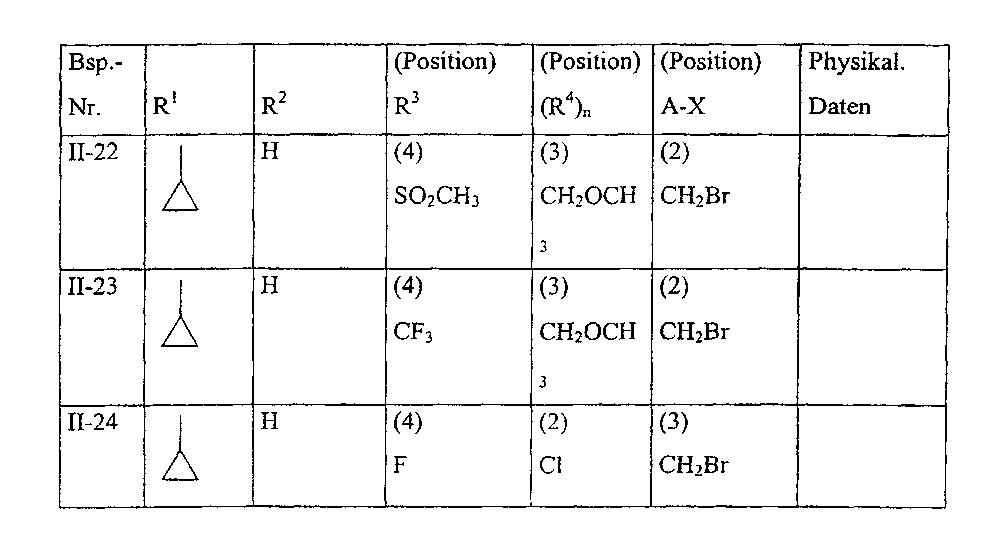

### Ausgangsstoffe der Formel (IV):

### Beispiel (IV-1)

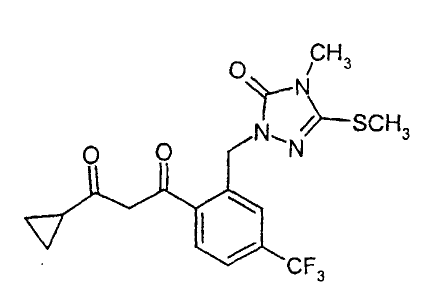

Eine Mischung aus 0,94 g (11 mMol) Cyclopropyl-methyl-keton, 0,35 g (11 mMol) Natriumhydrid (75%ig) und 15 ml Tetrahydrofuran wird 30 Minuten bei 20°C gerührt. Dann wird tropfenweise eine Lösung von 2,0 g (5,5 mMol) 4-Methyl-5-methylthio-2-(2-methoxycarbonyl-5-trifluormethyl-benzyl)-2,4-dihydro-3H-1,2,4-triazol-3-on in 8 ml Tetrahydrofuran dazu gegeben und nach Zugabe von 0,2 g Dibenzo-18-Krone-6 wird die Reaktionsmischung 60 Minuten unter Rückfluß erhitzt. Nach Abkühlen auf Raumtemperatur wird mit 100 ml Essigsäureethylester verdünnt, mit gesättigter wässriger Ammoniumchlorid-Lösung geschüttelt, mit Natriumsulfat getrocknet und über Kieselgel filtriert. Vom Filtrat wird das Lösungsmittel im Wasserstrahlvakuum sorgfältig abdestilliert.

Man erhält 1,5 g (66 % der Theorie) 1-Cyclopropyl-3-[4-methyl-3-methylthio-5-oxo-4,5-dihydro-[1,2,4]-triazol-1-yl-methyl)-phenyl]-propan-1,3-dion als amorphes Produkt, welches ohne Reinigung weiter umgesetzt werden kann.

### Ausgangsstoffe der Formel (VII):

### Beispiel (VII-1)

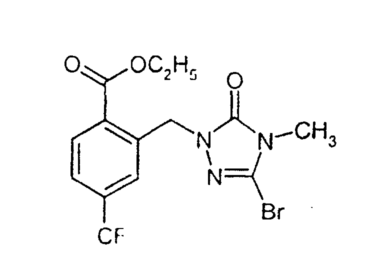

### Stufe 1

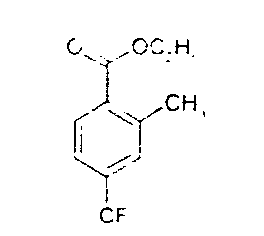

10 g (49 mMol) 2-Methyl-4-trifluormethyl-benzoesäure werden in 150 ml Ethanol gelöst und mit 1 ml konz. Schwefelsäure versetzt. Nach 24 Stunden Erhitzen unter Rückfluß wird die Lösung eingeengt, in Methylenchlorid aufgenommen und mit gesättigter wäßriger Natriumhydrogencarbonat-Lösung extrahiert. Die Methylenchlorid-Phase wird über Natriumsulfat getrocknet und im Wasserstrahlvakuum eingeengt.

Man erhält 9 g (80 % der Theorie) 2-Methyl-4-trifluormethyl-benzoesäure-ethylester als amorphen Rückstand.

### Stufe 2

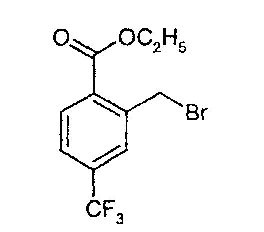

9 g (39 mMol) 2-Methyl-4-trifluormethyl-benzoesäure-ethylester werden in 200 ml Tetrachlormethan gelöst und mit 7 g (39 mMol) *N*-Brom-succinimid und 0.1 g Dibenzoylperoxid versetzt. Nach 6 Stunden Erhitzen unter Rückfluß wird das abgeschiedene Succinimid abfiltriert und das Filtrat im Wasserstrahlvakuum eingeengt.

Man erhält 12 g eines amorphen Rückstandes, der neben 2-Brommethyl-4-trifluormethyl-benzoesäure-ethylester noch 17% 2,2-Dibrommethyl-4-trifluormethylbenzoesäure-ethylester und 12 % 2-Methyl-4-trifluormethyl-benzoesäure-ethylester enthält.

### Stufe 3

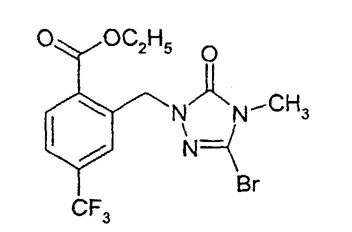

4 g 2-Brommethyi-4-trifluormethyl-benzoesäure-ethylester (ca. 70%ig) und 2.28 g (12,8 mMol) 5-Brom-4-methyl-2,4-dihydro-3H-1,2,4-triazol-3-on werden in 150 ml Acetonitril gelöst, mit 5.3 g (38,4 mMol) Kaliumcarbonat versetzt und unter kräftigem Rühren 2 Stunden zum Rückfluß erhitzt. Das Reaktionsgemisch wird in Wasser aufgenommen und mit Methylenchlorid mehrfach extrahiert. Die gesammelten Methylenchlorid-Phasen werden über Natriumsulfat getrocknet, im Wasserstrahlvakuum eingeengt und chromatographiert.

Man erhält 2 g (38 % der Theorie) 5-Brom-4-methyl-2-(2-ethoxycarbonyl-5-trifluormethyl-benzyl)-2,4-dihydro-3H-1,2,4-triazol-3-on als amorphes Produkt.
¹H-NMR (CDCl₃, δ): 5,46 ppm.

### Beispiel (VII-2)

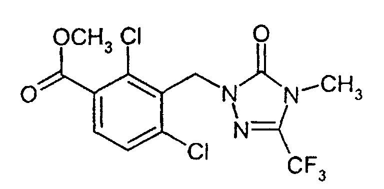

6,7 g (40 mMol) 4-Methyl-5-trifluormethyl-2,4-dihydro-3H-1,2,4-triazol-3-on werden in 150 ml Acetonitril vorgelegt und mit 11 g (80 mMol) Kahumcarbonat verrührt. Nach Erwärmen der Mischung auf 50°C wird dann eine Lösung von 13,1 g (44 mMol) 3-Brommethyl-2,4-dichlor-benzoesäure-methylester in 20 ml Acetonitril unter Rühren tropfenweise dazu gegeben und die Reaktionsmischung wird noch 15 Stunden unter Rühren zum Rückfluß erhitzt. Anschließend wird im Wasserstrahlvakuum eingeengt, der Rückstand in Methylenchlorid aufgenommen, mit 1N-Salzsäure gewaschen, mit Natriumsulfat getrocknet und filtriert. Das Filtrat wird unter vermindertem Druck eingeengt, der Rückstand mit Petrolether digeriert und das kristallin angefallene Produkt durch Absaugen isoliert.

Man erhält 14,9 g (97 % der Theorie) 4-Methyl-5-trifluormethyl-2-(2,6-dichlor-3-methoxycarbonyl-benzyl)-2,4-dihydro-3H-1,2,4-triazol-3-on vom Schmelzpunkt 109°C.

Analog zu den Beispielen (VII-1) und (VII-2) können beispielsweise auch die in der nachstehenden Tabelle 3 aufgeführten Verbindungen der allgemeinen Formel (VII) hergestellt werden.

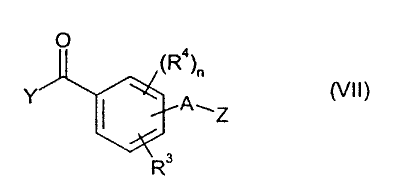

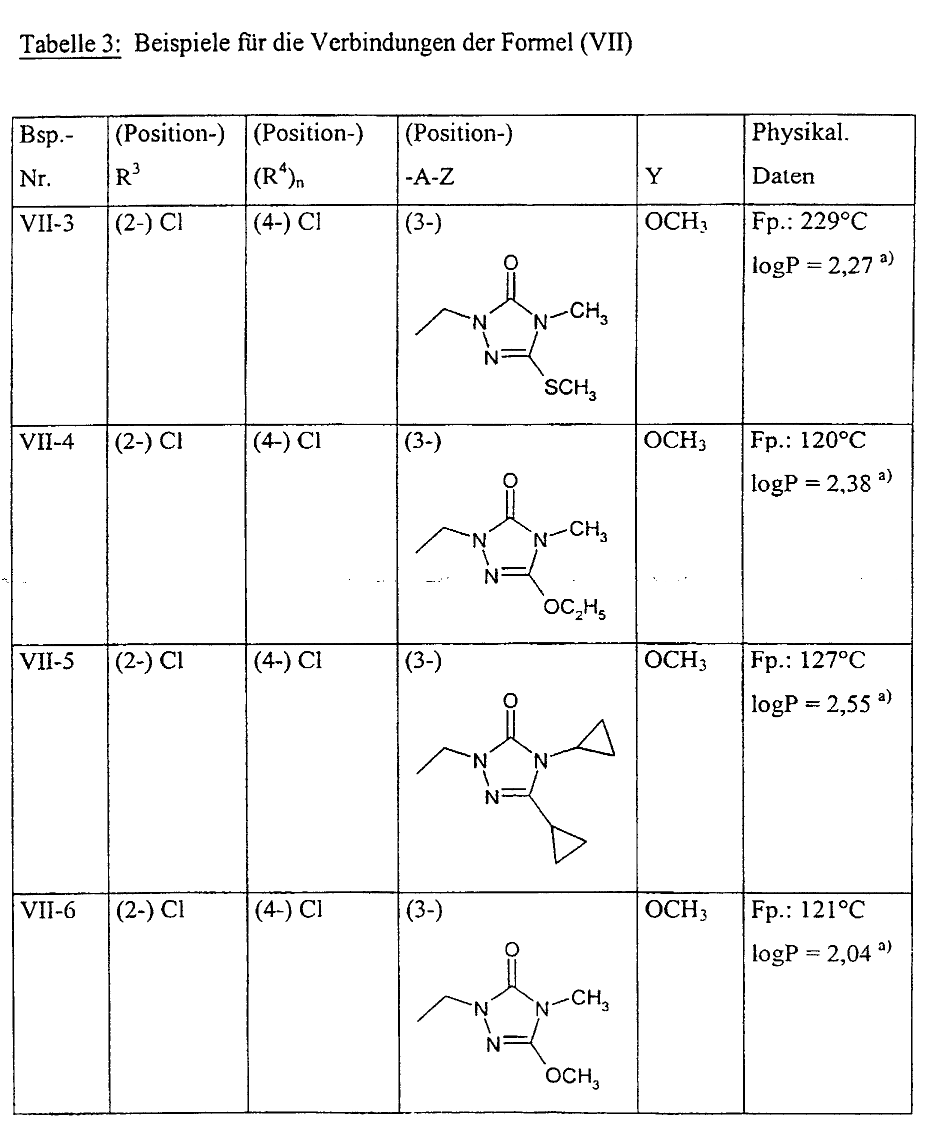

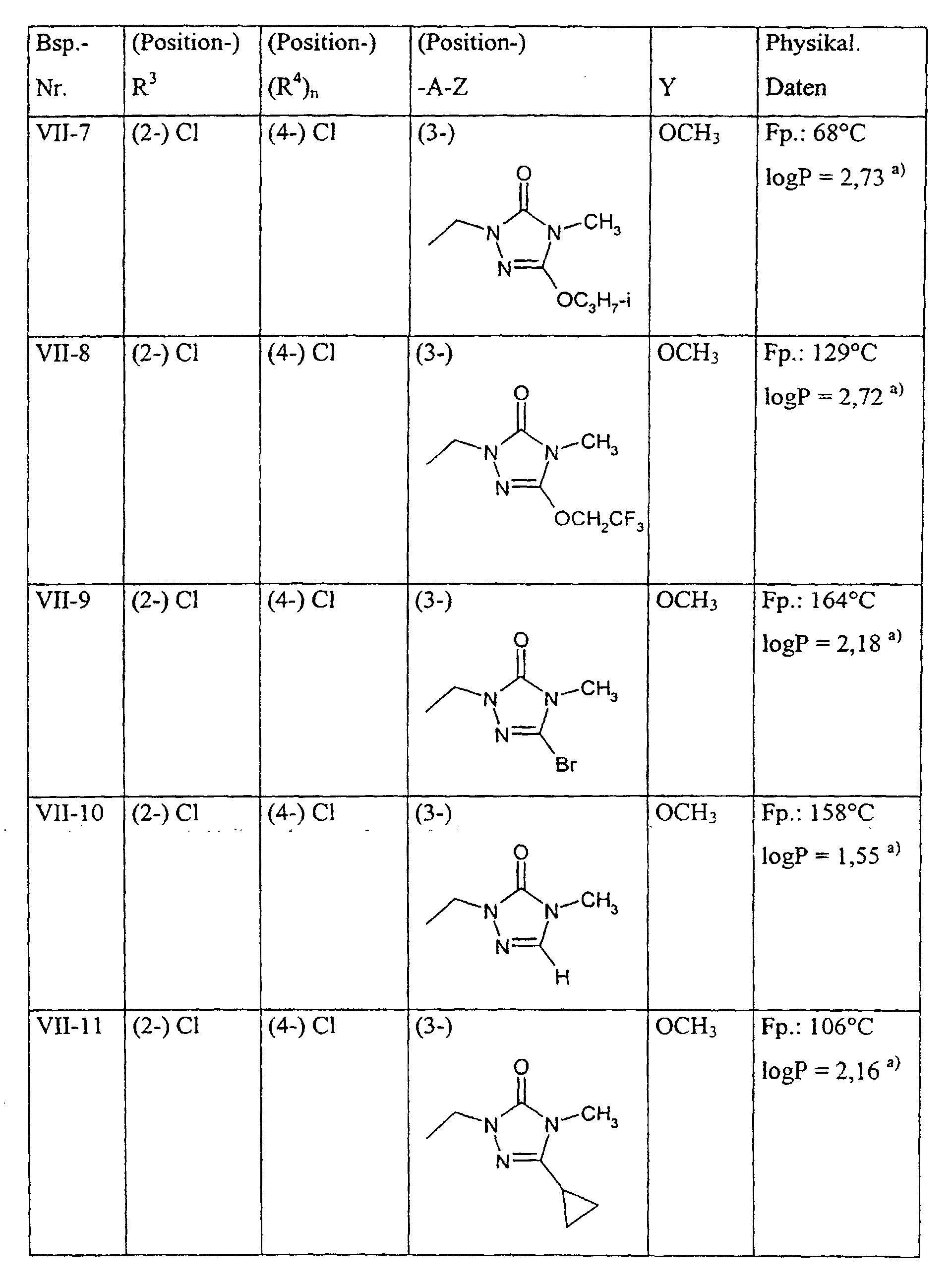

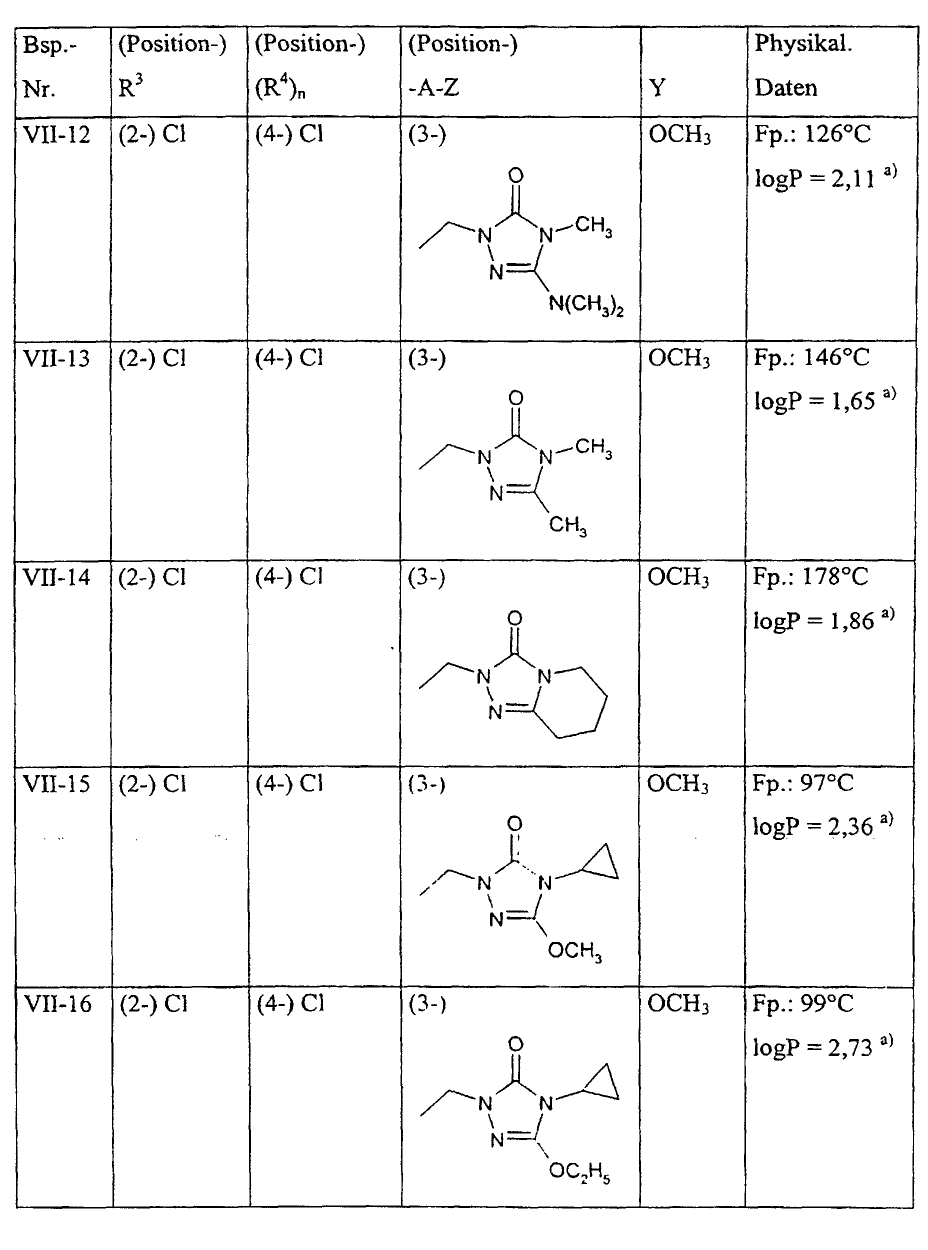

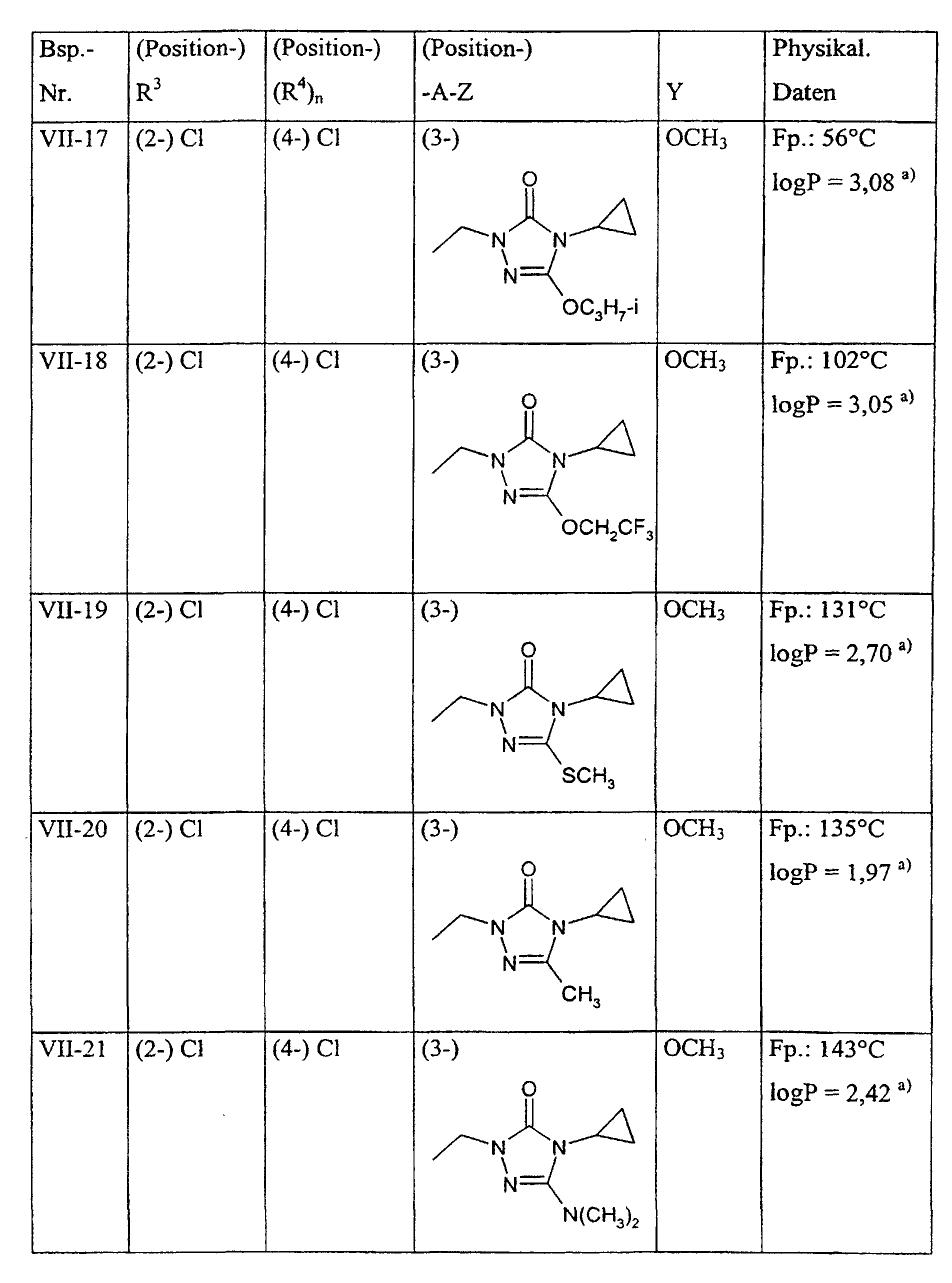

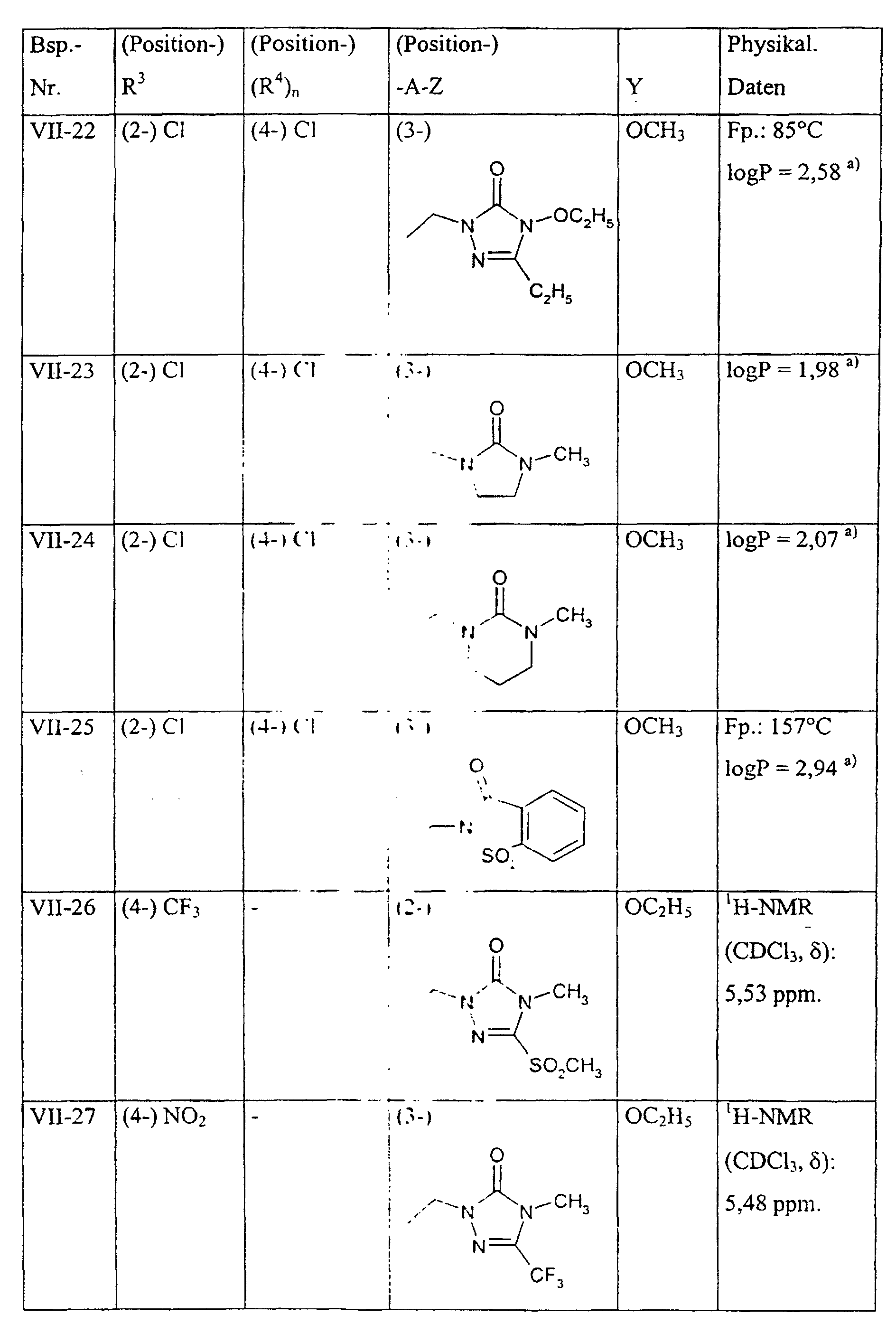

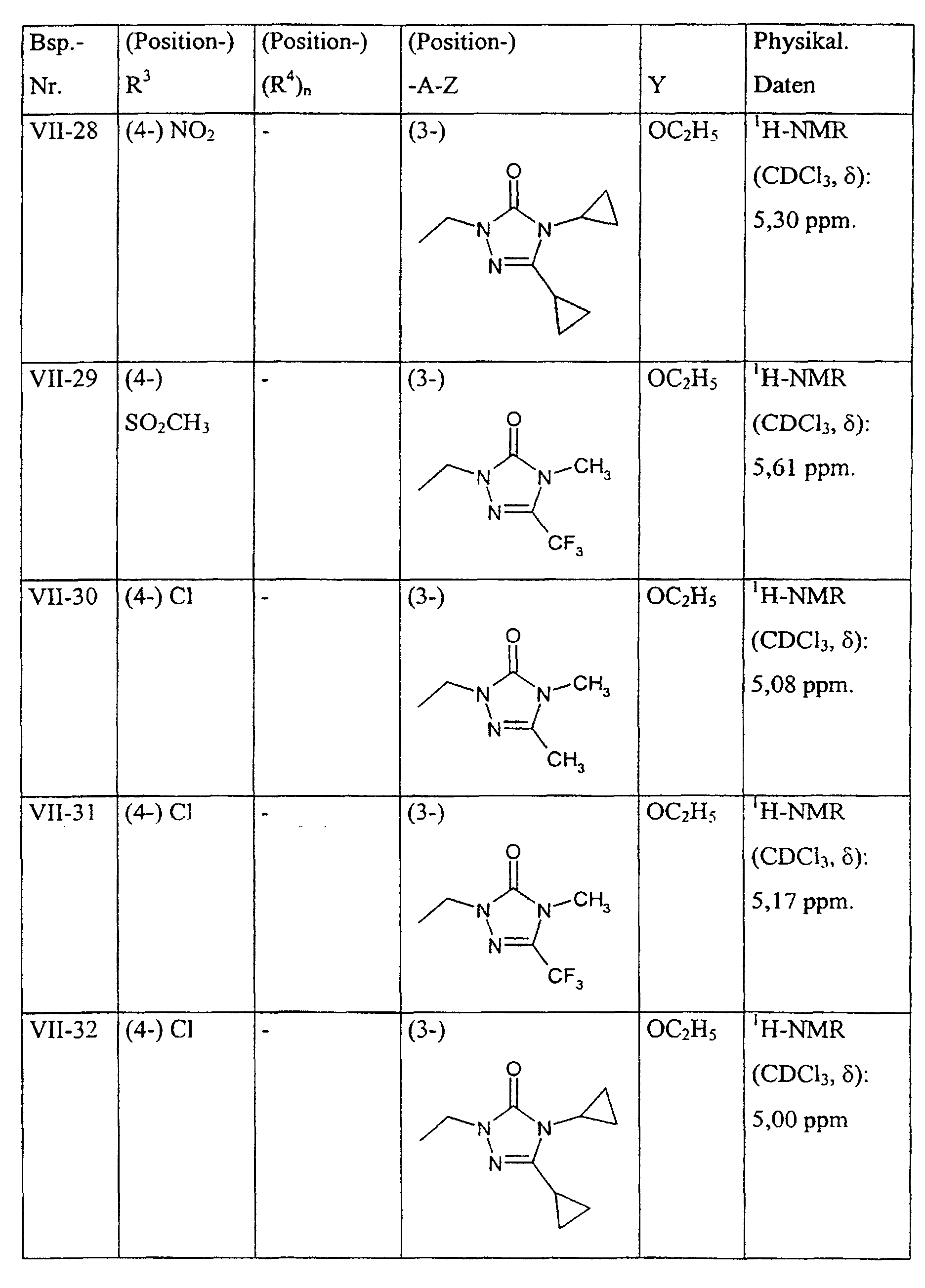

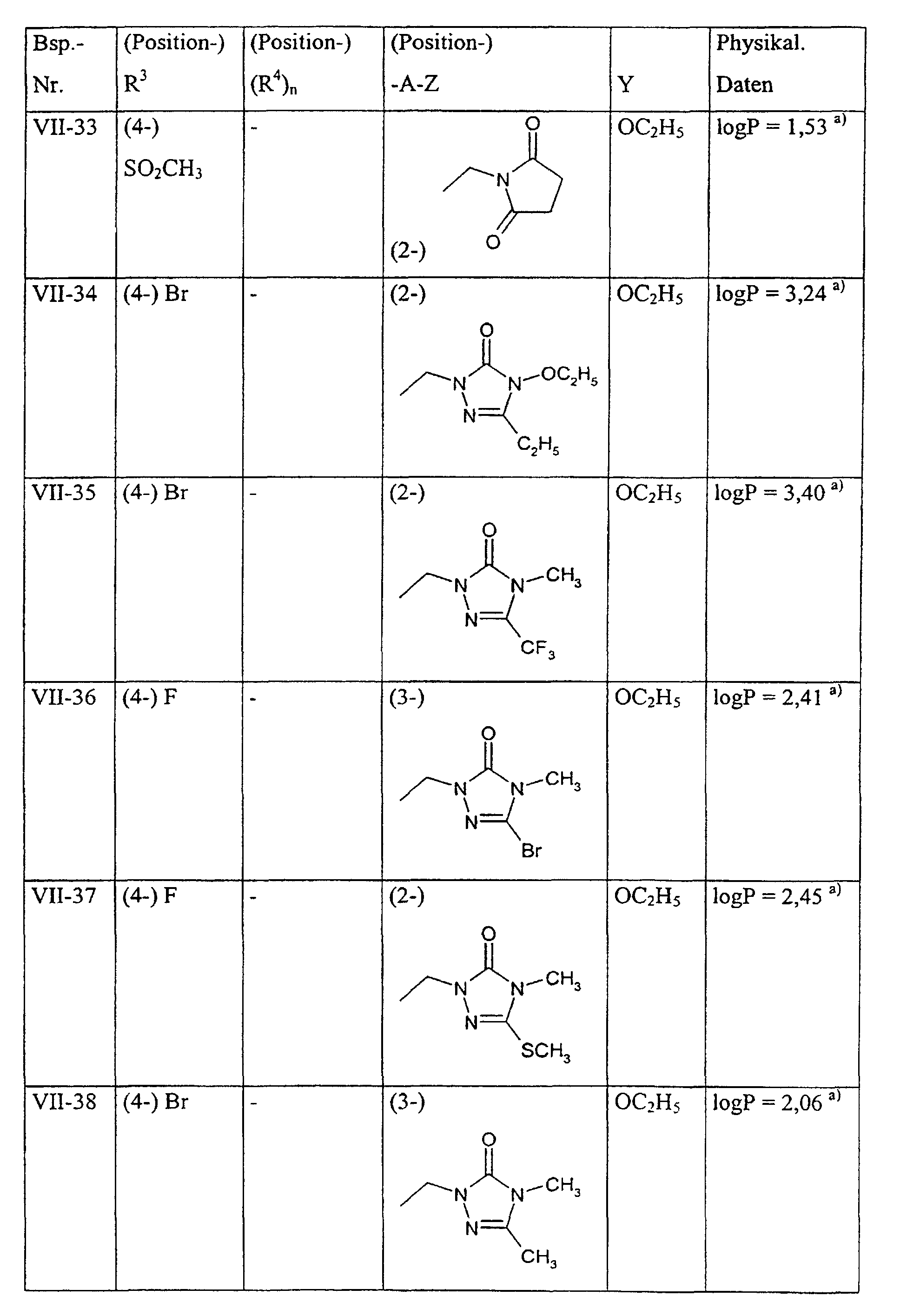

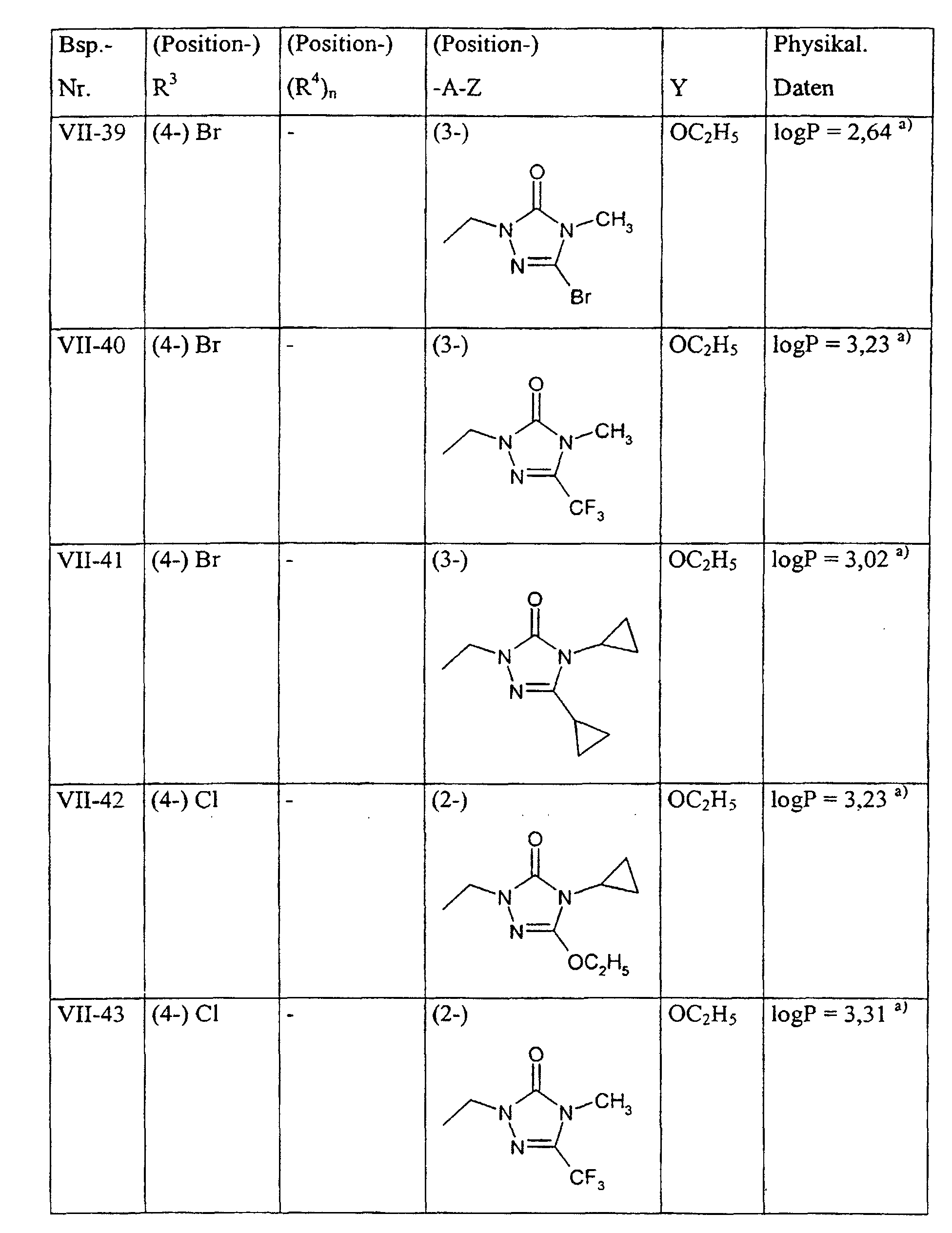

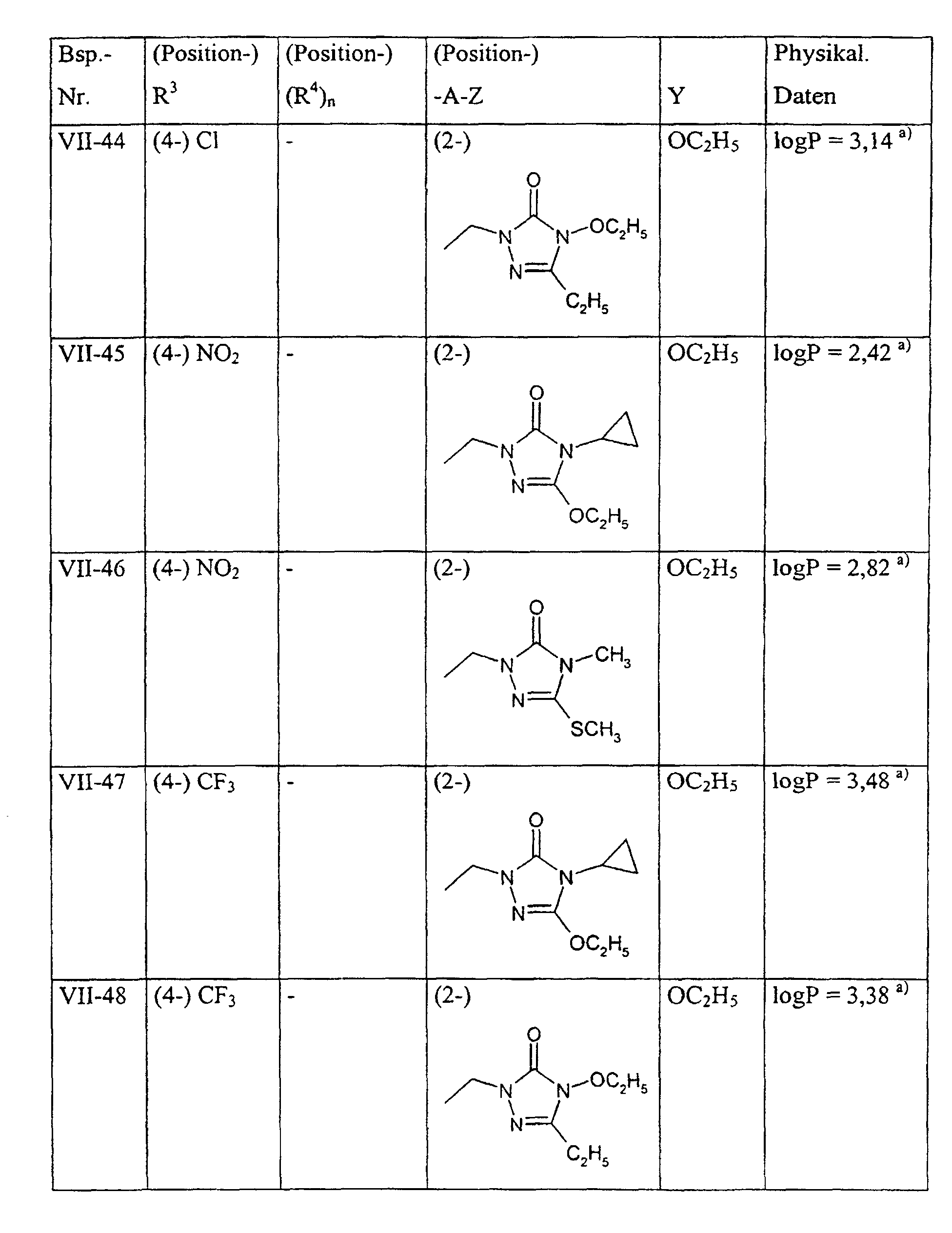

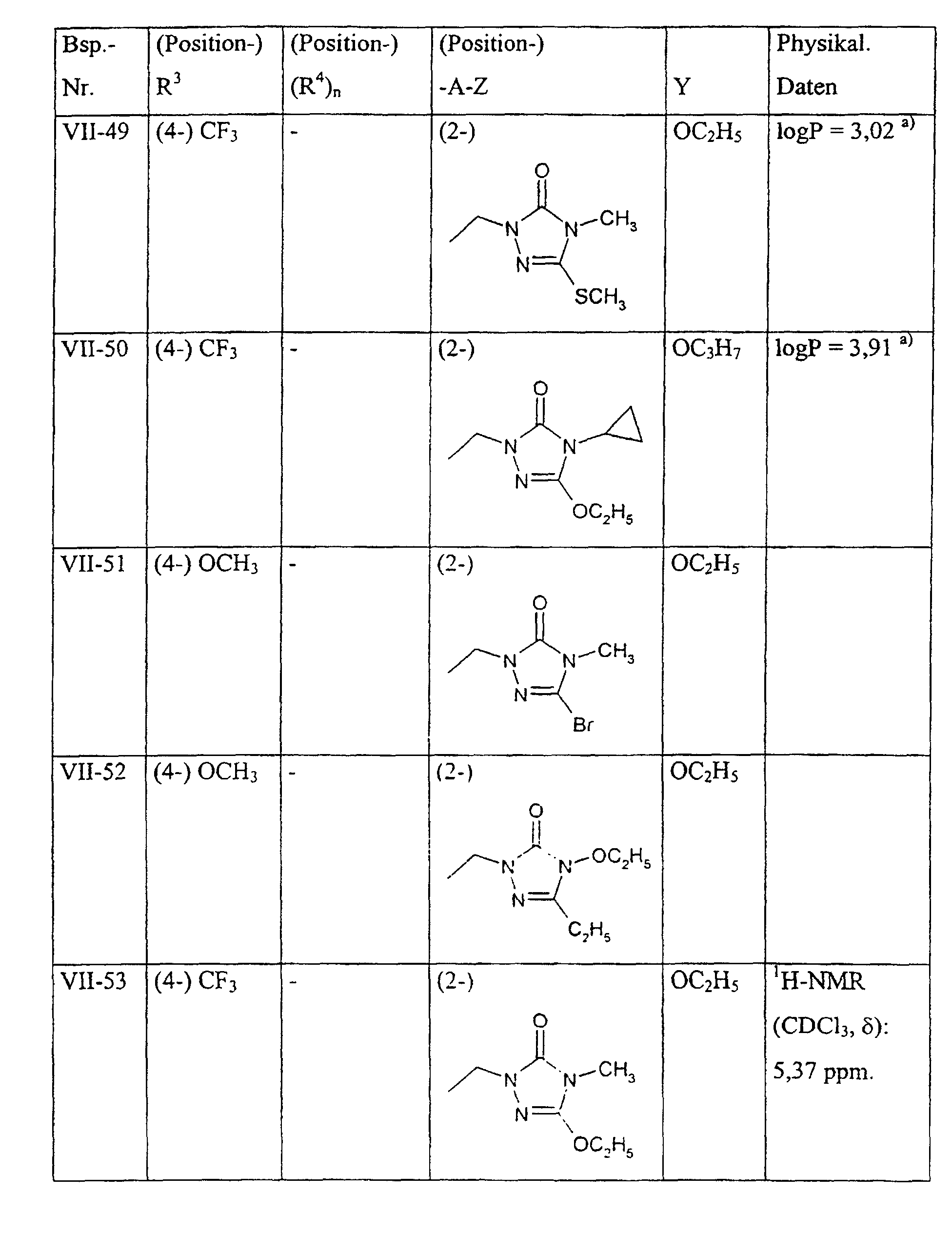

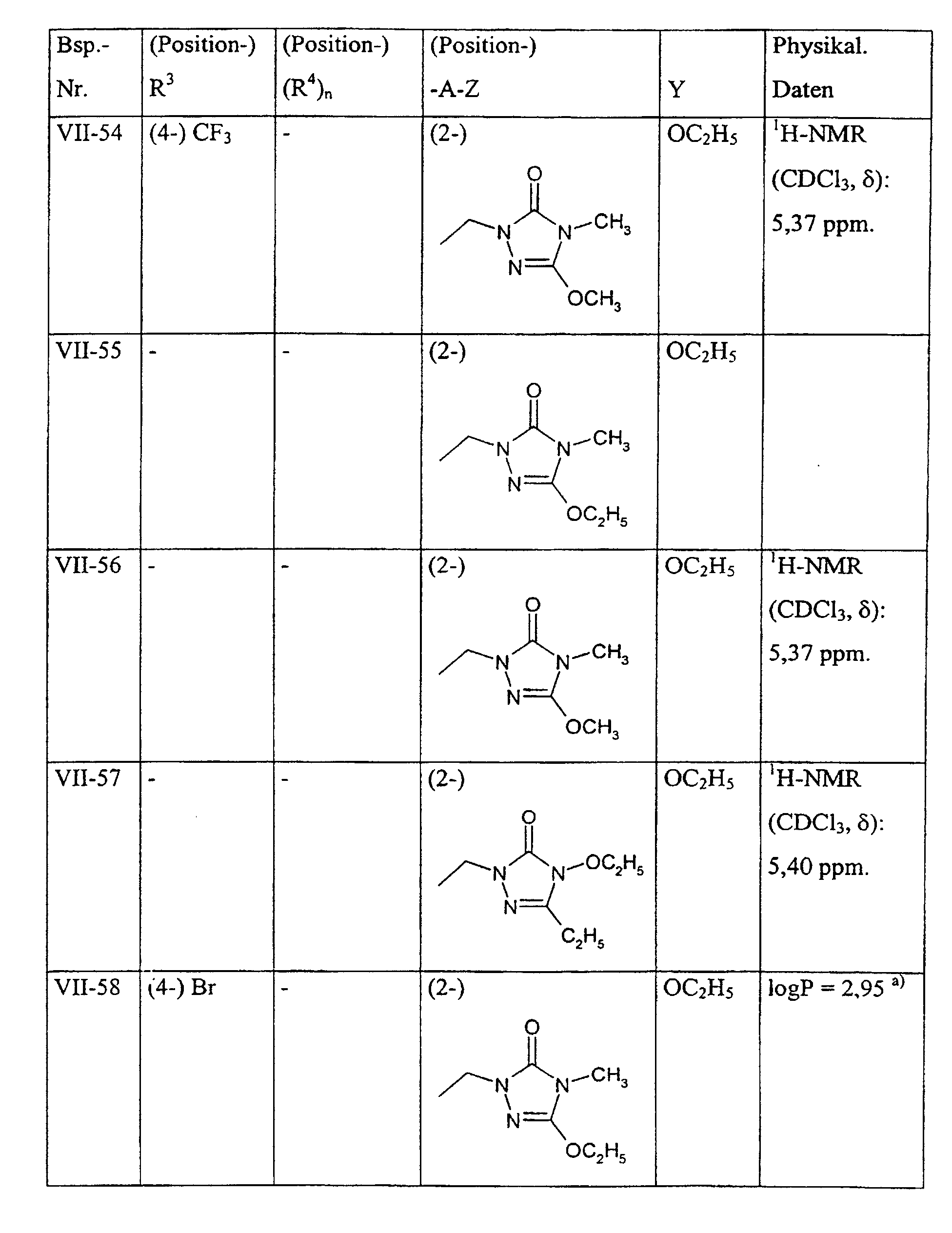

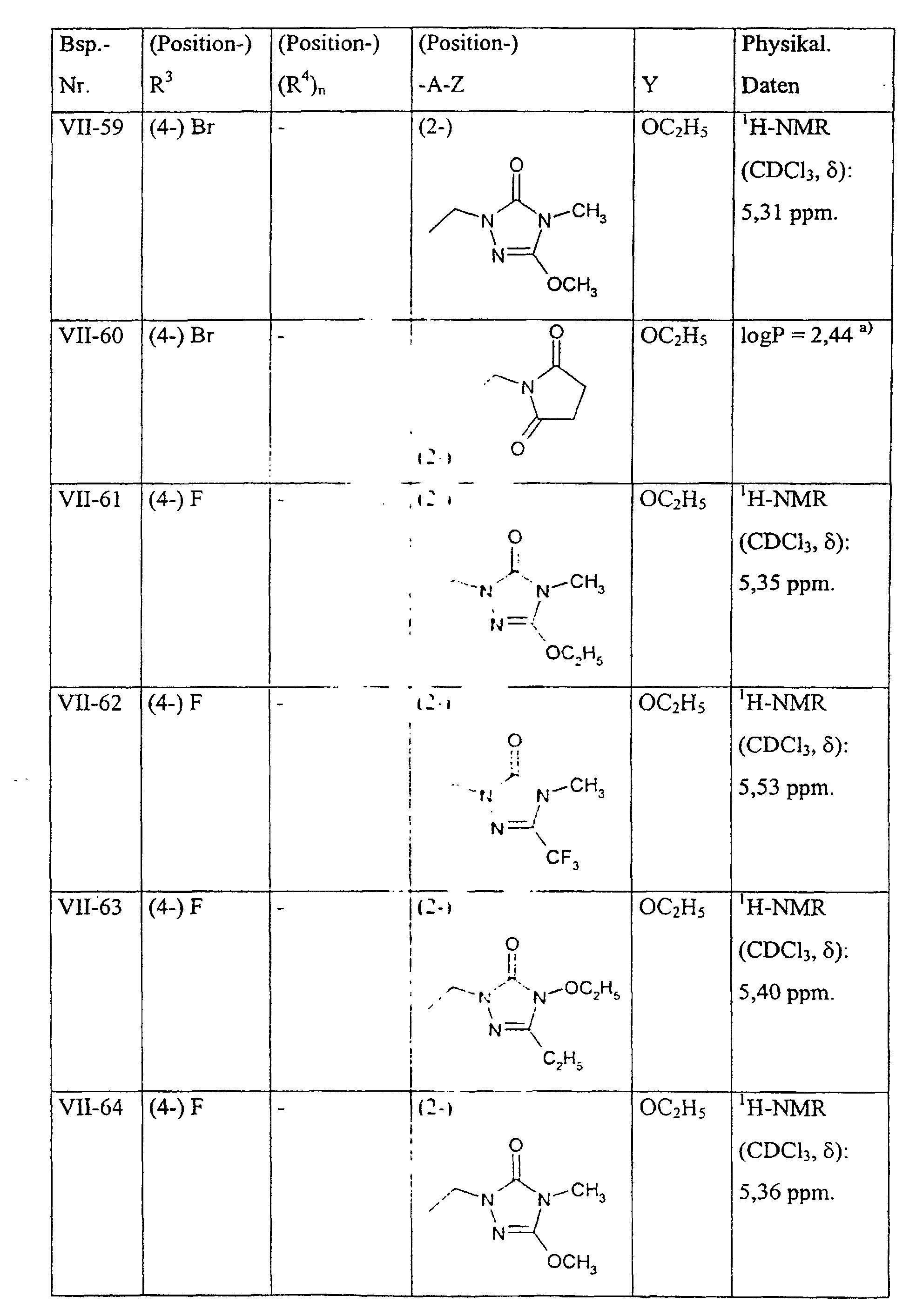

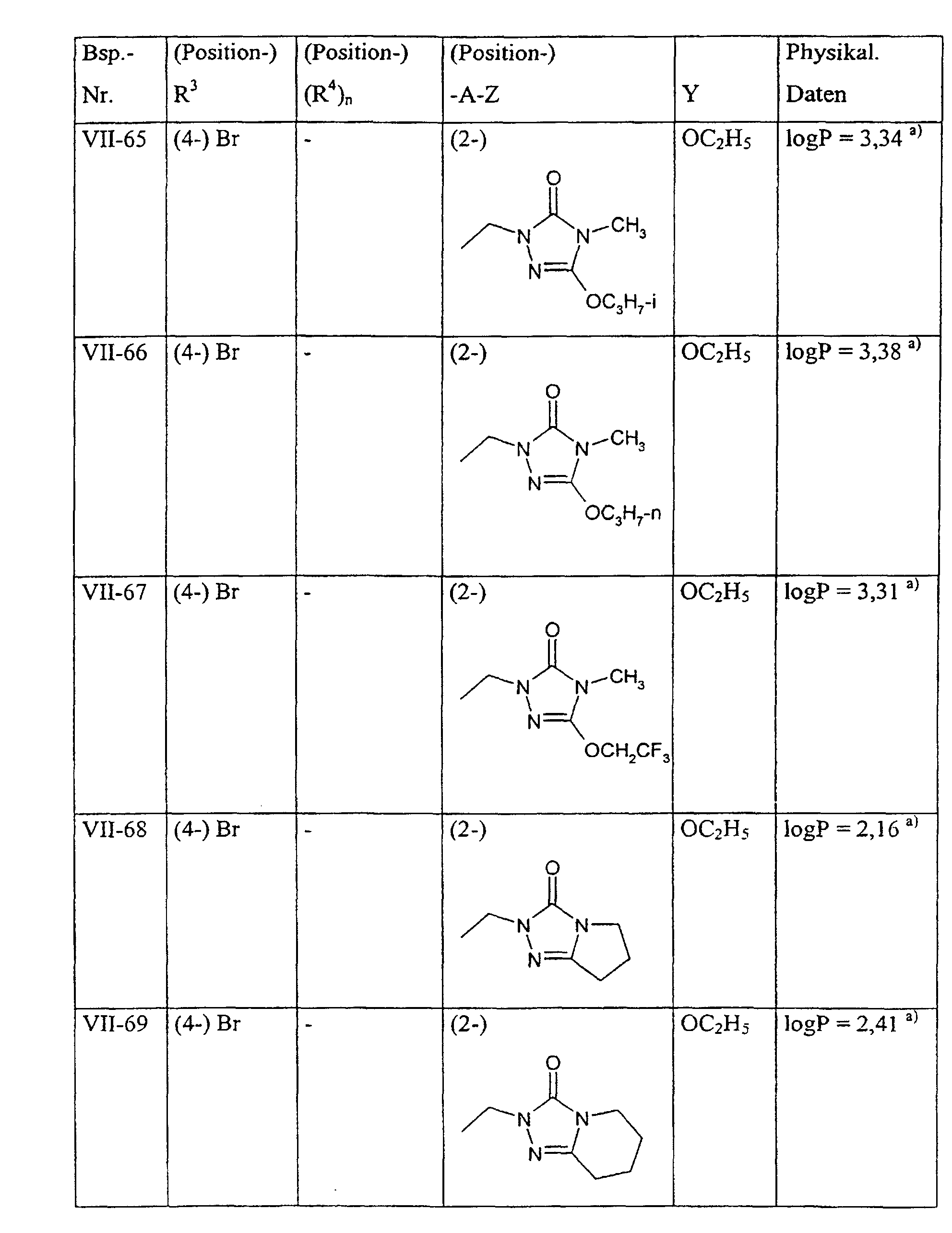

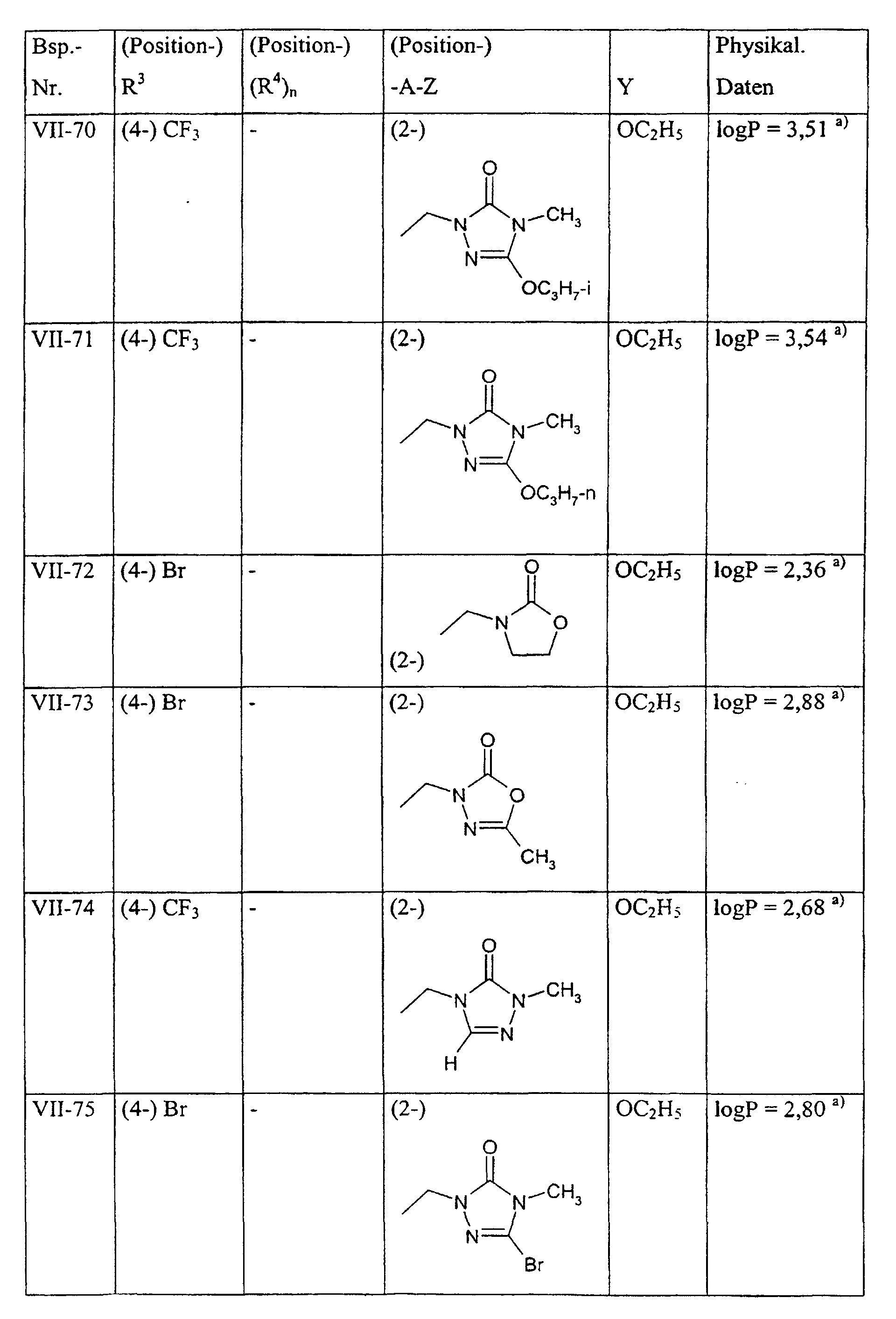

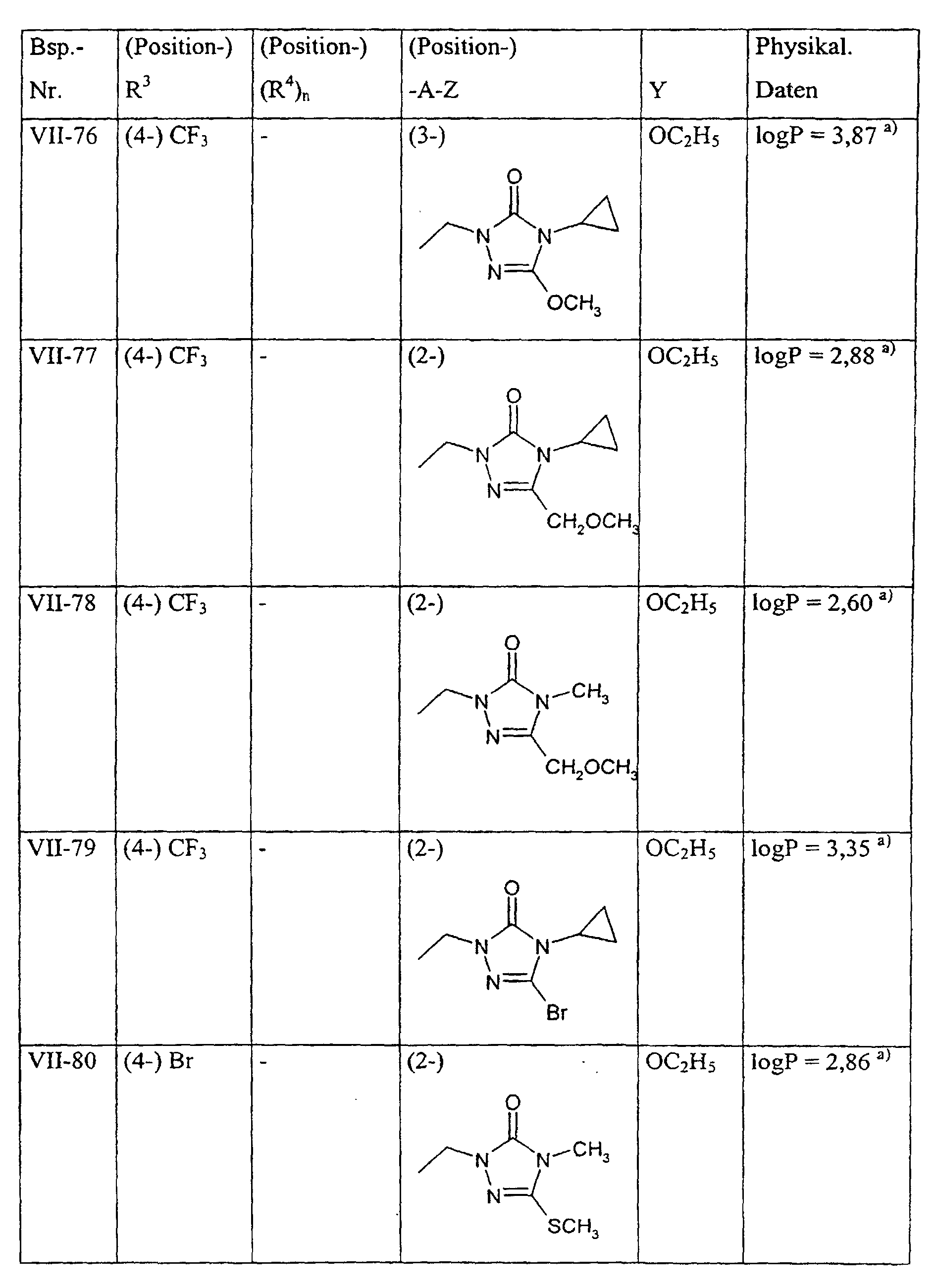

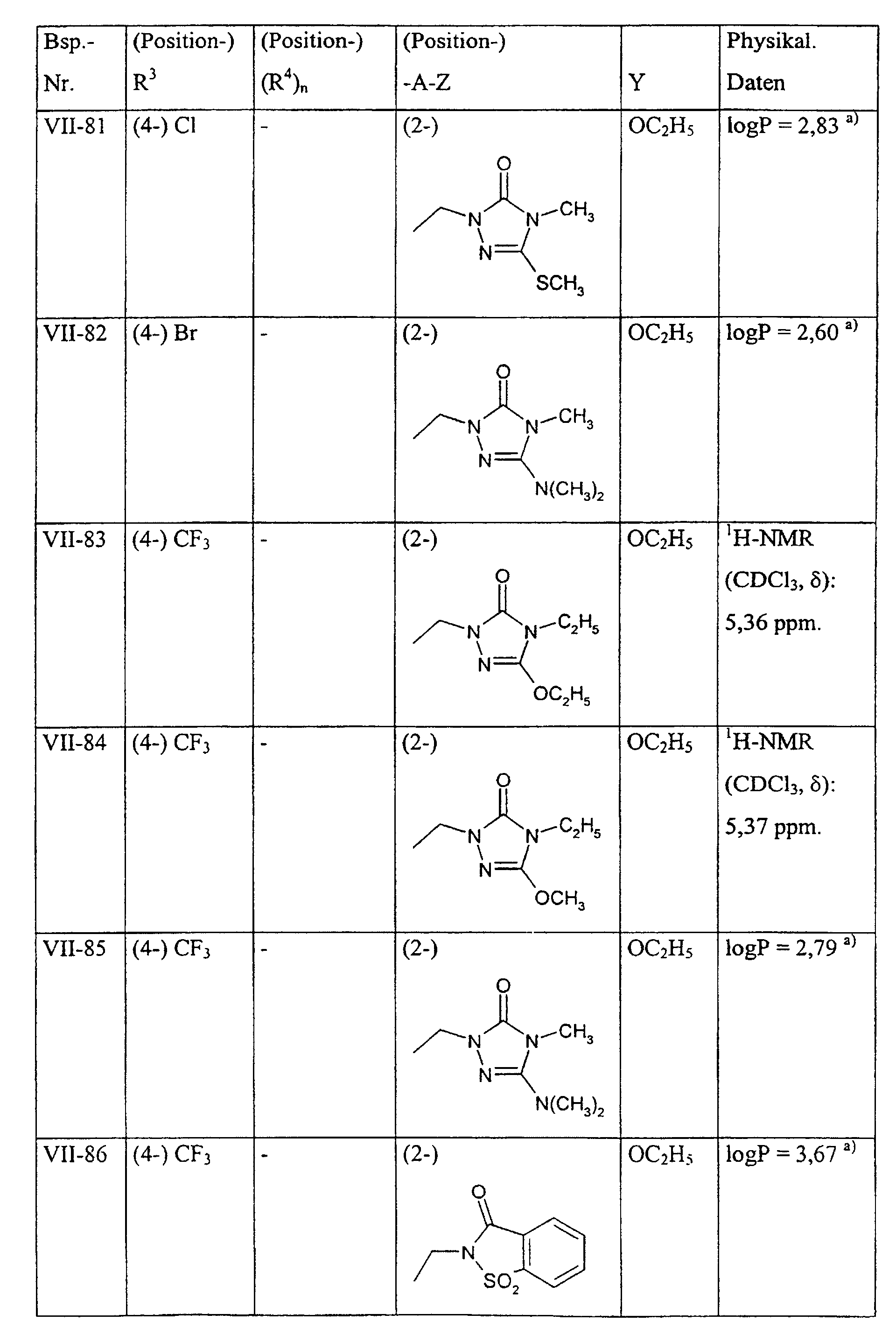

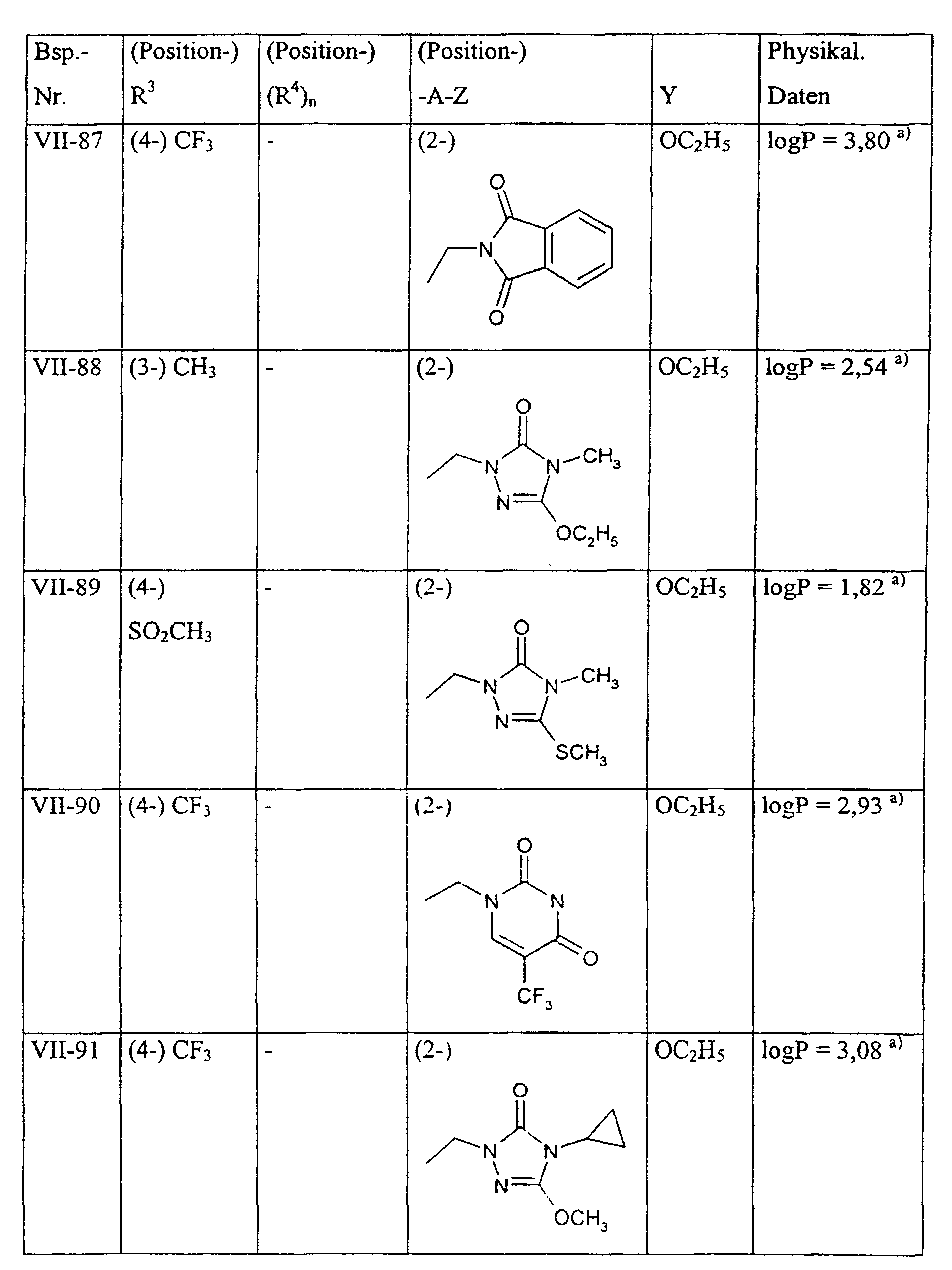

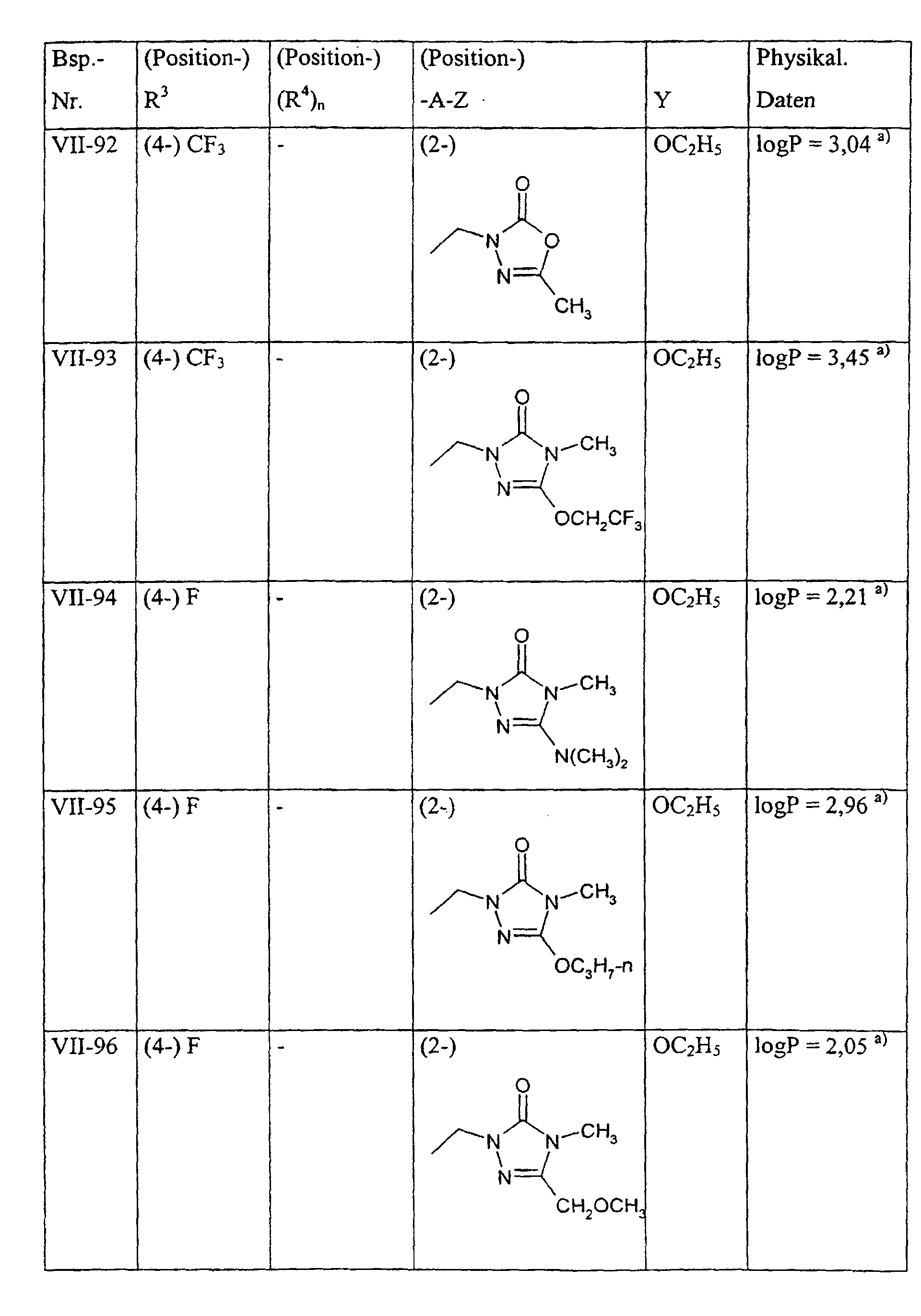

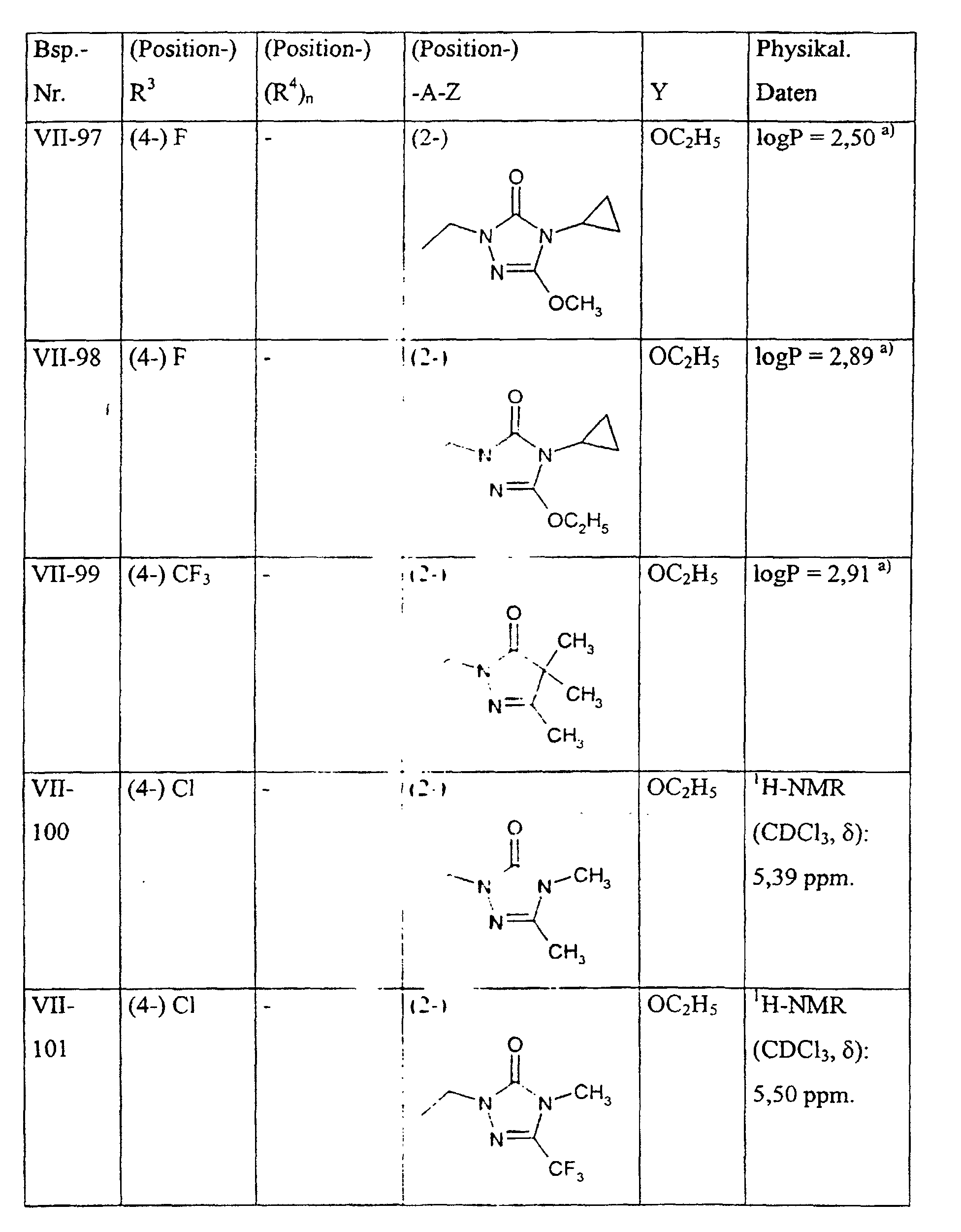

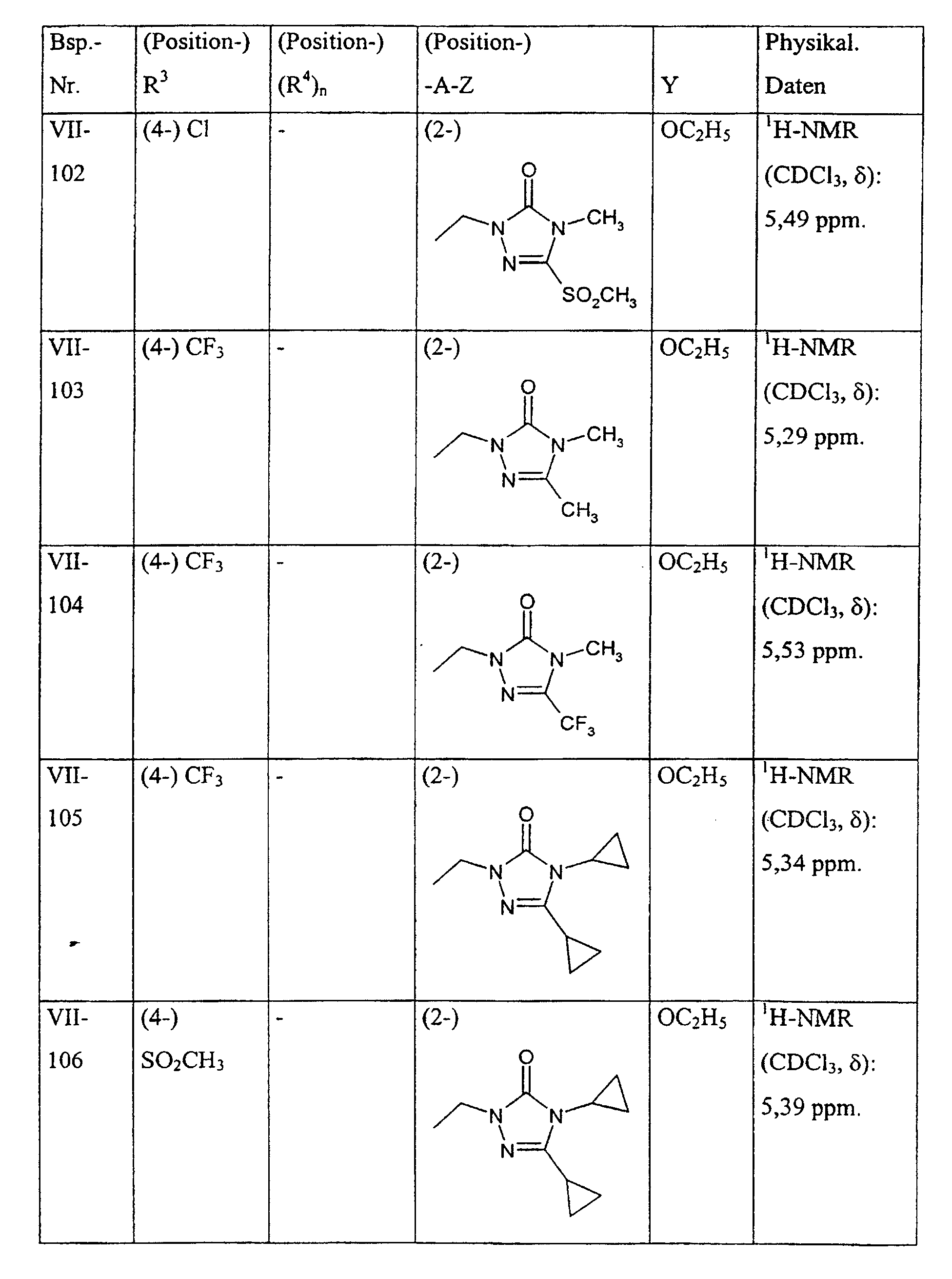

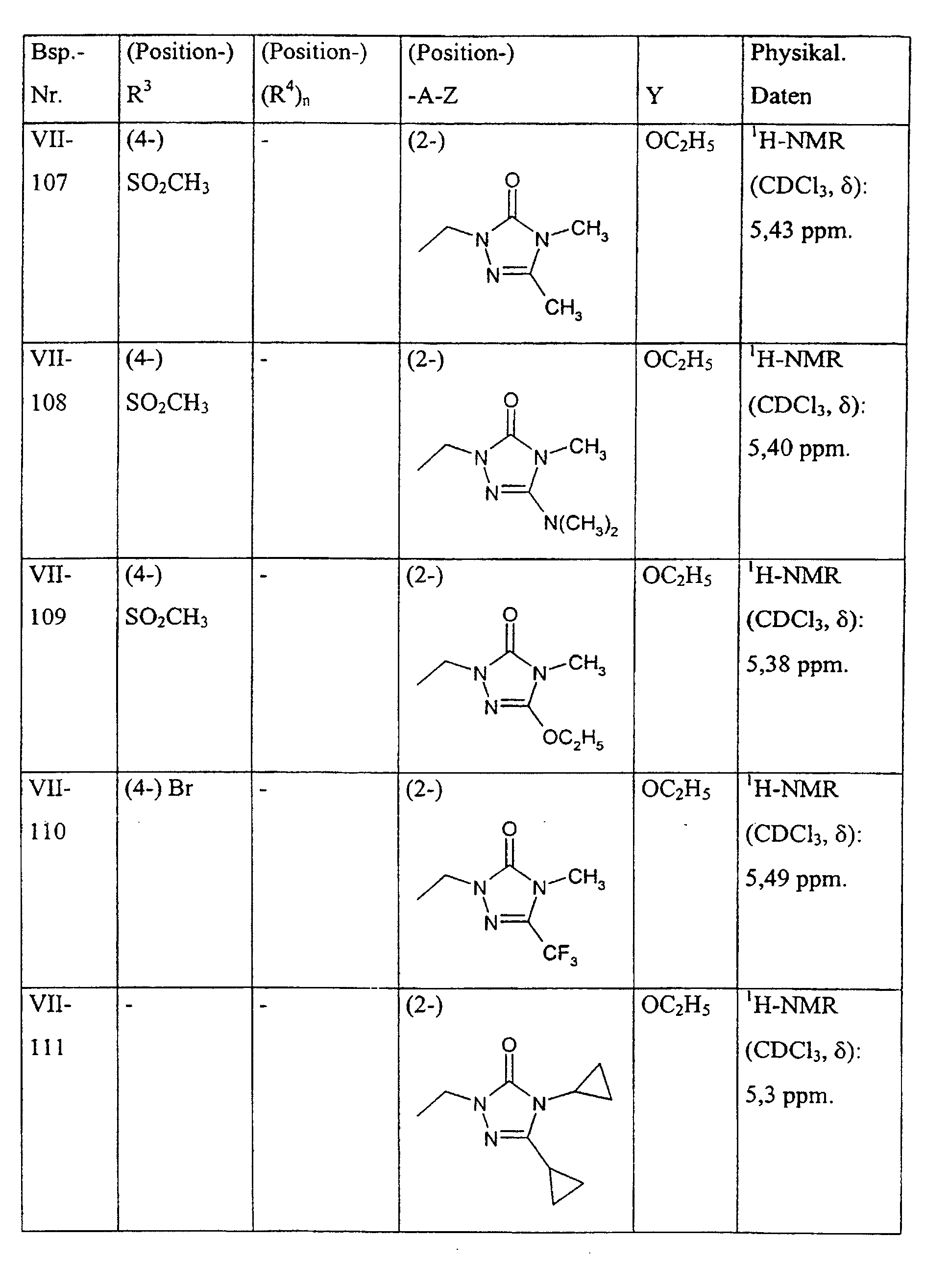

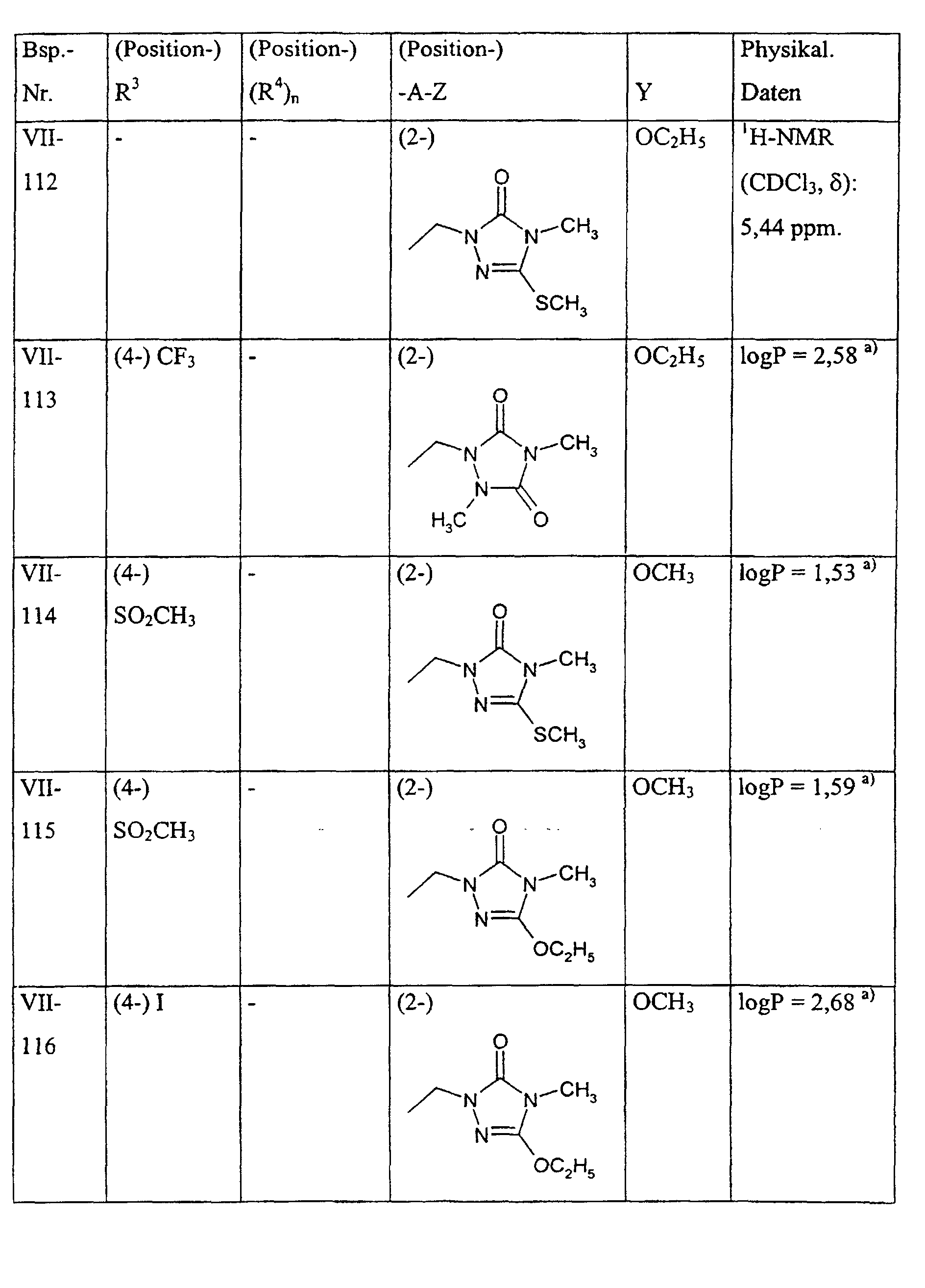

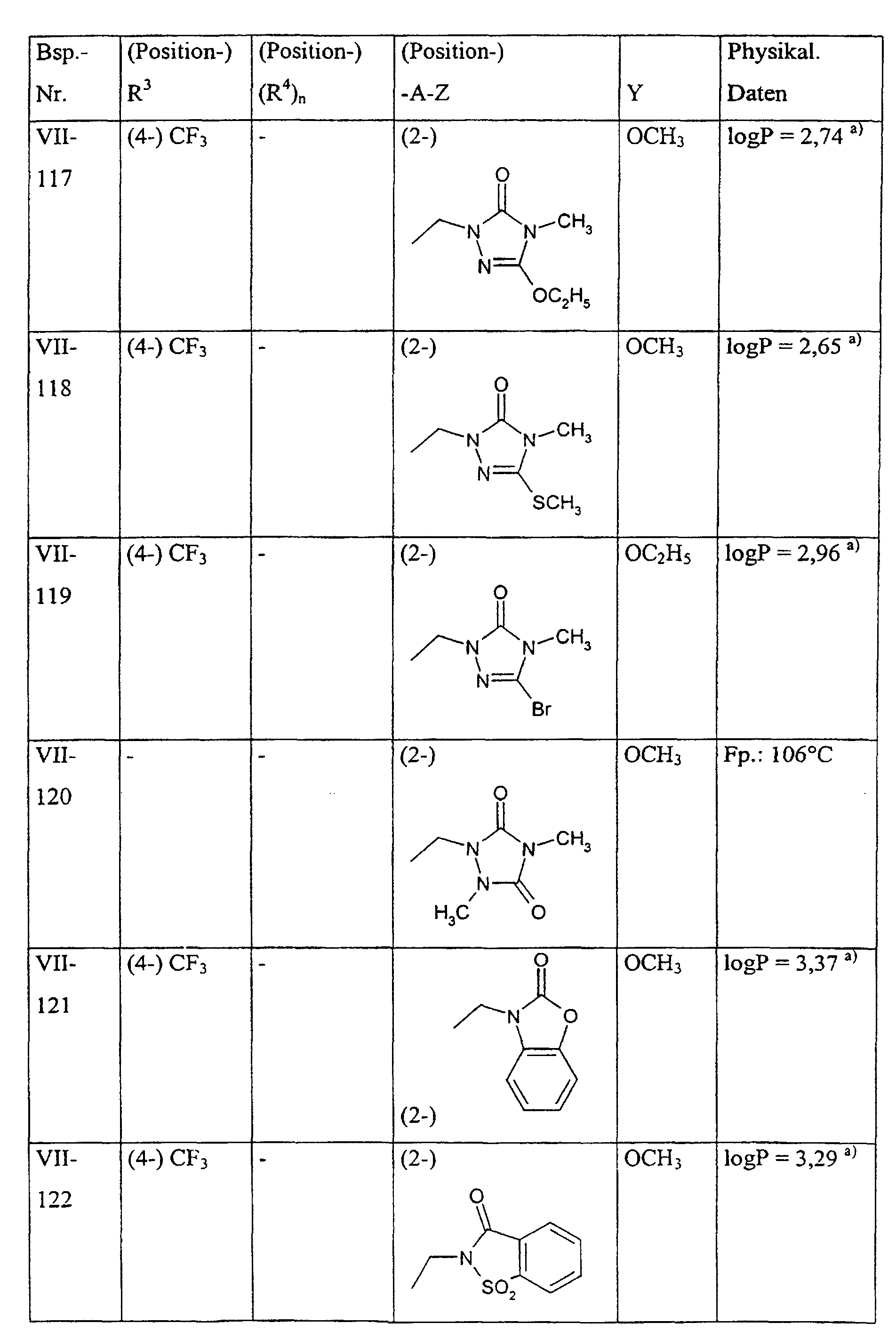

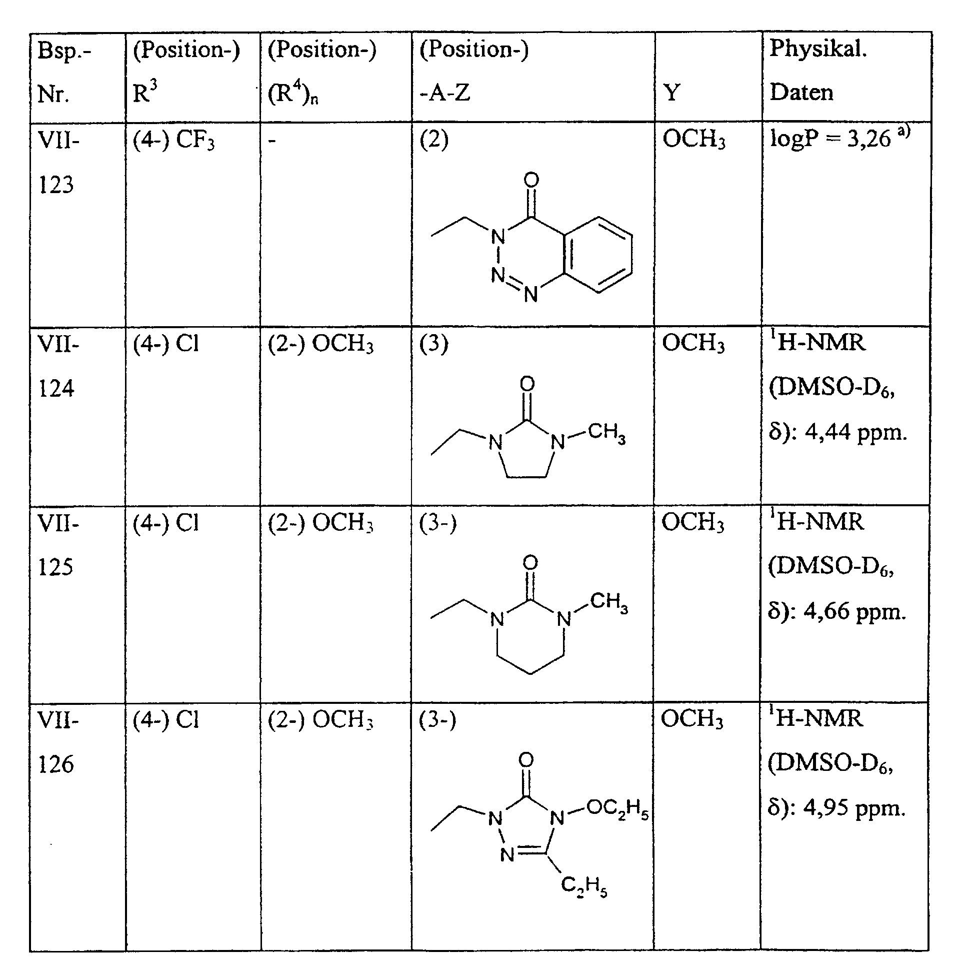

Die Bestimmung der in Tabelle 3 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10 % Acetonitril bis 90 % Acetonitril - entsprechende Messergebnisse sind in Tabelle 3 mit ^{a)} markiert.
(b) Eluenten für die Bestimmung im neutralen Bereich: 0,01-molare wässrige Phosphatpuffer-Lösung, Acetonitril; linearer Gradient von 10 % Acetonitril bis 90 % Acetonitril - entsprechende Messergebnisse sind in Tabelle 3 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird

Nach drei Wochen wird der Schadigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 3 und 4 bei guter Verträglichkeit gegenüber Kulturpflanzen, wie z.B. Mais, starke Wirkung gegen Unkräuter.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 3 und 4 bei guter Verträglichkeit gegenüber Kulturpflanzen, wie z.B. Weizen, sehr starke Wirkung gegen Unkräuter.

## Patentansprüche

1. Verbindungen der allgemeinen Formel (I), 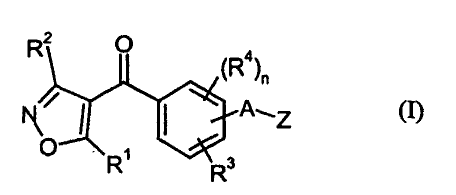 in welcher
n für die Zahlen 0, 1, 2 oder 3 steht,
A für Methylen, Ethyliden (Ethan-1,1-diyl) oder Dimethylen (Ethan-1,2-diyl) steht,
R¹ für Wasserstoff, für jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, für jeweils gegebenenfallsdurch Fluor, Chlor oder Brom substituiertes Propenyl,
Butenyl, Propinyl oder Butinyl, oder für jeweils gegebenenfalls durch Cyano, Fluor, Chlor, Brom, Methyl oder Ethyl substituiertes Cyclopropyl, Cyclobutyl, Cyclopentyl oder Cyclohexyl steht,
R² für Wasserstoff, Cyano, Carbamoyl, Fluor, Chlor, Brom, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxy, Ethoxy, n- oder i-Propoxy, Methoxycarbonyl, Ethoxycarbonyl, n- oder i-Propoxycarbonyl, oder für jeweils gegebenenfalls durch Fluor und/oder Chlor substituiertes Methylthio, Ethylthio, n- oder i-Propylthio steht,
R³ für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder fiir Methylamino, Ethylamino, n- oder i-Propylamino, Dimethylamino, Diethylamino, Dimethylaminosulfonyl oder Diethylaminosulfonyl steht,
R⁴ für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
Z für eine der Gruppierungen 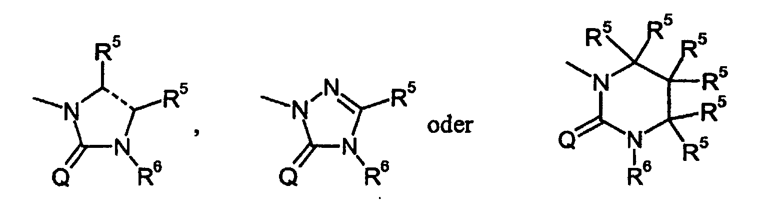 steht,
R⁵ für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino oder Butenylamino, fiir jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder fiir jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino steht, für Pyrrolidino, Piperidino oder Morpholino steht, oder - für den Fall, daß zwei benachbarte Reste R⁵ und R⁵ sich an einer Doppelbindung befinden - zusammen mit dem benachbarten Rest R⁵ auch für eine Benzogruppierung steht,
R⁶ für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl steht, oder zusammen mit einem benachbarten Rest R⁵ oder R⁶ für jeweils gegebenenfalls durch Methyl und/oder Ethyl substituiertes Propan-1,3-diyl (Trimethylen) oder Butan-1,4-diyl (Tetramethylen) steht, und
Q für Sauerstoff oder Schwefel steht.

2. Verbindungen gemäß Anspruch 1, **dadurch gekennzeichnet, daß**
R¹ für Wasserstoff, für jeweils gegebenenfalls durch Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s-oder t-Butyl, oder für gegebenenfalls durch Cyano, Fluor, Chlor, Brom, Methyl oder Ethyl substituiertes Cyclopropyl steht,
R² für Wasserstoff, Cyano, Carbamoyl, Fluor, Chlor, Brom, fiir jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxycarbonyl, Ethoxycarbonyl, n- oder i-Propoxycarbonyl, oder für jeweils gegebenenfalls durch Fluor und/oder Chlor substituiertes Methylthio, Ethylthio, n- oder i-Propylthio steht,
R³ für Wasserstoff, Nitro, Cyano, Fluor, Chlor, Brom, Iod, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁴ für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁵ für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy steht, und
R⁶ für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl steht, oder zusammen mit R⁵ fiir Propan-1,3-diyl (Trimethylen), Butan-1,4-diyl (Tetramethylen) oder Pentan-1,5-diyl (Pentamethylen) steht.

3. Verbindungen gemäß einem der Ansprüche 1 bis 2, **dadurch gekennzeichnet, daß**
A für Methylen steht.

4. Verbindungen gemäß einem der Ansprüche 1 bis 3, **dadurch gekennzeichnet, daß**
Q für Sauerstoff (O) steht.

5. Verbindungen gemäß einem der Ansprüche 1 bis 4, **dadurch gekennzeichnet, daß**
R¹ für Cyclopropyl steht.

6. Verbindungen gemäß einem der Ansprüche 1 bis 5, **dadurch gekennzeichnet, daß**
R² für Wasserstoff, Methoxycarbonyl oder Ethoxycarbonyl steht.

7. Verbindungen gemäß einem der Ansprüche 1 bis 6, **dadurch gekennzeichnet, daß**
R⁶ für Methyl, Dimethylamino oder Cyclopropyl steht.

8. Verbindungen gemäß einem der Ansprüche 1 bis 7, **dadurch gekennzeichnet, daß**
R³ für Chlor, Brom, Cyano, Trifluormethyl oder Methylsulfonyl steht.

9. Verbindungen gemäß einem der Ansprüche 1 bis 8, **dadurch gekennzeichnet, daß**
R⁴ für Wasserstoff, Cyano, Chlor, Nitro, Methyl, Trifluormethyl, Methoxy oder Methylsulfonyl steht.

10. Verbindungen gemäß einem der Ansprüche 1 bis 9 mit der allgemeinen Formel (IA) 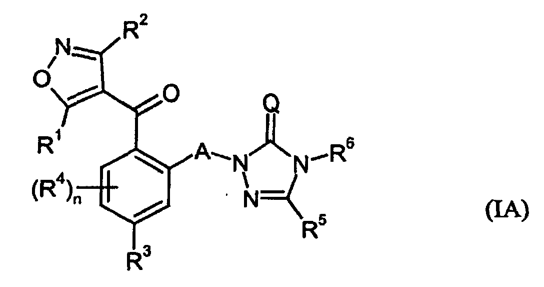 in welcher
n, A, Q, R¹, R², R³, R⁴, R⁵ und R⁶ die in einem der Ansprüche 1 bis 9 angegebene Bedeutung haben.

11. Verbindungen gemäß einem der Ansprüche 1 bis 9 mit der allgemeinen Formel (TB) 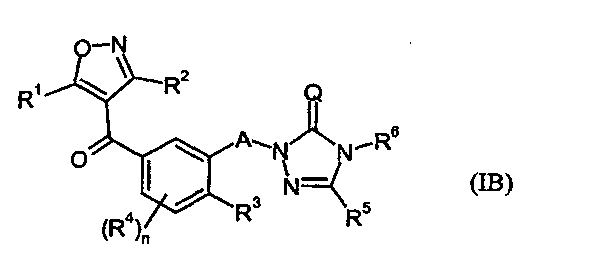 in welcher
n, A, Q, R¹, R², R³, R⁴, R⁵ und R⁶ die in einem der Ansprüche 1 bis 9 angegebene Bedeutung haben.

12. Verbindungen gemäß einem der Ansprüche 1 bis 9 mit der allgemeinen Formel (IC) 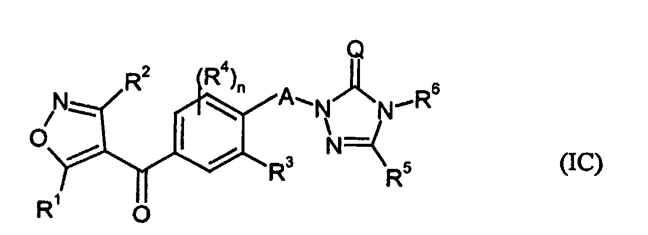 in welcher
n, A, Q, R¹, R², R³, R⁴, R⁵ und R⁶ die in einem der Ansprüche 1 bis 9 angegebene Bedeutung haben.

13. Verfahren zum Herstellen von Verbindungen gemäß einem der Ansprüche 1 bis 12, **dadurch gekennzeichnet, daß** man
(a) Benzoylisoxazole der allgemeinen Formel (II) 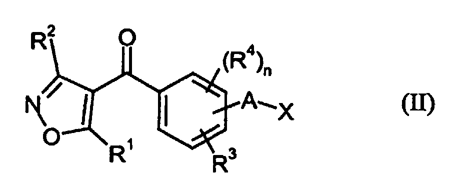 in welcher
n, A, R¹, R², R³ und R⁴ die in einem der Ansprüche 1 bis 3, 5, 6, 8 und 9 angegebene Bedeutung haben und
X für Halogen steht,
mit Heterocyclen der allgemeinen Formel (III)
H-Z (III)
in welcher
Z die in Anspruch 1 angegebene Bedeutung hat,
gegebenenfalls in Gegenwart eines oder mehrerer Reaktionshilfsmittel und gegebenenfalls in Gegenwart eines oder mehrerer Verdünnungsmittel umsetzt,
oder daß man
- für den Fall, daß R² für Wasserstoff steht -
(b) Benzoylketone der allgemeinen Formel (IV) 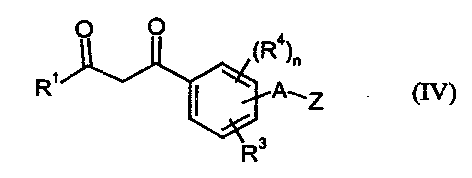 in welcher
n, A, R¹, R³, R⁴ und Z die in einem der Ansprüche 1 bis 3, 5, 8 und 9 angegebene Bedeutung haben,
mit einem Orthoameisensäureester oder einem N,N-Dimethyl-formamidacetal
und anschließend mit Hydroxylamin oder einem Säureaddukt hiervon gegebenenfalls in Gegenwart eines oder mehrerer Reaktionshilfsmittel und gegebenenfalls in Gegenwart eines oder mehrerer Verdünnungsmittel umsetzt,
oder daß man
- für den Fall, daß R² für gegebenenfalls substituiertes Alkoxycarbonyl steht -
(c) Benzoylketone der allgemeinen Formel (IV) 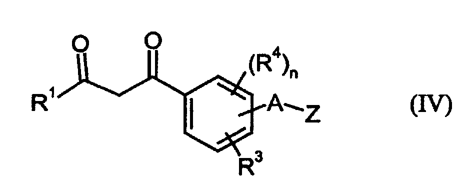 in welcher
n, A, R¹, R³, R⁴ und Z die in einem der Ansprüche 1 bis 3, 5, 8 und 9 angegebene Bedeutung haben,
mit einem Cyanoameisensäureester und anschließend mit Hydroxylamin oder einem Säureaddukt hiervon, oder mit einem Chlor-hydroximino-essigsäurealkylester
gegebenenfalls in Gegenwart eines oder mehrerer Reaktionshilfsmittel und gegebenenfalls in Gegenwart eines oder mehrerer Verdünnungsmittel umsetzt,
oder daß man
- für den Fall das R² für Alkylthio steht -
(d) Benzoylketone der allgemeinen Formel (IV) 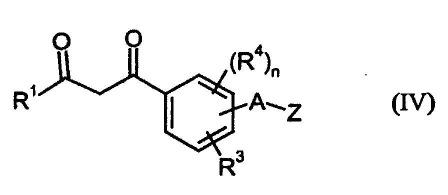
in welcher
n, A, R¹, R³, R⁴ und Z die in einem der Ansprüche 1 bis 3, 5, 8 und 9 angegebene Bedeutung haben,
mit Carbondisulfid (Schwefelkohlenstoff) und mit einem Alkylierungsmittel
und anschließend mit Hydroxylamin oder einem Säureaddukt hiervon
gegebenenfalls in Gegenwart eines oder mehrerer Reaktionshilfsmittel und gegebenenfalls in Gegenwart eines oder mehrerer Verdünnungsmittel umsetzt,
und gegebenenfalls im Anschluß daran an den gemäß Verfahren (a) bis (d) erhaltenen Verbindungen der Formel (I) im Rahmen der Substituentendefinition auf übliche Weise elektrophile oder nucleophile Substitutionsreaktionen bzw. Oxidations- oder Reduktionsreaktionen durchführt.

14. Verbindungen der allgemeinen Formel (IE) 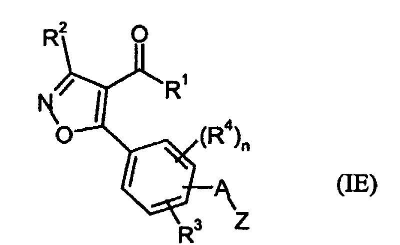 in welcher
n, A, R¹, R², R³, R⁴ und Z die in einem der Ansprüche 1 bis 3, 5, 6, 8 und 9 angegebene Bedeutung haben.

15. Verbindungen der allgemeinen Formel (II) mit Ausnahme von 4-(2-Brommethyl-benzoyl)-5-cyclopropyl-isoxazol-3-carbonsäure-ethylester 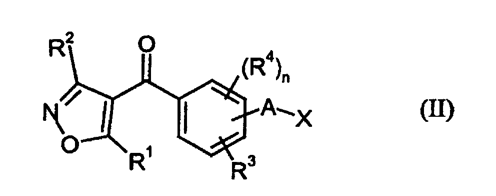 in welcher
n, A, R¹, R², R³ und R⁴ die in einem der Ansprüche 1 bis 3, 5, 6, 8 und 9 angegebene Bedeutung haben und
X für Halogen steht.

16. Verbindungen der allgemeinen Formel (IV) 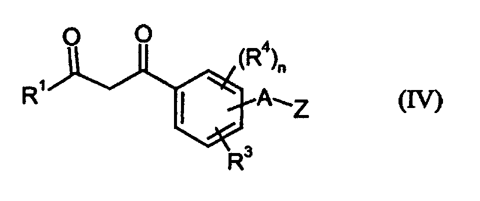 in welcher
n, A, R¹, R³, R⁴ und Z die in einem der Ansprüche 1 bis 3, 5, 8 und 9 angegebene Bedeutung haben.

17. Herbizide Mittel, **gekennzeichnet durch** den Gehalt mindestens einer Verbindung gemäß einem der Ansprüche 1 bis 12 und üblichen Streckmitteln.

18. Verwendung von mindestens einer Verbindung gemäß einem der Ansprüche 1 bis 12 zur Bekämpfung von unerwünschten Pflanzen.

19. Verbindungen der allgemeinen Formel (II) nach Anspruch 15 in welcher A für Methylen steht.

## Claims

1. Compounds of the general formula (I), 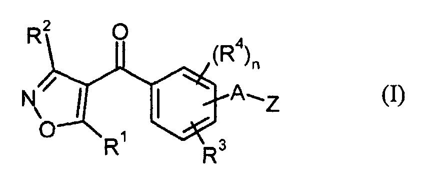 in which
n represents the number 0, 1, 2 or 3,
A represents methylene, ethylidene (ethane-1,1-diyl) or dimethylene (ethane-1,2-diyl),
R¹ represents hydrogen, represents in each case optionally fluorine-, chlorine-, methoxy-, ethoxy-, n- or i-propoxy-, methylthio-, ethylthio-, n- or i-propylthio-, methylsulphinyl-, ethylsulphinyl, n- or i-propylsulphinyl-, methylsulphonyl-, ethylsulphonyl-, n- or i-propylsulphonyl-substituted methyl, ethyl, n- or i-propyl, n-, i-, s- or t-butyl, represents in each case optionally fluorine-, chlorine- or bromine-substituted propenyl, butenyl, propinyl or butinyl, or represents in each case optionally cyano-, fluorine-, chlorine-, bromine-, methyl- or ethyl-substituted cyclopropyl, cyclobutyl, cyclopentyl or cyclohexyl,
R² represents hydrogen, cyano, carbamoyl, fluorine, chlorine, bromine, represents in each case optionally cyano-, fluorine-, chlorine-, methoxy- or ethoxy-substituted methyl, ethyl, n- or i-propyl, acetyl, propionyl, n- or i-butyroyl, methoxy, ethoxy, n- or i-propoxy, methoxycarbonyl, ethoxycarbonyl, n- or i-propoxycarbonyl, or represents in each case optionally fluorine- and/or chlorine-substituted methylthio, ethylthio, n- or i-propylthio,
R³ represents hydrogen, nitro, cyano, carboxyl, carbamoyl, thiocarbamoyl, fluorine, chlorine, bromine, iodine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n-or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, n-or i-propylsulphonyl, or represents methylamino, ethylamino, n- or i-propylamino, dimethylamino, diethylamino, dimethylaminosulphonyl or diethylaminosulphonyl,
R⁴ represents nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents methylamino, ethylamino, n- or i-propylamino, dimethylamino, diethylamino, dimethylaminosulphonyl or diethylaminosulphonyl,
Z represents one of the groupings 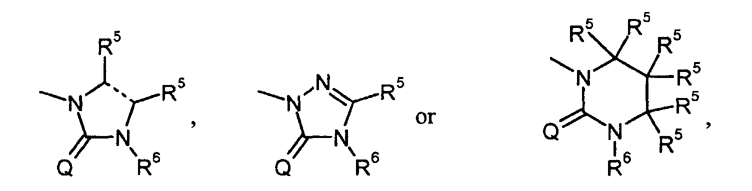
R⁵ represents hydrogen, hydroxyl, mercapto, cyano, fluorine, chlorine, bromine, iodine, represents in each case optionally fluorine-, chlorine-, methoxy-, ethoxy-, n- or i-propoxy-, n-, i-, s- or t-butoxy-, methylthio-, ethylthio-, n- or i-propylthio-, n-, i-, s- or t-butylthio-, methylsulphinyl-, ethylsulphinyl-, n- or i-propylsulphinyl-, methylsulphonyl-, ethylsulphonyl-, n- or i-propylsulphonyl-substituted methyl, ethyl, n-or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t-butylamino, dimethylamino, diethylamino, di-n-propylamino or di-i-propylamino, represents in each case optionally fluorine- and/or chlorine-substituted ethenyl, propenyl, butenyl, ethinyl, propinyl, butinyl, propenyloxy, butenyloxy, propenylthio, butenylthio, propenylamino or butenylamino, represents in each case optionally fluorine- and/or chlorine-substituted cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents in each case optionally fluorine-, chlorine-, methyl-, ethyl-, n- or i-propyl-, n-, i-, s- or t-butyl-, methoxy-, ethoxy-, n- or i-propoxy-substituted phenyl, phenyloxy, phenylthio, phenylamino, benzyl, benzyloxy, benzylthio or benzylamino, represents pyrrolidino, piperidino or morpholino, or - if two adjacent radicals R⁵ and R⁵ are located at a double bond - together with the adjacent radical R⁵ also represents a benzo grouping,
R⁶ represents hydrogen, hydroxyl, amino, represents in each case optionally fluorine- and/or chlorine-, methoxy- or ethoxy-substituted methyl, ethyl, n- or i-propyl, n-, i- or s-butyl, methoxy, ethoxy, n- or i-propoxy, methylamino, ethylamino or dimethylamino, represents in each case optionally fluorine- and/or chlorine-substituted ethenyl, propenyl, ethinyl, propinyl or propenyloxy, represents in each case optionally fluorine- and/or chlorine-substituted cyclopropyl, cyclobutyl, cyclopentyl, cyclohexyl, cyclopropylmethyl, cyclobutylmethyl, cyclopentylmethyl, cyclohexylmethyl, or represents in each case optionally fluorine-, chlorine-, methyl-, ethyl-, n- or i-propyl-, n-, i-, s-or t-butyl-, methoxy-, ethoxy-, n- or i-propoxy-substituted phenyl or benzyl, or together with an adjacent radical R⁵ or R⁶ represents in each case optionally methyl- and/or ethyl-substituted propane-1,3-diyl (trimethylene) or butane-1,4-diyl (tetramethylene), and
Q represents oxygen or sulphur.

2. Compounds according to Claim 1, **characterized in that**
R¹ represents hydrogen, represents in each case optionally fluorine-, chlorine-, methoxy-, ethoxy-, methylthio-, ethylthio-, methylsulphinyl-, ethylsulphinyl-, methylsulphonyl- or ethylsulphonyl-substituted methyl, ethyl, n- or i-propyl, n-, i-, s- or t-butyl, or represents optionally cyano-, fluorine-, chlorine-, bromine-, methyl- or ethyl-substituted cyclopropyl,
R² represents hydrogen, cyano, carbamoyl, fluorine, chlorine, bromine, represents in each case optionally cyano-, fluorine-, chlorine-, methoxy- or ethoxy-substituted methyl, ethyl, n- or i-propyl, n-, i-, s- or t-butyl, methoxycarbonyl, ethoxycarbonyl, n- or i-propoxycarbonyl, or represents in each case optionally fluorine- and/or chlorine-substituted methylthio, ethylthio, n- or i-propylthio,
R³ represents hydrogen, nitro, cyano, fluorine, chlorine, bromine, iodine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁴ represents nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁵ represents hydrogen, hydroxyl, chlorine, bromine, methyl, ethyl, n- or i-propyl, n-, i-, s- or t-butyl, difluoromethyl, dichloromethyl, trifluoromethyl, trichloromethyl, chlorodifluoromethyl, fluorodichloromethyl, fluoroethyl, chloroethyl, difluoroethyl, dichloroethyl, fluoro-n-propyl, fluoro-i-propyl, chloro-n-propyl, chloro-i-propyl, methoxymethyl, ethoxymethyl, methoxyethyl, ethoxyethyl, methoxy, ethoxy, n- or i-propoxy, n-, i-, s- or t-butoxy, fluoroethoxy, chloroethoxy, difluoroethoxy, dichloroethoxy, trifluoroethoxy, trichloroethoxy, chlorofluoroethoxy, chlorodifluoroethoxy, fluorodichloroethoxy, methylthio, ethylthio, n- or i-propylthio, fluoroethylthio, chloroethylthio, difluoroethylthio, dichloroethylthio, chlorofluoroethylthio, chlorodifluoroethylthio, fluorodichloroethylthio, methylsulphinyl, ethylsulphinyl, n-or i-propylsulphinyl, methylsulphonyl, ethylsulphonyl, n- or i-propylsulphonyl, dimethylamino, propenylthio, butenylthio, propinylthio, butinylthio, cyclopropyl, cyclopropylmethyl, cyclopropylmethoxy, phenyl or phenoxy, and
R⁶ represemts amino, methyl, ethyl, n- or i-propyl, n-, i-, s- or t-butyl, methoxy, ethoxy, methylamino, dimethylamino, cyclopropyl or cyclopropylmethyl, or together with R⁵ represents propane-1,3-diyl (trimethylene), butane-1,4-diyl (tetramethylene) or pentane-1,5-diyl (pentamethylene).

3. Compounds according to either of Claims 1 and 2, **characterized in that**
A represents methylene.

4. Compounds according to any of Claims 1 to 3, **characterized in that**
Q represents oxygen (O).

5. Compounds according to any of Claims 1 to 4, **characterized in that**
R¹ represents cyclopropyl.

6. Compounds according to any of Claims 1 to 5, **characterized in that**
R² represents hydrogen, methoxycarbonyl or ethoxycarbonyl.

7. Compounds according to any of Claims 1 to 6, **characterized in that**
R⁶ represents methyl, dimethylamino or cyclopropyl.

8. Compounds according to any of Claims 1 to 7, **characterized in that**
R³ represents chlorine, bromine, cyano, trifluoromethyl or methylsulphonyl.

9. Compounds according to any of Claims 1 to 8, **characterized in that**
R⁴ represents hydrogen, cyano, chlorine, nitro, methyl, trifluoromethyl, methoxy or methylsulphonyl.

10. Compounds according to any of Claims 1 to 9 of the general formula (IA) 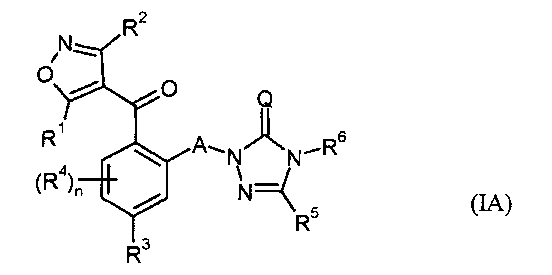 in which
n, A, Q, R¹, R², R³, R⁴, R⁵ and R⁶ are each as defined in any of Claims 1 to 9.

11. Compounds according to any of Claims 1 to 9 of the general formula (IB) 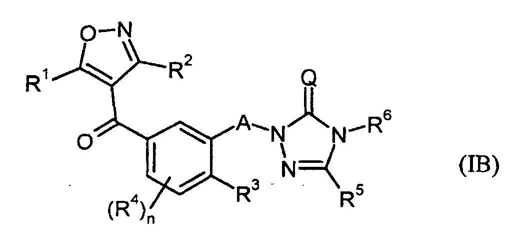 in which
n, A, Q, R¹, R², R³, R⁴, R⁵ and R⁶ are each as defined in any of Claims 1 to 9.

12. Compounds according to any of Claims 1 to 9 of the general formula (IC) 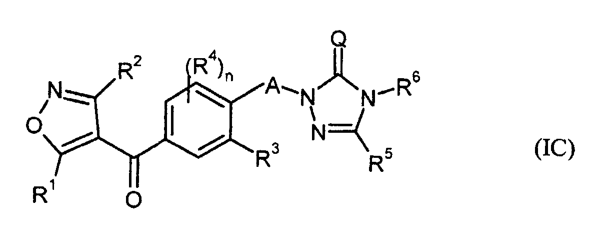 in which
n, A, Q, R¹, R², R³, R⁴, R⁵ and R⁶ are each as defined in any of Claims 1 to 9.

13. Process for preparing compounds according to any of Claims 1 to 12, **characterized in that**
(a) benzoylisoxazoles of the general formula (II) 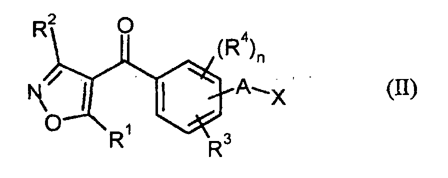 in which
n, A, R¹, R², R³ and R⁴ are each as defined in any of Claims 1 to 3, 5, 6, 8 and 9 and
X represents halogen
are reacted with heterocycles of the general formula (III)
H-Z (III)
in which
Z is as defined in Claim 1,
if appropriate in the presence of one or more reaction auxiliaries and if appropriate in the presence of one or more diluents,
or that
- if R² is hydrogen-
(b) benzoyl ketones of the general formula (IV) 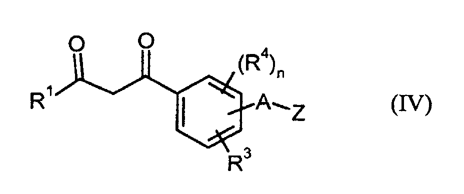 in which
n, A, R¹, R³, R⁴ and Z are each as defined in any of Claims 1 to 3, 5, 8 and 9
are reacted with an orthoformic ester or an N,N-dimethylformamide acetal
and subsequently with hydroxylamine or an acid adduct thereof,
if appropriate in the presence of one or more reaction auxiliaries and if appropriate in the presence of one or more diluents,
or that
- if R² represents optionally substituted alkoxycarbonyl -
(c) benzoyl ketones of the general formula (IV) 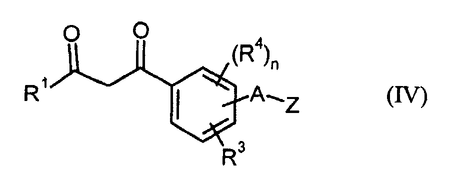 in which
n, A, R¹, R³, R⁴ and Z are each as defined in any of Claims 1 to 3, 5, 8 and 9
are reacted with a cyanoformic ester and then with hydroxylamine or an acid adduct thereof, or with an alkyl chloro-hydroximino-acetate,
if appropriate in the presence of one or more reaction auxiliaries and if appropriate in the presence of one or more diluents,
or that
- if R² represents alkylthio -
(d) benzoyl ketones of the general formula (IV) 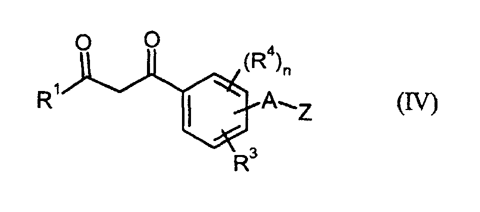 in which
n, A, R¹, R³, R⁴ and Z are each as defined in any of Claims 1 to 3, 5, 8 and 9
are reacted with carbon disulphide and with an alkylating agent
and then with hydroxylamine or an acid adduct thereof,
if appropriate in the presence of one or more reaction auxiliaries and if appropriate in the presence of one or more diluents,
and electrophilic or nucleophilic substitutions and/or oxidations or reductions within the scope of the definition of the substituents are, if appropriate, subsequently carried out in a customary manner on the compounds of the formula (I) obtained according to processes (a) to (d).

14. Compounds of the general formula (IE) 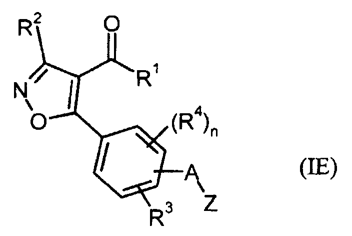 in which
n, A, R¹, R², R³, R⁴ and Z are each as defined in any of Claims 1 to 3, 5, 6, 8 and 9.

15. Compounds of the general formula (II), except for ethyl 4-(2-bromo-methylbenzoyl)-5-cyclopropyl-isoxazole-3-carboxylate, 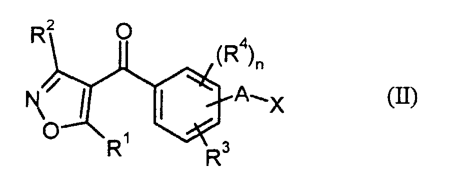 in which
n, A, R¹, R², R³ and R⁴ are each as defined in any of Claims 1 to 3, 5, 6, 8 and 9 and
X represents halogen.

16. Compounds of the general formula (IV) 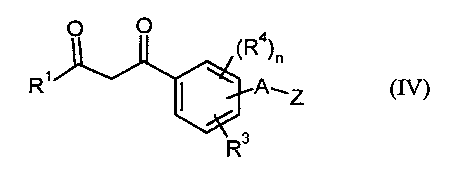 in which
n, A, R¹, R³, R⁴ and Z are each as defined in any of Claims 1 to 3, 5, 8 and 9.

17. Herbicidal compositions, **characterized in that** they comprise at least one compound according to any of Clams I to 12 and customary extenders.

18. Use of at least one compound according to any of Claims 1 to 12 for controlling undesirable plants.

19. Compounds of the general formula (II) according to Claim 15 in which
A represents methylene.

## Revendications

1. Composés de formule générale (I) 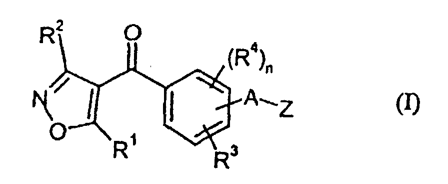 dans laquelle
n représente les nombres 0, 1, 2 ou 3,
A est un groupe méthylène, éthylidène(éthane-1,1-diyle) ou diméthylène(éthane-1,2-diyle),
R¹ représente l'hydrogène, un reste méthyle, éthyle, n-propyle, isopropyle, n-butyle, isobutyle, sec.-butyle ou tertio-butyle portant chacun, le cas échéant, un substituant fluoro, chloro, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle, un reste propényle, butényle, propynyle ou butynyle portant chacun, le cas échéant, un substituant fluoro, chloro ou bromo, ou un reste cyclopropyle, cyclobutyle, cyclopentyle ou cyclohexyle portant chacun, le cas échéant, un substituant cyano, fluoro, chloro, bromo, méthyle ou éthyle,
R² représente l'hydrogène, un groupe cyano, carbamoyle, le fluor, le chlore, le brome, un reste méthyle, éthyle, n-propyle, isopropyle, acétyle, propionyle, n-butyroyie, isobutyroyle, méthoxy, éthoxy, n-propoxy, isopropoxy, méthoxycarbonyle, éthoxycarbonyle, n-propoxycarbonyle; isopropoxycarbonyle portant chacun, le cas échéant, un substituant cyano, fluoro, chloro, méthoxy ou éthoxy, ou bien un reste méthylthio, éthylthio, n-propylthio ou isopropylthio portant chacun, le cas échéant, un substituant fluoro et/ou chloro,
R³ représente l'hydrogène, un groupe nitro, cyano, carboxy, carbamoyle, thiocarbamoyle, le fluor, le chlore, le brome, l'iode, un reste méthyle, éthyle, n-propyle, isopropyle, n-butyle, isobutyle, sec.-butyle ou tertio-butyle portant chacun, le cas échéant, un substituant fluoro et/ou chloro, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthoxy, éthoxy, n-propoxy ou isopropoxy portant chacun, le cas échéant, un substituant fluoro et/ou chloro, méthoxy, éthoxy, n-propoxy ou isopropoxy, un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle portant chacun, le cas échéant, un substituant fluoro et/ou chloro, ou un reste méthylamino, éthylamino, n-propylamino, isopropylamino, diméthylamino, diéthylamino, diméthylaminosulfonyle ou diéthylaminosulfonyle,
R⁴ représente un groupe nitro, cyano, carboxy, carbamoyle, thiocarbamoyle, le fluor, le chlore, le brome, un reste méthyle, éthyle, n-propyle, isopropyle, n-butyle, isobutyle, sec.-butyle ou tertiobutyle portant chacun, le cas échéant, un substituant fluoro et/ou chloro, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthoxy, éthoxy, n-propoxy ou isopropoxy portant chacun, le cas échéant, un substituant fluoro et/ou chloro, méthoxy, éthoxy, n-propoxy ou isopropoxy, un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle portant chacun, le cas échéant, un substituant fluoro et/ou chloro, ou un reste méthylamino, éthylamino, n-propylamino, isopropylamino, diméthylamino, diéthylamino, diméthylaminosulfonyle ou diéthylaminosulfonyle,
Z représente l'un des groupements 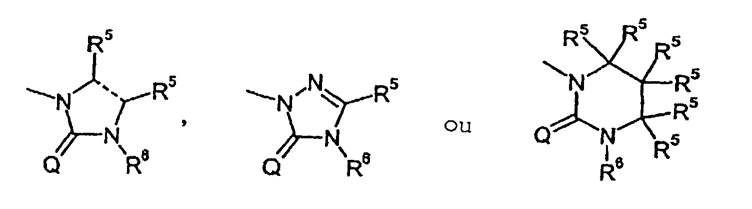
R⁵ représente l'hydrogène, un groupe hydroxy, mercapto, cyano, le fluor, le chlore, le brome, l'iode, un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylsulfinyle, éthylsulfinyle, n-propylsulfinyle, isopropylsulfinyle, méthylsulfonyle, éthylsulfonyle, n-propylsulfonyle, isopropylsulfonyle portant chacun, le cas échéant, un substituant fluoro, chloro, méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylsulfinyle, éthylsulfinyle, n-propylsulfinyle, isopropylsulfinyle, méthylsulfonyle, éthylsulfonyle, n-propylsulfonyle ou isopropylsulfonyle, un reste méthylamino, éthylamino, n-propylamino, isopropylamino, n-butylamino, isobutylamino, sec.-butylamino, tertio-butylamino, diméthylamino, diéthylamino, di-n-propylamino ou diisopropylamino, un reste éthényle, propényle, butényle, éthynyle, propynyle, butynyle, propényloxy, butényloxy, propénylthio, buténylthio, propénylamino ou buténylamino portant chacun, le cas échéant, un substituant fluoro et/ou chloro, un reste cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylthio, cyclobutylméthylamino, cyclopentylméthylamino ou cyclohexylméthylamino portant chacun, le cas échéant, un substituant fluoro et/ou chloro, ou bien un reste phényle, phényloxy, phénylthio, phénylamino, benzyle, benzyloxy, benzylthio ou benzylamino portant chacun, le cas échéant, un substituant fluoro, chloro, méthyle, éthyle, n-propyle, isopropyle, n-butyle, isobutyle, sec.-butyle, tertio-butyle, méthoxy, éthoxy, n-propoxy ou isopropoxy, un reste pyrrolidino, pipéridino ou morpholino, ou bien - au cas où deux restes R⁵ et R⁵ contigus se trouvent sur une double liaison -, R⁵ forme aussi conjointement avec le reste R⁵ voisin un groupement benzo,
R⁶ représente l'hydrogène, un groupe hydroxy, amino, un reste méthyle, éthyle, n-propyle, isopropyle, n-butyle, isobutyle, sec.-butyle, méthoxy, éthoxy, n-propoxy, isopropoxy, méthylamino, éthylamino ou diméthylamino portant chacun, le cas échéant, un substituant fluoro et/ou chloro, un reste éthényle, propényle, éthynyle, propynyle ou propényloxy portant chacun, le cas échéant, un substituant fluoro et/ou chloro, un reste cyclopropyle, cyclobutyle, cyclopentyle, cyclohexyle, cyclopropylméthyle, cyclobutylméthyle, cyclopentylméthyle, cyclohexylméthyle portant chacun, le cas échéant, un substituant fluoro et/ou chloro, ou bien un reste phényle ou benzyle portant chacun, le cas échéant, un substituant fluoro, chloro, méthyle, éthyle, n-propyle, isopropyle, n-butyle, isobutyle, sec.-butyle, tertio-butyle, méthoxy, éthoxy, n-propoxy ou isopropoxy, ou bien forme conjointement avec un reste R⁵ ou R⁶ voisin un reste propane-1,3-diyle(triméthylène) ou butane-1,4-diyle(tétraméthylène) portant chacun, le cas séchéant, un substituant méthyle et/ou éthyle, et
Q représente l'oxygène ou le soufre.

2. Composés suivant la revendication 1, **caractérisés en ce que**
R¹ représente l'hydrogène, un reste méthyle, éthyle, n-propyle, isopropyle, n-butyle, isobutyle, sec.-butyle ou tertio-butyle portant chacun, le cas échéant, un substituant fluoro, chloro, méthoxy, éthoxy, méthylthio, éthylthio, méthylsulfinyle, éthylsulfinyle, méthylsulfonyle ou éthylsulfonyle, ou un reste cyclopropyle portant éventuellement un substituant cyano, fluoro, chloro, bromo, méthyle ou éthyle,
R² représente l'hydrogène, un groupe cyano, carbamoyle, le fluor, le chlore, le brome, un reste méthyle, éthyle, n-propyle, isopropyle, n-butyle, isobutyle, sec.-butyle, tertio-butyle, méthoxycarbonyle, éthoxycarbonyle, n-propoxycarbonyle ou isopropoxy-carbonyle portant chacun, le cas échéant, un substituant cyano, fluoro, chloro, méthoxy ou éthoxy, ou un reste méthyl- thio, éthylthio, n-propylthio ou isopropylthio portant chacun un substituant fluoro et/ou chloro,
R³ représente l'hydrogène, un groupe nitro, cyano, le fluor, le chlore, le brome, l'iode, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁴ est un groupe nitro, cyano, le fluor, le chlore, le brome, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁵ représente l'hydrogène, un groupe hydroxy, le chlore, le brome, un reste méthyle, éthyle, n-propyle, isopropyle, n-butyle, isobutyle, sec.-butyle, tertio-butyle, difluorométhyle, dichlorométhyle, trifluorométhyle, trichlorométhyle, chlorodifluorométhyle, fluorodichlorométhyle, fluoréthyle, chloréthyle, difluoréthyle, dichloréthyle, fluoro-n-propyle, fluorisopropyle, chloro-n-propyle, chlorisopropyle, méthoxyméthyle, éthoxyméthyle, méthoxyéthyle, éthoxyéthyle, méthoxy, éthoxy, n-propoxy, isopropoxy, n-butoxy, isobutoxy, sec.-butoxy, tertio-butoxy, fluoréthoxy, chloréthoxy, difluoréthoxy, dichloréthoxy, trifluoréthoxy, trichloréthoxy, chlorofluoréthoxy, chlorodifluoréthoxy, fluorodichloréthoxy, méthylthio, éthylthio, n-propylthio, isopropylthio, fluoréthylthio, chloréthylthio, difluoréthylthio, dichloréthylthio, chlorofluoréthylthio, chlorodifluoréthylthio, fluorodichlor- éthylthio, méthylsulfinyle, éthylsulfinyle, n-propylsulfinyle, isopropylsulfinyle, méthylsulfonyle, éthylsulfonyle, n-propylsulfonyle, isopropylsulfonyle, diméthylamino, propénylthio, buténylthio, propynylthio, butynylthio, cyclopropyle, cylopropylméthyle, cyclopropylméthoxy, phényle ou phénoxy, et
R⁶ est un groupe amino, un reste méthyle, éthyle, n-propyle, isopropyle, n-butyle, isobutyle, sec.-butyle, tertio-butyle, méthoxy, éthoxy, méthylamino, diméthylamino, cyclopropyle ou cyclopropylméthyle ou forme conjointement avec R⁵ un reste propane-1,3-diyle(triméthylène), butane-1,4-diyle(tétraméthylène) ou pentane-1,5-diyle(pentaméthylène).

3. Composés suivant la revendication 1 ou 2, **caractérisés en ce que**
A est un reste méthylène.

4. Composés suivant l'une des revendications 1 à 3, **caractérisés en ce que**
Q est l'oxygène(O).

5. Composés suivant l'une des revendications 1 à 4, **caractérisés en ce que**
R¹ est un reste cyclopropyle.

6. Composés suivant l'une des revendications 1 à 5, **caractérisés en ce que**
R² représente l'hydrogène, un reste méthoxycarbonyle ou éthoxycarbonyle.

7. Composés suivant l'une des revendications 1 à 6, **caractérisés en ce que**
R⁶ représente un reste méthyle, diméthylamino ou cyclopropyle.

8. Composés suivant l'une des revendications 1 à 7, **caractérisés en ce que**
R³ représente le chlore, le brome, un groupe cyano, un reste trifluorométhyle ou méthylsulfonyle.

9. Composés suivant l'une des revendications 1 à 8, **caractérisés en ce que**
R⁴ représente l'hydrogène, un groupe cyano, le chlore, un groupe nitro, un reste méthyle, trifluorométhyle, méthoxy ou méthylsulfonyle.

10. Composés suivant l'une des revendications 1 à 9, de formule générale (IA) 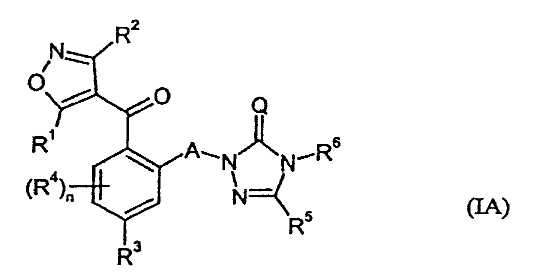 dans laquelle
n, A, Q, R¹, R², R³, R⁴, R⁵ et R⁶ ont la définition indiquée dans l'une des revendications 1 à 9.

11. Composés suivant l'une des revendications 1 à 9, de formule générale (IB) 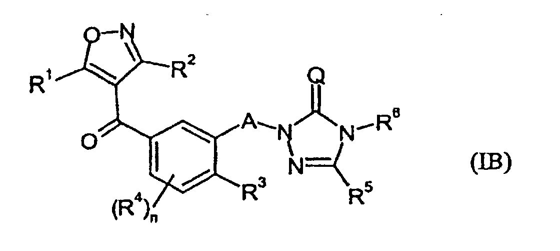 dans laquelle
n, A, Q, R¹, R², R³, R⁴, R⁵ et R⁶ ont la définition indiquée dans l'une des revendications 1 à 9.

12. Composés suivant l'une des revendications 1 à 9, de formule générale (IC) 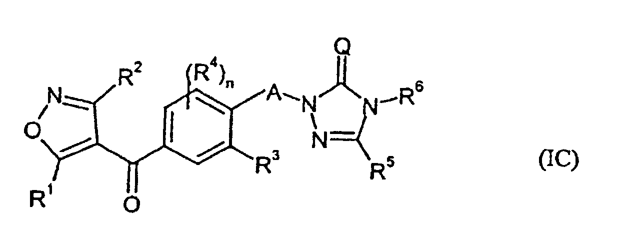 dans laquelle
n, A, Q, R¹, R², R³, R⁴, R⁵ et R⁶ ont la définition indiquée dans l'une des revendications 1 à 9.

13. Procédé de production de composés suivant l'une des revendications 1 à 12, **caractérisé en ce que** :
(a) on fait réagir des benzoylisoxazoles de formule générale (II) 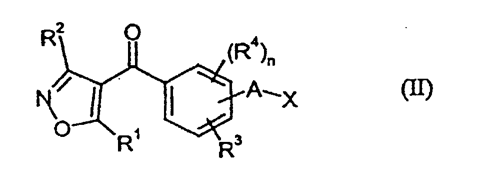 dans laquelle
n, A, R¹, R², R³ et R⁴ ont la définition indiquée dans l'une des revendications 1 à 3, 5, 6, 8 et 9 et
X représente un halogène,
avec des hétérocycles de formule générale (III)
H-Z (III)
dans laquelle
Z a la définition indiquée dans la revendication 1,
le cas échéant en présence d'un ou plusieurs auxiliaires de réaction et en présence éventuelle d'un ou plusieurs diluants,
ou **en ce que**
- au cas où R² représente l'hydrogène -
(b) on fait réagir des benzoylcétones de formule générale (IV) 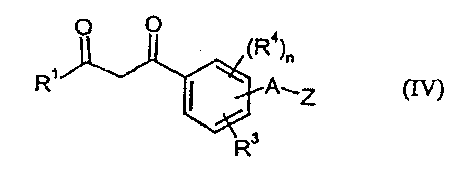 dans laquelle
n, A, R¹, R³, R⁴ et Z ont la définition indiquée dans l'une des revendications 1 à 3, 5, 8 et 9,
avec un ester d'acide orthoformique ou un N,N-diméthyl-formamide-acétal,
puis avec l'hydroxylamine ou un produit d'addition d'acide de l'hydroxylamine,
le cas échéant en présence d'un ou plusieurs auxiliaires de réaction et en présence éventuelle d'un ou plusieurs diluants,
ou **en ce que**
- au cas où R² représente un reste alkoxycarbonyle éventuellement substitué -
(c) on fait réagir des benzoylcétones de formule générale (IV) 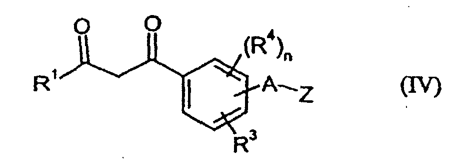 dans laquelle
n, A, R¹, R³, R⁴ et Z ont la définition indiquée dans l'une des revendications 1 à 3, 5, 8 et 9,
avec un ester d'acide cyanoformique, puis avec l'hydroxylamine ou un produit d'addition d'acide de l'hydroxylamine, ou avec un ester alkylique d'acide chlorhydroximino-acétique,
le cas échéant en présence d'un ou plusieurs auxiliaires de réaction et en présence éventuelle d'un ou plusieurs diluants,
ou **en ce que**
- au cas où R² est un reste alkylthio -
(d) on fait réagir des benzoylcétones de formule générale (IV) 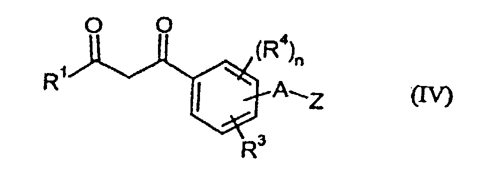
dans laquelle
n, A, R¹, R³, R⁴ et Z ont la définition indiquée dans l'une des revendications 1 à 3, 5, 8 et 9,
avec le sulfure de carbone et un agent d'alkylation, puis avec l'hydroxylamine ou un produit d'addition d'acide de l'hydroxylamine,
le cas échéant en présence d'un ou plusieurs auxiliaires de réaction et en présence éventuelle d'un ou plusieurs diluants,
après quoi on conduit, le cas échéant, d'une manière classique sur les composés de formule (I) obtenus conformément aux procédés (a) à (d), dans le cadre de la définition des substituants, des réactions de substitution électrophiles ou nucléophiles ou des réactions d'oxydation ou de réduction.

14. Composés de formule générale (IE) 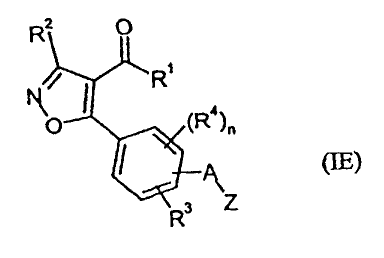 dans laquelle
n, A, R¹, R³, R⁴ et Z ont la définition donnée dans l'une des revendications 1 à 3, 5, 6, 8 et 9.

15. Composés de formule générale (II) 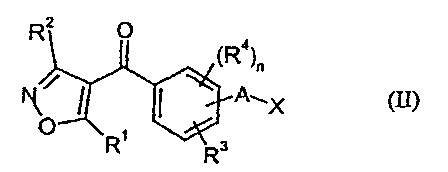 dans laquelle
n, A, R¹, R², R³ et R⁴ ont la définition indiquée dans l'une des revendications 1 à 3, 5, 6, 8 et 9, et
X représente un halogène,
excepté l'ester éthylique d'acide 4-(2-bromométhylbenzoyl)-5-cyclopropylisoxazole-3-carboxylique.

16. Composés de formule générale (IV) 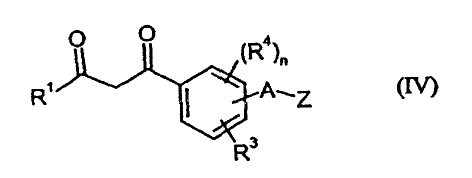 dans laquelle
n, A, R¹, R³, R⁴ et Z ont la définition indiquée dans l'une des revendications 1 à 3, 5, 8 et 9.

17. Compositions herbicides, **caractérisées par** la teneur en au moins un composé suivant l'une des revendications 1 à 12 avec des diluants usuels.

18. Utilisation d'au moins un composé suivant l'une des revendications 1 à 12 pour combattre une végétation indésirable.

19. Composés de formule générale (II) suivant la revendication 15, dans laquelle A est le groupe méthylène.
